(19) Europäisches Patentamt
European Patent Office
Office européen des brevets

(11) EP 4 487 857 A1

(12) EUROPEAN PATENT APPLICATION
published in accordance with Art. 153(4) EPC

(43) Date of publication:
**08.01.2025 Bulletin 2025/02**

(21) Application number: **23763500.8**

(22) Date of filing: **01.03.2023**

(51) International Patent Classification (IPC):
***A61K 35/28** (2015.01)* ***A61P 25/02** (2006.01)*
***A61P 25/28** (2006.01)* ***C12N 5/0775** (2010.01)*
***C12N 5/10** (2006.01)* ***C12N 15/12** (2006.01)*
***C12N 15/37** (2006.01)* ***C12N 15/54** (2006.01)*
***C12N 15/86** (2006.01)*

(52) Cooperative Patent Classification (CPC):
**A61K 35/28; A61P 25/02; A61P 25/28; C07K 14/025; C07K 14/435; C12N 5/06; C12N 5/10; C12N 9/10; C12N 15/86**

(86) International application number:
**PCT/JP2023/007619**

(87) International publication number:
**WO 2023/167243 (07.09.2023 Gazette 2023/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.03.2022 JP 2022031332**

(71) Applicants:
- **Tokyo Medical University**
  **Tokyo 160-8402 (JP)**
- **Cysay Inc.**
  **Tokyo 105-0001 (JP)**

(72) Inventors:
- **YOSHIMOTO Takayuki**
  **Tokyo 160-8402 (JP)**
- **HASEGAWA Hideaki**
  **Tokyo 160-8402 (JP)**
- **MIZOGUCHI Izuru**
  **Tokyo 160-8402 (JP)**
- **YAMASHITA Yasuhiro**
  **Tokyo 105-0001 (JP)**

(74) Representative: **Kuhnen & Wacker**
**Patent- und Rechtsanwaltsbüro PartG mbB**
**Prinz-Ludwig-Straße 40A**
**85354 Freising (DE)**

Remarks:
The complete document including Reference Table(s) and the Sequence Listing(s) can be downloaded from the EPO website

# (54) PHARMACEUTICAL COMPOSITION FOR TREATMENT OF DEMYELINATING DISEASES CONTAINING SUPERNATANT OF IMMORTALIZED MESENCHYMAL STEM CELLS, AND PHARMACEUTICAL PREPARATION CONTAINING SAME COMPOSITION AS ACTIVE INGREDIENT

(57) The purpose of the present invention is to provide a therapeutic agent for demyelinating diseases in which a culture supernatant of immortalized mesenchymal stem cells into which specific genes are incorporated serves as an active ingredient. Provided is a therapeutic agent for demyelinating diseases in which a culture supernatant of immortalized mesenchymal stem cells into which either the hTERT or pTERT gene and the bmi-1 gene, the HPV-E6 gene, and the HPV-E7 gene are incorporated serves as an active ingredient. The mesenchymal stem cells are preferably of human or porcine origin, and the culture supernatant is preferably obtained by culturing the mesenchymal stem cells for 24-96 hours and contains a component that acts on the receptor c-MET of hepatocyte growth factor (HGF).

EP 4 487 857 A1

# Figure 3

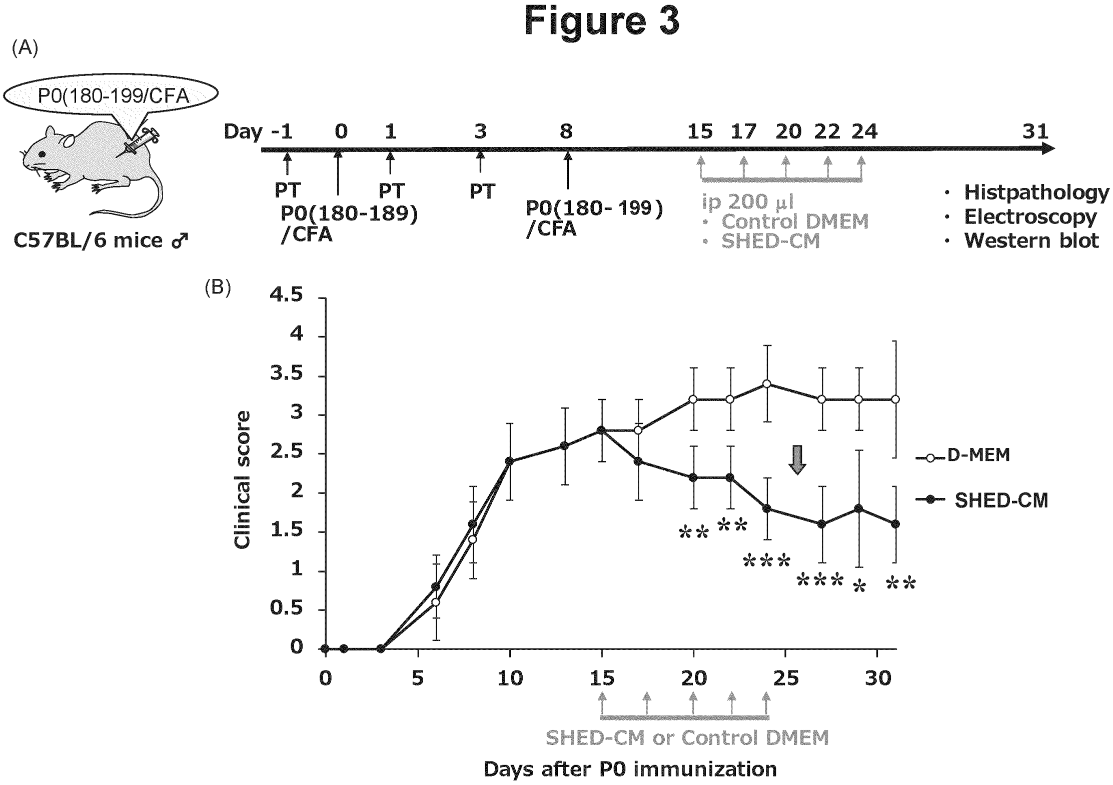
(A)
P0(180-199/CFA
C57BL/6 mice ♂
Day -1 0 1 3 8 15 17 20 22 24 31
PT
P0(180-189)
/CFA
PT
PT
P0(180- 199)
/CFA
ip 200 µl
・ Control DMEM
・ SHED-CM
・ Histpathology
・ Electroscopy
・ Western blot
(B)
Clinical score
4.5
4
3.5
3
2.5
2
1.5
1
0.5
0
0 5 10 15 20 25 30
D-MEM
SHED-CM
** ** *** *** * **
SHED-CM or Control DMEM
Days after P0 immunization

## Description

### Technical field

The present invention relates to a pharmaceutical composition for treating demyelinating diseases containing a culture supernatant of immortalized mesenchymal stem cells, and a pharmaceutical preparation containing the composition as an active ingredient. More particularly, the invention relates to a pharmaceutical composition for treating demyelinating diseases induced by infection or autoimmune reactions, such as Guillain-Barré syndrome, or as a side effect of treatment of other diseases, such as chemotherapy for cancer, and a pharmaceutical preparation containing thereof as an active ingredient.

In recent years, cell therapy using mesenchymal stem cells (hereafter abbreviated as "MSC") has been actively investigated. There, the terms "cell therapy" is defined as a method that uses cells themselves as a means of treatment and aims to cure various diseases using either one's own cells (autologous) or non-self-cells (derived from the third party, namely non-autologous). With recent advances in cellular and genetic engineering technologies, various cell therapy techniques have been developed, including (1) a method to treat such diseases by using the administered cells themselves, and (2) the method to treat such diseases by producing the cells for the therapy in the body to which the cells are administrated, and (3) the method to treat such disease by using the cells being taken out from the living body is artificially processed so as to have treatment effects. There are mentioned as an example of (1), blood transfusion therapy, and as the example of (2), bone marrow transplantation is mentioned. As the example of (3), there is mentioned such as CAR-T cell therapy or iPS cell therapy and the like.

It is known that the administered MSCs secrete various cytokines, when administering MSCs to a damaged part of a tissue. It is also known that the secreted cytokines give rise to restoring the damaged tissue or suppressing immune reactions through their paracrine effects. On the other hand, by removing the cells from the cell culture supernatant of MSCs (Conditioned Medium, hereafter abbreviated as "CM"), immunological rejection due to histocompatibility antigens need not be considered. In addition, since it is highly safe as described below, it is focused on (see, the non-patent document 1, hereafter, referred to as "prior art 1").

Nervous tissue is roughly divided into the central nervous system, which consists of the brain and spinal cord, and the peripheral nerves, which extend from the brain and spinal cord to the periphery. The nerve network consists of axons and dendrites, which are extended from the cell bodies of neurons. The axons are covered by an insulating structure called the myelin sheath, which allows them to rapidly transmit information to distant cells. The cells that make up the myelin sheath are oligodendrocytes in the central nervous system and Schwann cells in the peripheral nerves.

The myelin sheaths as described above sometimes degenerate or demyelinate due to a variety of causes. Diseases caused by degeneration or loss of the myelin sheath are collectively referred to as demyelinating diseases. It is known that the causes of demyelinating diseases are diverse, such as immune origine, genetic origine, ischemic origine, infectious origine, drug-induced origine, and the like. For example, there are mentioned such as central nervous system demyelinating diseases, multiple sclerosis, neuromyelitis optica, acute disseminated encephalomyelitis, atopic myelitis, progressive multifocal leukoencephalopathy, HTLB-1-related myelopathy, HIV-related leukoencephalopathy, subacute sclerosing panencephalitis, osmotic demyelinating syndrome, drug-induced leukoencephalopathy and the like. As peripheral neurogenic demyelinating diseases, there are mentioned such as, for example, Guillain-Barré syndrome, Fisher syndrome, diabetic neuropathy, chronic inflammatory demyelinating polyradiculoneuropathy, multifocal motor neuropathy, herpes zoster, and the like.

It is well known that abnormalities in autoimmunity cause the above-mentioned immune demyelinating diseases. The body has inherently an immune system as a biological defense mechanism. When it functions normally, foreign substances (recognized as non-self) are attacked and eliminated, whereas they do not respond against the antigens owned by self-tissues. However, disruption of the normal function thereof may result in induction of T cells or production of antibodies called autoantibodies which recognize the self-tissues as foreign. This causes attacks on specific cells and tissues as the target by the immune cells, leading to the immune response against the self-tissues as the target. It is known to cause a neurological disorder called autoimmune neuritis (sometimes abbreviated as "EAN"), when such autoimmune response occurs in nervous tissue. **In** particular, immune abnormalities, which attack on one's own myelin sheath, cause demyelinating disease.

As Demyelinating diseases caused by EAN, there are mentioned such as multiple sclerosis, acutely progressive Guillain-Barré syndrome and Fisher syndrome, chronic inflammatory demyelinating polyradiculoneuropathy (hereinafter abbreviated as "CIDP") which progresses chronically, MAG antibody positive neuropathy, multifocal motor neuropathy (hereinafter abbreviated as "MMN") and the like. These are accompanied by peripheral neuropathy.

Among the disease described above, symptoms such as numbness and weakness in the tips of the hands and feet appear in Guillain-Barré syndrome, and they develop rapidly from a few days to two weeks after the onset of symptoms, reaching a peak within two to four weeks after the onset of neurological symptoms. It is known that quadriplegia progresses, requiring assistance in walking in severe cases, and more than 10% of patients have paralysis of the respiratory muscles, requiring a ventilator. About half of the patients develop cranial nerve deficits such as facial paralysis, diplopia, and dysphagia. It is considered that severe disease is caused by axonal degeneration associated with demyelination, and early treatment is important.

It is said that 60% of Guillain-Barré syndrome is caused by the production of anti-ganglioside antibodies. Therefore, transvenous immunoglobulin therapy and plasma exchange therapy are used to attenuate the effects of anti-ganglioside antibodies (Non-Patent Document 2, hereinafter referred to as "Prior art 2"). However, some patients do not respond to these therapies. This is sometimes attributed to cells, for example the cells related to cellular immunity including T cells, other than the antibodies mentioned above, however, the details are not known.

Peripheral neuropathy is a series of symptoms caused by damage to the nerves that originate in the brain and spinal cord and extend to the periphery (peripheral nerves); and it is a disease that manifests as numbness and pain in the limbs due to a variety of causes. It is known that peripheral neuropathy is caused by infections, diabetes, and other diseases, but it is also caused by the side effects of various medications.

As the example of such medications, there are mentioned such as hyperlipidemia agents, antiviral agents, anti-tuberculosis agents, and the like, and anti-cancer drugs are especially problematic. Recently, almost half of the people in Japan have gotten cancer and one-third of the cause of death is cancer. It is known that many patients have peripheral neuropathy due to cancer therapy. In other words, it is known that chemotherapeutics are often used in the cancer therapy, and peripheral neuropathy tends to be caused as a side effect, when administering platinum-based drugs such as cisplatin, carboplatin, oxaliplatin, and nedaplatin; vinca alkaloids such as vincristine, vinblastine, and vinorelbine; taxanes such as paclitaxel and docexel; cytarabine, an antimetabolite; and ifosfamide, an alkylating agent.

For example, vinca alkaloids or taxanes as described above act on microtubules in cancer cells, however, they also supposed to damage microtubules in nerve cells, causing neurological symptoms. In addition, the platinum-based agents are considered to directly damage nerve cells and give damage to the axons of the nerve cells.

Also, anti-cancer agents such as amiodarone hydrochloride, interferon-$\alpha$, and tacrolimus are said to cause peripheral neuropathy due to damage to the Schwann cells lining the axons. In this case, axons and cell bodies are often relatively normal and good recovery can be expected with early discontinuation of drug administration. However, as the damage progresses, axons may degenerate secondarily, and recovery may not be possible.

Peripheral neuropathy described above is a side effect often seen in anti-cancer chemotherapy and it is known as chemotherapeutic agent-induced peripheral neuropathy (hereinafter referred to as "CIPN"). The structure of nerves, neurons, and its degeneration are described below.

In the case of CIPN, several means are employed to alleviate peripheral neurological symptoms such as numbness in the hands and feet: temporarily suspending anticancer agents, using calcium/magnesium in combination, or administering herbal medicines or vitamin preparations (vitamin B6, vitamin B12, vitamin complex, and the like) (Non-Patent Document 3, hereinafter referred to as the prior art 3).

## Prior art documents

### Non-patent documents

[Non-patent document 1]
Vizoso FJ, Eiro N, Cid S, Schneider J, Perez-Fernandez R. Mesenchymal Stem Cell Secretome: Toward Cell-Free Therapeutic Strategies in Regenerative Medicine. Int J Mol Sci. 2017 Aug 25;18(9):1852. doi: 10.3390/ijms18091852.
[Non-patent document 2]
Dimachkie MM, Barohn RJ. Guillain-Barre syndrome and variants. Neurol Clin. 2013 May;31(2):491-510. doi: 10.1016/j.ncl.2013.01.005.
[Non-patent document 3]
Tsai CH, Lin YH, Li YS, Ho TL, Hoai Thuong LH, Liu YH. Integrated Medicine for Chemotherapy-Induced Peripheral Neuropathy. Int J Mol Sci. 2021 Aug 26;22(17):9257. doi: 10.3390/ijms22179257.


## Summary of invention

### Problems to be solved by the invention

Prior art 1 discloses highly safe and a good technique in terms of no need for consideration of immune rejection, ethological restriction problematic in cell therapy, and low possibility of an embolus or tumor formation, because CM of MSC without the cells is used. However, when not-immortalized MSCs are cultured, their proliferation capability decreases around at most the tenth passage due to cell senescence. Therefore, MSCs shall be re-prepared from a donor, and it leads difficulty to manage the quality of the product such as continuously providing CM containing components stably.

Prior art 2 discloses a standard method for the treatment of Guillain-Barré syndrome described above, and there is intravenous immunoglobulin therapy (hereinafter sometimes referred to as "IVIg") or plasma exchange therapy. These treatment methods are performed to attenuate the effect given by anti-ganglioside antibodies. However, some patients do not respond to these therapies, although the cause is not known. Therefore, there is a strong social demand for novel therapeutic agents that provide superior effects on demyelinating diseases including Guillain-Barré syndrome.

As described above, chemotherapy against the cancer is the treatment of choice for the majority of cancer patients, and it makes possible to increase the administration amount of the agents to the maximum tolerated dose. However, increasing the administration amount of the agent often generating CIPN as a side effect. Prior art 3 is a superior method in terms of alleviating CIPN symptoms. However, since it is a symptomatic treatment, if symptom relief dose not work successfully, it requires does reduction, withdrawal, or discontinuation of medication. As a result, this leads to problems such as the progression of cancer symptoms. Also, if CIPN is not alleviated, there is another problem such as decreasing QOL of patients.

From the above, there is a strong social demand for pharmaceutical compositions that is used in cell-free therapy with high safety and fundamentally treat demyelinating diseases, as well as pharmaceutical formulations with such compositions as active ingredients.

**Means for solving the problem**

The applicants of the present invention proceeded with the development of the present invention under the above-mentioned situation and accomplished it. The purpose of the present invention is to provide a pharmaceutical composition that is used for the treatment of demyelinating diseases and a pharmaceutical preparation for demyelinating diseases, comprising a culture supernatant of immortalized mesenchymal stem cells incorporating a specific gene as an active ingredient.

The first embodiment of the present invention is the composition that regenerates myelin sheath or proliferates Schwann cells, wherein the composition contains a culture supernatant of immortalized mesenchymal stem cells to which a gene set of telomerase reverse transcription enzyme (TERT) and two or three genes selected from a group consisting of Bmi-1 gene, HPV16-E6 gene, and HPV16-E7 gene are introduced. Here, the term, regeneration of the myelin sheath, indicates the re-myelination of axons that have become bare due to the shedding of Schwann cells or the restoration of a thinned myelin sheath to its original thickness by the Schwann cell proliferative effect of the invention.

In addition, it is preferable that the gene set consists of Bmi-1 gene, HPVE16-E6, HPV16-E7, and hTERT.

Here, it is preferable that the mesenchymal stem cells are dental pulp stem cells, which are derived from humans. Also, it is preferable that the composition contains culture supernatant obtained from the immortalized mesenchymal stem cells, which are immortalized by introducing said genes with a viral vector, and by culturing them for 24 to 96 hours, preferably for 48 to 80 hours. More preferably, the culture period is about 72 hours.

Also, it is preferable that the composition contains at least a component that functions on c-MET, a hepatocyte growth factor (HGF) receptor; more preferably, it is HGF.

Another embodiment of the invention is a therapeutic pharmaceutical preparation for demyelinating diseases that contains the composition as an active ingredient. Here, it is preferable that the preparation contains from 10 to 90 % by weight of the composition, when the total weight of the preparation is 100 %. More preferably, it contains from 25 to 75% by weight.

It is preferable that the demyelinating disease is central nerve one, and anyone selected from a group consisting of multiple sclerosis, neuromyelitis optica, acute disseminated encephalomyelitis, atopic myelitis, progressive multifocal leukoencephalopathy, HTLB-1-related myelopathy, HIV-related leukoencephalopathy, subacute sclerosing panencephalitis, osmotic demyelinating syndrome and drug-induced leukoencephalopathy.

Also, it is preferred that the demyelinating disease is peripheral nerve one, and anyone selected from a group consisting of Guillain-Barré syndrome, Fisher syndrome, diabetic neuropathy, chronic inflammatory demyelinating polyradiculoneuropathy, multifocal motor neuropathy, chemotherapeutic agent-induced peripheral neuropathy, and herpes zoster.

It is preferable that the preparation has a dosage form selected from a group consisting of powders, tablets, capsules, liquids, injectables, and plasters.

The second embodiment of the present invention is a pharmaceutical preparation for the treatment of chemotherapeutic agent-induced peripheral neuropathy that comprises a composition as an active ingredient, wherein the composition contains a culture supernatant of immortalized mesenchymal stem cells to which a gene set of telomerase reverse transcription enzyme (TERT) and two or three genes selected from a group consisting of Bmi-1 gene, HPV16-E6 gene, and HPV16-E7 gene are introduced. Here, it is preferable that the chemotherapeutic agent is an anti-cancer agent, and that the anti-cancer agent is a vinca alkaloid preparation or a taxane preparation.

## Advantageous Effect of the Invention

According to the therapeutic agent of the present invention, the therapeutic agent comprising the culture supernatant of immortalized mesenchymal stem cells as the active ingredient, it relieves symptoms of or promote rapid recovery from demyelinating diseases or peripheral neuropathies, especially Guillain-Barre syndrome and CIPN. In the immortalized mesenchymal stem cell, Bmi-1, HPV-E6, HPV-E7, and TERT genes are incorporated.
Also, according to the therapeutic preparation for demyelinating diseases of the present application, it promotes the proliferation of Schwann cells, thereby restoring impaired neurological functions.

## Brief Description of the Drawings

[Figure 1] Figure 1 illustrates the myelination and neurotransmission status of neurons in normal, Guillain-Barré syndrome, and chemotherapy-induced peripheral neuropathy. (A) shows the status of the myelin sheath and neurotransmission of a normal neuron. (B) shows the status of the myelin sheath and neurotransmission of neurons developing Guillain-Barré syndrome. (C) shows the status of the myelin sheath and neurotransmission of a neuron developing chemotherapy-induced peripheral neuropathy.
[Figure 2] Figure 2 schematically illustrates the relationship between the occurrence of peripheral neuropathy and the immune system in the case of a patient with Guillain-Barré syndrome.
[Figure 3] Figure 3 shows how to produce autoimmune neuritis mice, and the changes in clinical scores when SHED-CM was administered intraperitoneally to them. Figure 3(A) shows how to produce the autoimmune neuritis model mice by administering P0(180-199) to C57BL/6 mice, and the administration schedule of DMEM or SHED-CM. Also, Figure 3(B) shows the change in clinical symptom scores, when treated with the administration schedule shown in Figure 3(A) described above.
[Figure 4] Figure 4 shows toluidine blue-stained optical microscope images (Figures 4(A1, 2) and 4(B1, 2)), and electron microscope images (Figures 4(A3) and 4(B3)) of the neuron axon and myelin sheath, when either of DMEM (Figure 4(A)) or SHED-CM (Figure 4(B)) was intraperitoneally administered to the autoimmune neuritis mice, which are produced as shown in Figure 3. The black bars in the electron microscope image are scales (10 $\mu$m).
[Figure 5] Figure 5 shows the results of expression analysis by quantified PCR. The vertical axis of Figure 5(A) shows the relative ratio of the expression level of Myelin Basic Protein (herein below, sometimes abbreviated as "MBP") divided by that of the internal standard gene, Hypoxanthine guanine phosphoribosyltransferase (herein below, sometimes abbreviated as "HPRT"). Figure 5(B) the results of Western blot showing the effect of SHED-CM on expression of MBP or Actin.
[Figure 6] Figure 6 is graphs showing the expression level of TNF-$\alpha$, IFN-$\gamma$, and IL-17A genes in neurons, when the SHED-CM is applied to them. Figure 6(A1) to (A3) show the expression level of the genes in ischiatic nerve cells. Figure 6(B1) to (B3) show the expression level of the genes in caudal nerve cells.
[Figure 7] Figure 7 is a Western blot image showing the increase in the protein amount of phosphorylated c-Met in ischiatic nerve cells by SHED-CM application.
[Figure 8] Figure 8 shows the therapeutic effects of each SHED-CM on EAN model mice produced from wild-type (herein below sometimes abbreviated as "WT") mice. Figure 8(A) shows how to produce the model mice and the schedule of SHED-CM applications to them. Figure 8(B) is the graph showing a tendency that the effect of SHED-CM on the model mice decreases by co-application of anti-HGF antibody.
[Figure 9] Figure 9 shows photographs by optical microscope images showing the appearance changes of Schwan cell strain, IMS32, when culturing in either DMEM alone or DMEM containing SHED-CM. Figure 9(A) shows IMS32 shape before the start of the experiment, Fig. 9(B1) shows that in the control containing 10 % (v/v) DMEM, Fig. 9(B2) shows that of in the control containing 30 % (v/v) DMEM, Fig. 9(C1) shows that in sample 1 containing 10 % (v/v) SCHED-CM, and Fig. 9(C2) shows that in sample 2 containing 30 % (v/v) SHED-CM, respectively.
[Figure 10] Figure 10 shows the graphs showing the change in proliferation of IMS32, when it is cultured in the medium containing SHED-CM. Figure 10(A) shows a comparison of the proliferation under 1 to 30 % (v/v) DMEM or DMEM containing SHED-CM. Figure 10(B) shows the change in the proliferation, when they are cultured in the medium used in Figure 10(A), to which an anti-HGF antibody or reference antibody is added.
[Figure 11] Figure 11 shows the graphs showing cell proliferation cultured in DMEM containing 10 to 50 % (v/v) SHED-CM, or DMEM containing 0.1 to 50 ng/mL HGF under the presence or absence of FBS. Figures 11(A) shows the change of the cell proliferation of IMS32 under the absence of FBS, and Fig. 11(B) shows the change thereof under the presence of 1 % FBS, respectively.
[Figure 12] Figure 12 shows the results of the Western blot after gel electrophoresis showing the amount of the phosphorylated ones (c-met, ERK1/2, or AKT) or the total proteins in IMS32, which is stimulated by either of SHED-CM or HGF.

[Figure 13] Figure 13 shows the images of the results of the Western blot showing the protein amount both of MBP and p75, which are produced by IMS32 stimulated with SHED-CM, HGF, NRG1, DBcAMP, or NRG1 + DBcAMP in DMEM containing 5 % FBS.

[Figure 14] Figure 14 is the graph showing the therapeutic effect given by SHED-CM on the chemotherapy-induced peripheral neuropathy model mice produced from WT mice. The graph shows that the application of SHED-CM to the model mice of treatment group to which 80 μg/200 μL paclitaxel is intraperitoneally injected every other day mitigates neurological disorder and cures CIPN earlier than those of the control group to which DMEM is injected as the control.

[Figure 15] Figure 15 is a table showing the filament pressure strength and accompanying information (cited from the Dinamic Global, LLC commentary).

[Figure 16] Figure 16 is the table 1 showing k value used for calculation of von Frey filament test.

[Figure 17] Figure 17 is the table 2 showing k value used for calculation of von Frey filament test.

**Mode for carrying out the invention**

One embodiment of the present invention is explained below, referring to Figures 1 through 17. In the following description and drawings, identical or equivalent elements are marked with the same symbol, and redundant explanations are omitted.

The first embodiment of the present invention, the therapeutic composition using conditioned medium (CM) of mesenchymal stem cells, is produced by the following procedures: that is, (S1) generating immortalized stem cells by incorporating a specific gene set into human dental pulp stem cells, (S2) preparing a supernatant of the culture medium by culturing the immortalized stem cells obtained above, (S3) producing the composition that induce regeneration of myelin sheath or proliferation of Schwann cells, using the culture supernatant as the active ingredient.

Here, we describe the use of dental pulp stem cells as an example of mesenchymal stem cells. Dental pulp stem cells are defined as a type of stem cells contained in the nerves of the tooth having regenerative capability. Since they are protected by the hard material, the tooth, they are not exposed to ultraviolet rays or radioactive rays, they have features that their genes are not easily damaged.

Also, the gene set is consisted of bmi-1, HPV-E6, HPV-E7, and TERT. TERT is a gene for the telomere regeneration enzyme. bmi-1 is a gene of a protein Bmi-1 that consists of a polycomb complex. Here, Bmi-1 is needed for maintaining hematopoietic stem cells and its activation leads to the proliferation of hematopoietic stem cells. E6 and E7 are early genes of HPV-16 or HPV-18 DNA.

Firstly, the human deciduous tooth is disinfected with a disinfectant, for example, such as chlorhexidine, isodine solution, or others. After that, the crown section of the tooth is divided, and the dental pulp tissue therein is collected by using a dental reamer. The collected dental pulp tissue is suspended in a basic medium such as Dulbecco’s Modified Eagle’s medium (Dulbecco’s Modified Eagle’s Medium, hereinbelow referred to as "DMEM") containing 5 to 15% calf serum (hereinbelow, referred to as "CS") and 50 to 150 units/mL of antibiotic. Then, they are treated with 1 to 5 mg/mL collagenase and 1 to 5 mg/mL dispase at 37 °C for 0.5 to 2 hours.

As the basic medium, in addition to DMEM, there are mentioned such as Iskov’s modified Dulbecco’s medium (IMDM) (GIBCO, etc.), Ham F12 medium (Sigma, GIBCO, etc.), RPMI 1640 medium, and the like. Two or more basic media may be used in combination. An example of a mixed medium, there is mentioned such as a mixture of equal amounts of IMDM and HamF12 (e.g., commercially available as the product name: IMDM/HamF12 (GIBCO)).

Also, as supplements for the basic medium, there are mentioned such as fetal calf serum (herein below, referred to as "FCS"), human serum, sheep serum, other sera, serum substitutes (e.g. Knockout serum replacement (KSR)), bovine serum albumin (herein below, referred to as "BSA"), penicillin, streptomycin and other antibiotics, various vitamins, various minerals, and the like.

The basic culture media may be also used for cell selection and for cell culture after the selection as described later.

After enzymatic treatment thereof, the samples are centrifuged for 3 to 10 minutes (3,000 to 7,000 rpm) to collect the dental pulp stem cells. Depending on the necessity, the cells are sorted by using a cell strainer. The sorted cells are resuspended in, for example, 3 to 6 mL of the basic medium and spread into adherent cell culture dishes having 4 to 8 cm in diameter.

Next, a culture medium such as DMEM containing 10% FCS is added therein, and then the cells are incubated in a 5% $CO_2$ incubator at 37 °C for around 2 weeks. After removal of the culture medium, the cells are washed one to several times with PBS or the like. Instead of removing the culture medium and washing the cells, the adherent dental pulp stem cells that forms colonies may be collected. For example, the adherent dental pulp stem cells are detached from the dish by treatment with, for example, 0.025 to 0.1% trypsin and 0.3 to 1 mM EDTA at 37 °C for several minutes, and then the detached cells are collected.

Next, the adherent cells selected as above are cultured. For example, the dental pulp stem cells obtained as described above are inoculated into adherent cell culture dishes and cultured in an incubator under the condition of 5% $CO_2$ and 37 °C.

For example, when the cells reach subconfluent or confluent as observed by the naked eye, cell passage is conducted by detaching them from the culture vessel, collecting them by using trypsin and EDTA as described above, and then again inoculating them into a culture vessel with the culture medium.

Here, the term, subconfluent is defined as cells are attached to the culture vessel, which is covered approximately 70% by the cells. For example, passaging culture is conducted 1 to 8 times, and the sorted cells are grown to the required cell number, for example, about $1 \times 10^7$ cells/mL. After culturing as described above, the cells are collected and stored in liquid nitrogen. Cells collected from various donors may be stored in the form of a dental pulp stem cell bank.

Then, primary culture cells obtained from the primary culture of the stem cells are transfected with three or four different genes to produce transgenic cells. It is preferable to transfect two or three different genes selected from the group consisting of hTERT, bmi-1, E6, and E7, because they give immortalized stem cells with a higher number of population doublings. Here, hTERT is a gene of human telomerase reverse transcription enzymes; bmi-1 is a polycomb group gene relating to self-replication and differentiation regulation of the stem cells; and both E6 and E7 are genes existed in an open reading frame coding early genes that are used for self-replication by human papillomaviruses.

Transfection of these genes is conducted as follows.

A plasmid to incorporate the gene of the interest is prepared, and it is incorporated into a shuttle vector such as pSuttle2 to clone the genes. The vector is used for E. coli transformation, from which kanamycin-resistant transformants are selected. The plasmid DNA of the selected kanamycin-resistant transformants is purified and subjected to analysis of the restriction enzyme sites to identify the recombinants.

1. Processes for preparation of DNA fragments and virus vectors

1.1 Preparation of DNA fragments for virus incorporation

First, sequence information on the vector used to incorporate the DNA fragments into the virus is obtained. The virus used as a vector for gene transfer in the present invention is preferably one selected from the group consisting of lentiviruses, adenoviruses, and retroviruses, because the introduced gene is expressed in a non-transient manner.

The following is an example of using a lenti-plasmid vector (pLVSIN). pLVSIN sequence information is downloaded from, for example, the Takara Bio Web Catalog, and the multicloning site is checked.

Then, DNA fragments are prepared as below. In the present invention, immortalized stem cells are generated by incorporating 2 to 4 genes into the DNA fragments. The cells used are not limited to stem cells obtained from mammals, but porcine dental pulp tissue, porcine adipose tissue, and human dental pulp tissue are preferred because they are easy to obtain, and immortalized stem cells with stable phenotypes can be obtained.

It is also preferred that the genes introduced here be a set of at least two or more genes selected from the group consisting of two papillomavirus early genes, telomerase reverse transcriptase (TERT), and bmi, because this may produce immortalized stem cells with stable traits. As the early genes of papillomavirus, human papillomavirus (sometimes abbreviated as "HPV") E6 and human papillomavirus E7 are preferable due to expression efficiency. As TERT, human TERT (sometimes abbreviated as "hTERT") is preferable due to gene expression efficiency.

Of the genes, TERT is a gene that encodes an enzyme that elongates telomere sequences, which become shorter with age. Both human papillomavirus E6 and E7 are early genes of human papillomavirus, and E6 is known to reactivate TERT and degrade proteins with PDZ domains. Bmi-1 is the polycomb group gene and known to relate the self-replication and differentiation regulation of the stem cells.

The following is an example for the explanation to introduce three genes into the cells by using a lentiviral vector. In order to incorporate the human papillomavirus E6 gene, human papillomavirus E7 gene, and telomerase reverse transcriptase (TERT), double-strand DNA (SEQ. ID No. 1) is synthesized according to sequence information, for example, EcoRI/KoZal/E6/T2A3/E7/T2A4/hTERT/BamHI, by standard procedures. The DNA fragments obtained are cloned into the multicloning sites of the lentiviral plasmid vectors (pLVSIN-CMV Neo) by conventional procedures to obtain plasmid vectors (E6E7T) for lentiviruses in which the three genes are introduced.

In the case of retroviruses, different from lentivirus vectors, the genes are introduced by co-transfection of prepared vectors having one of the genes. For example, five immortalized genes (hTERT (SEQ. ID No. 2), human papillomavirus E6 (SEQ. ID No. 3), human papillomavirus E7 (SEQ. ID No. 4), pigTERT (SEQ. ID No. 5), and hBmi-1 (SEQ. ID No. 6)) with Kozak sequence (gccacc) upstream of the start codon may be prepared according to the conventional procedure and cloned into the PmaC I-Hpa I site of pDON-5 NEO DNA (TaKaRa Code 3657) according to the conventional procedure to prepare a retrovirus plasmid vector (pDON-5 Neo hTERT vector, pDON-5 Neo HPV16E6 vector, pDON-5 Neo HPV16E7 vector, pDON-5 Neo pHTERT vector, and pDON-5 Neo hBmi1 vector).

After that, E. coli is transformed according to the conventional procedures by using the five types of plasmid DNA prepared as described above, and the resulting transformants are cultured in a $CO_2$ incubator to obtain the plasmid DNA for the transfection grade.

Next, G3T-hi cells are inoculated at 5.5 to 6.5 $\times$ $10^6$ cells/dish in desired plates, and they are incubated in a 5 % $CO_2$ incubator at 37 °C for about 20 to 28 hours. Then, a desirable amount of a transfection reagent (for example, 0.3 to 0.5 mL of TransIT-293) is added to the culture dishes. Subsequently, 4 plasmid vectors including either human or pig-derived TERT are chosen from 5 plasmid vectors obtained as described above. These are used for co-introduction with both pGP and pE-Ampho (both are the vectors provided as the appendix of Retrovirus Packaging Kit Ampho), and further cultured under the same condition as that employed in the preceding conducted culture for 40 to 56 hours for introduction of the genes into the cells.

1.2 Preparation of vector-producing cells

In parallel with the above-mentioned work, recombinant lentiviral vector-producing cells are prepared. As the example of the cell lines, Lenti-X 293T (Clontech, Code No. 632180) and other commercially available cell lines may be used. In order to culture of Lenti-X 293T, medium containing both fetal bovine serum and antibiotics may be used. As these media, there are mentioned such as, for example, minimal medium (MEM), DMEM, and the like. For example, DMEM (Sigma, St. Louis, MO) containing 5 to 15% fetal bovine serum (FBS, Hyclone) and 0.5 to 2% antibiotics (penicillin/-streptomycin, Gibco) is preferably used because of cell growth efficiency.

For example, the medium, DMEM supplemented with 10 % FBS and 1 % penicillin/streptomycin, is prepared, and it may be employed as the basic medium. Herein below, in the specification, the medium is referred to as "293T basal medium".

For example, when Lenti-X 293T is used as the cell strain, the cell suspension including the cells at 1 to 5 $\times$ $10^5$ cells/mL is prepared, and 10 mL of the suspension is poured into 10 cm dishes. Then, the dishes are incubated in a 5 % $CO_2$ incubator for 24 hours. After that, the cells in the dishes are passaged depending on the cell condition until use. When using the cells, firstly, the culture supernatant of the cells experiencing several passages are removed. Then, the cells are washed with PBS, and are detached from the bottom surface of the dish by using commercially available detachment reagent to be collected. The cell suspension is diluted to 1/3 or 1/5 and desirable volume of the portion is taken from the diluted suspension to which trypan blue solution is added to count viable cell number with a hemacytometer.

The cell suspension at 5 $\times$ $10^5$ cells/mL is prepared by adding the basal medium, and then, for example, the cell suspension is inoculated into dishes so as to be the desirable concentration and incubated under the desirable conditions. For example, the cells are placed in the dishes having a diameter of 6 cm so as to 1 to 4 $\times$ $10^6$ cells/4mL, and are incubated in the 5 % $CO_2$ incubator at about 37 °C for about 24 hr. The culture supernatant is replaced with fresh one, and then they are further cultured.

It is preferable to use G3T-hi cells (produced by TAKARA Bio), a high expression cell line of GnT-III as cells for retrovirus preparation, which is generated by introducing human N-acetylglucosaminyltransferase III (GnT-III) with the hygromycin-resistant genes into the human kidney-derived cell line 293T (G418 resistant).

G3T-hi cells are designed to produce high titer recombinant virus promptly and transiently by co-introducing an expression vector harboring gag-pol and env genes, and recombinant retrovirus vector plasmid harboring objective genes with Retrovirus Packaging Kit Eco or Ampho (both are produced by Takara Bio, product code Nos.: 6160, 6161). Since G3T-hi cells are derived from 293T cells, it has an introduced T-antigen gene of SV40, thereby retroviral RNAs are amplified to produce high-titer virus solutions. In general, the transient expression by using a Retrovirus Packaging Kit gives the solutions containing $10^5$ to $10^7$ cfu/mL viruses.

In G3T-hi cells, glycans on the cell surface are modified by GnT-III. It is assumed that the glycan on the membrane proteins of the recombinant retroviruses obtained from the G3T-hi cells are modified by GnT-III, because budding retroviruses wear the host cell membrane. The glycan modification enhances the affinity of the retrovirus prepared with the G3T-hi cells to RetroNectin (recombinant human fibronectin filament). Therefore, the use of RetroNectin as a coating agent for the culture dish highly improves the efficiency of gene introduction into target cells. In particular, RetroNectin effectively functions for gene introduction, targeting into hematopoietic cells.

When using the G3T-hi cells, it is preferable to use DMEM containing glucose (4.5 g/L), L-glutamine (584 mg/L), supplemented with 10 % FBS and 1 % penicillin/streptomycin, instead of 293T basal medium due to the production efficiency of the retroviruses.

1.3 Preparation of the virus vector

Herein below, it is explained by using the lentivirus vector as an example.

The co-transfection is conducted by adding the lentivirus vector plasmids prepared as described above into the dishes, wherein the cells are cultured as explained above. For such a co-transfection, commercially available packaging systems, for example, Lenti-X HTX packaging system (Clontech Laboratories Inc.) and the like may be used. Concrete procedures may be conducted according to manuals attached to them.

After co-transfection, the medium in the dish is replaced with fresh complete medium and incubated at about 37 °C for 24 to 28 hours. The virus vector is titrated to decide the harvesting time point, and the culture supernatant including the virus vector is collected when the virus titer becomes maximal. For the titration of the virus vector, the commercially available convenient titration kit is used. As the examples of such kits, there are mentioned such as Lenti-X GoStix (Clontech Laboratories Inc.) and the like.

For example, the day after the medium change, the culture supernatant in the petri dish is collected by using the desired size of a syringe. The collected supernatant is filtrated to obtain the virus vectors. For example, the supernatant is taken out by using 10 mL of disposable syringe (Terumo Corporation), and then, it is filtrated by using a filter, for example, 0.45 μm of the filter (MILEX-HV, Millipore limited), attached to the syringe. By this, the virus vector solution is collected into a tube, for example, 15 mL of a tube.

Next, reagents included in the commercially available convenient titration kit are used to confirm the presence of the recombinant lentivirus vector. For example, the desirable volume of the virus vector solution, for example, 20 μL, is added to S of the Lenti-X GoStix, and Chase Buffer 1 is further added to the solution. Then, the solution is reacted at the desirable temperature and time period, for example, 10 minutes. The presence of the recombinant lentivirus vector is confirmed with/without the appearance of bands.

Cells harboring E6E7T plasmid obtained as described above are streaked on agar plates. Formed colonies on the agar are picked up for plating into the desirable medium, for example, 2 mL of LB medium containing antibiotics, and then they are vigorously cultured in a shaker at about 37 °C for 8 hours. Next, the desired amount of cultivated solution of the cells as described above, for example, about 100 μL, is added into LB medium, for example, 50 mL of LB medium, supplemented with different antibiotics, for example, ampicillin. Then, the medium is vigorously cultured in a shaker at about 37 °C for about 14 to 18 hours.

By using the commercially available kit, for example, MN NucleoBond Xtra Mid Kit (Clontech Laboratories Inc.), the amount of plasmid eluted with water is measured. Together with this procedure, the plasmid is analyzed by using a desirable gel, for example, about 1 % agarose gel for confirming that the inserted DNA is mutually different. As the marker for the gel electrophoresis, for example, supercoil DNA ladder, λ-Hind II digested DNA, and the like may be used. As described above, the virus vector is prepared.

2. Preparation of mammal stem cells

2.1 Preparation of the swine dental pulp stem cell

Swine jaw (lower jaw having teeth) from the swine of 5 to 6 months old is obtained. According to the following procedure, swine dental pulp stem cells are obtained from the swine teeth and the swine lower jaw.

Firstly, the swine teeth and the lower jaw are disinfected using an appropriate disinfection agent such as Isodine. Then, a crown of the tooth is cut in the horizontal direction by using, for example, a diamond point for a dentist and the like. Then, the dental pulp is collected from both of the dental crown and dental root portions treated as mentioned above by using, for example, a hand scaler for the dentist and the like.

Obtained dental pulps are chopped, for example, by using an ophthalmic knife to be suspended in a predetermined concentration of collagenase solution, for example, 1 to 3 mg/mL, to stand for desired time period, for example, 1 hour, in the incubator at about 37 °C to isolate the cells in a single cell. Then, they are preliminary cultured in desired medium, for example, DMEM containing 10 % FBS and 1 % Anti-Anti (Antibiotic-Antimyotic, Invitrogen, Carlsbad, CA) at about 37 °C in a 5 % $CO_2$ incubator to obtain the cells for passage.

Until the cells are cultured in the dish to reach sub-confluent, the medium is replaced 2 to 3 times per week. Then, the cells reaching sub-confluent state are detached from the dish using a detaching agent such as Hepes solution containing 0.05 % trypsin. After that, the cells are collected by centrifugation under the desirable conditions, for example, at 1,500 rpm for about 3 minutes at room temperature. The obtained cells are transferred into the fresh medium. Then, desirable amount of the obtained cells, for example, entire volume, is provided to cell passage.

2.2 Preparation for dental pulp stem cells from human exfoliated dens deciduous teeth

Exfoliated or extracted dens deciduous teeth are obtained from healthy children. These dens deciduous teeth are disinfected by using an appropriate disinfection agent, for example, Isodine solution, and then the dental pulp tissues are collected in the same as those for obtaining the swine dental pulp tissues. Obtained dental pulp tissues are digested in the solution including the desired concentrations of collagenase and dispase, for example, about 3 mg/mL of type I collagenase and about 4 mg/mL of dispase at the desired temperature for the desired time, for example, about 37 °C for about 1 hour. Next, the solution is filtrated by using, for example, 70 mm of a cell strainer (Falcon) to separate cells.

Thus, filtrated cells are re-suspended, for example, in 4 mL of above-mentioned medium, and spread on culture dishes for adherent cell culture with desirable diameter, for example, 6 cm. Desirable medium, for example, DMEM supplemented with about 10 % FCS, is added to the dishes and cultured for a desirable period, for example, around 2 weeks in the incubator under the conditions of 5% $CO_2$, at 37 °C. Adherent cells formed colonies, the dental pulp stem cells, are treated with the desirable releasing agent, for example, about 0.2 mM EDTA containing about 0.05 % of trypsin for a desirable time period, for example, 5 minutes, at about 37 °C to collect the cells released from the dishes.

Next, the adherent cells collected as described above are spread, for example, in the dishes for the adherent cells (collagen-coat dish), and they are primarily cultured in an incubator under the conditions of, for example, 5% $CO_2$, at 37 °C to obtain primary cultured cells. When the cells become sub-confluent or confluent by macroscopic observation, the cells are treated the same as those described above by using the releasing agent to collect the cells in the dish.

After that, the primarily cultured cells are passaged by using the medium at the desirable concentration, for example, about $1 \times 10^4$ cells/cm$^2$. The cells passaged 1 to 3 times are employed in experiments. As described above, human exfoliated dens deciduous dental pulp stem cells (SHED) may be obtained.

Depending on the necessity, the dental pulp stem cells from the swine dens deciduous dental pulp stem cells as described above and the dental pulp stem cells from the human exfoliated dens deciduous dental pulp stem cells may be respectively poured into vials to be cryopreserved -80 °C.

3. Preparation of gene-introduced cells

3.1 Gene introduction by the lentivirus vector

The swine dental pulp stem cells and the human dental pulp stem cells obtained as described above are cultured as mentioned above, respectively. When the cells reached about 70 to 90 % confluent, they are subjected to mycoplasma check, which is conducted with a commercially available kit. As a such kit, for example, MycoAlert Mycoplasma Detection Kit (Lonza, LT07-318) and the like may be used. Next, the lentivirus vector prepared as described above infects the cells respectively by using Polybrene reagent. Then, the cells are selected under the selection pressure of the reagent to obtain the strains stably expressing the target genes.

The culture supernatants of the cells cultured as described above are removed, and then the cells are washed, for example, with PBS (pH 7.4), respectively. After that, the cells are detached from the dishes using a desirable releasing agent, and then collected, respectively. As the releasing agent, for example, StemPro (Registered trademark: GIBCO) may be used for the swine dens deciduous dental pulp stem cells; and trypsin - EDTA and the like may be used for human dental pulp stem cells.

Obtained cells are counted according to the conventional method, and then they are spread into each well of the plate, for example, a 6-well plate with tissue culture treatment (T.C. treatment) (CORNING) so as to be about $1 \times 10^5$ cells/well. They are incubated in the $CO_2$ incubator at about 37 °C for about 24 hours to confirm even distribution of the cells in the well.

After that, the culture supernatants are removed. The desired volume of the desired medium containing the desired concentration of Polybrene, for example, the desirable amount of DMEM containing about 8 μg/mL of Polybrene and about 10 % of FBS, is added for the dental pulp stem cell of the swine dens deciduous dental pulp stem cells at 750 μL/well. Next, the lentivirus vector solution described above is added into each cell, for example, at about 250 μL/well.

When the human dental pulp stem cell of the dens deciduous teeth is used, DMEM containing the same amount of Polybrene and FBS is added into the wells at a desirable amount, for example about 1.2 mL/well. Then, the lentivirus vector solution is added to each well at the desirable amount, for example, about 400 μL/well.

Each well of the plate to which the virus vectors are added as described above is centrifuged under the desirable conditions such as at about 1,000 x g for about 30 minutes, at about 32 °C for virus infection to the cells. After that, they are cultured under the desirable conditions such as at about 37 °C in a $CO_2$ incubator for about 4 to about 6 hours; and a desirable volume of the fresh culture medium, for example, about 1 mL/well, is added into each well. Then, they are cultured under the desirable conditions, for example, at 37 °C in the $CO_2$ incubator for about 24 hours to conduct the gene introduction.

3.2 Selection under the presence of the agents

The culture supernatants of the cultured stem cells (namely, the swine dental pulp stem cells and human dental pulp stem cells), which are cultured as described above, are removed from each well of the culture plate. Then, the desirable concentration of the agents for selection, for example, selection medium containing either about 0.4 mg/mL or about 0.8 mg/mL of GENETICIN (G418, GIBCO) is added into each well in a desirable volume, for example, about 2 mL/well to exchange the culture medium. After that, the cells are cultured for a desirable period, for example, about 3 to 5 days exchanging the culture medium for selecting the gene-introduced cells.

A portion of the cultured cells is subjected to cloning, and the remains are successively cultured in the selection medium as pool cells. The cloning may be conducted as follows.

The selection medium without antibiotics, for example, the medium without penicillin, streptomycin, and G418 is used for dilution of the cells to a desirable concentration, for example, either about 1 × $10^3$ cells/4 mL or about 5 × $10^3$ cells/4 mL. They are plated in each dish and are incubated at about 37 °C in the $CO_2$ incubator for about 24 hours. Formed colonies are marked from the rear side of the dish until they reach the desirable number, for example, about 100. Then, the culture supernatant in the well is removed, followed by washing the cultured cells with PBS. Cloning rings are set in the dishes and the cells in each colony which are detached by the releasing agent are respectively spread at a desirable amount in each well in a 48-well plate, containing the desirable volume of the medium, for example, about 1 mL.

3.3 Preparation of total RNA

In order to confirm the gene expression, total RNA is prepared with, for example, NucleoSpin RNA II (a kit provided by MACHEREY- NAGEL). It is preferable to use the buffers, the ring filter, and the like employed below, which are included in the kit, and to conduct the procedure according to the manual provided in the kit due to obtaining total RNA with high purity.

Cell lysate is prepared by lysing a cell pellet being prepared from the desirable number of cells, for example, about 5 × $10^5$ cells in the collection tube. The lysate is poured onto the ring filter with a purple color, and then, it is centrifuged under the desirable conditions, for example, at about 11,000 x g for about 1 minute. After the centrifugation, the filter was discarded, and then, the desirable amount of ethanol, for example, about 350 μL of about 70 % ethanol is added into the collection tube. Then, they are mixed with pipetting in desirable numbers such as about 5 times in the tube.

Next, the desirable amount of the obtained solution, for example about 700 μL, is loaded on the ring column with a pale blue color set in the collection tube. Then, the tube is centrifuged under the desirable conditions such as about 11,000 x g for about 30 seconds to bind RNA. After the centrifugation, the column is set in the new collection tube. Then, the desirable reagent such as about 350 μL of MDB is added into the tube; and then it is centrifuged under the desirable conditions such as at about 11,000 x g for about 1 minute for desalination.

After the centrifugation, the DNA reaction mixture is prepared by mixing lightly, for example, the desirable amount, 10 μL of rDNase, and the desirable amount of DNase reaction buffer, for example, about 90 μL. The desirable amount of the mixture, for example, about 95 μL is added to the column, and incubated under the desirable conditions, for example, at room temperature for about 15 minutes to digest DNA therein. Subsequently, the desirable amount, for example, 200 μL of buffer RA2 is added to the column and they are centrifuged under the desirable conditions such as about 11,000 x g at about 30 seconds to wash out. Subsequently, the column is set in the new collection tube, and the desirable amount such as about 700 μL of buffer RA3 is added to the column for further centrifugation under the desirable conditions such as about 11,000 x g for about 30 seconds.

Subsequently, the eluted solution in the collection tube is discarded, and the desirable amount, for example, about 250 μL of the buffer RA3 is again added to the column for the centrifugation under the desirable condition, for example, at about 11,000 x g for about 2 minutes. Then, a silica membrane of the column is air-dried. The column is set in the desirable size such as about 1.5 mL of the collection tube, and the desirable amount of RNase-free water, for example, about 60 μL, is added to the column. Then, the column is centrifuged under the desirable conditions such as about 11,000 x g for about 1 minute to obtain the total RNA sample.

3.4 Reverse transcription

The total RNA sample obtained as described above is subjected to reverse transcription. Subsequently, it is subjected to real-time PCR to obtain PCR products. In order to conduct the reverse transcription, for example, a commercially available kit such as PrimeScript RT reagent Kit (Perfect Real-time, TaKaRa Bio) is used, and reverse transcription is conducted according to the manual attached thereof.

The real-time PCR is conducted by using the commercially available kit, for example, SYBR Premix Ex Taq and the like. Also, as the standard genes used here, there are mentioned, for example, swine β-actin, human β-actin and the like. As primers, there are mentioned, for example, those listed in the SEQ ID Nos. 7 to 12 are prepared for use.

Firstly, Premix for real-time PCR is prepared by mixing the desirable amount of followings: for example, about 350 μL of SYBR Premix Ex Taq II (x2), about 28 μL of Primer mix, and about 266 μL of redistilled water. Next, the desirable amount, for example, about 23 μL of the Premix is poured into each tube for real-time PCR, and about 2 μL of the template cDNA is added into each tube. Then, real-time PCR is conducted under the desirable conditions, for example, at about 95 °C for about 30 seconds, about 40 cycles of about 95 °C for about 5 seconds and subsequent about 60 °C for about 30 seconds. Subsequently, amplified products are denatured under the desirable conditions, for example, at about 95 °C for about 15 seconds, at about 60 °C for about 30 seconds, and at about 95 °C for about 15 seconds to obtain a melting curve. For the human dental pulp stem cells, the same procedures are conducted.

Based on the standard curve prepared as described above, the expression levels of the genes in the cells, wherein the genes are introduced by using each virus vector, are obtained. By calibrating with internal standard genes, accurate results are obtained.

In each gene-introduced stem cell, it is confirmed that the introduced genes are expressed. By the confirmation, the introduction efficiency of the genes by using each plasmid is obtained. Next, the gene-introduced cells (hereinbelow, they are sometimes referred to as "introduced-gene stably expressing cell population" or "pool cells".) are subjected to single cell cloning.

Firstly, the pool cells are plated in the desirable size dish with the desirable numbers, for example, into the dish having 60 mm diameter with about $1 \times 10^3$ cells/dish, and they are cultured in a $CO_2$ incubator at the desirable temperature and time period, for example, at 37 °C for 24 hours. After that, the culture supernatant is exchanged to the growth medium containing the selection reagents described above, and further continued to culture to form colonies. Every formed colony is individually detached using both the cloning ring and the releasing agent, and then they are spread on a desirable plate, for example, a 24-well plate.

After growing the spread cells, they are grown in the culture dishes and the like to culture in larger scale. By this, the cells which express the introduced-gene stably are obtained as single clones. Next, the obtained clone is cultured for growth as described above to obtain total mRNA. The obtained total RNA is used as the template for reverse transcription by using a desirable kit, for example, Prime Script RT reagent Kit to obtain template cDNA.

After that, real-time PCR is conducted similarly to those described above; for example, by using cDNA (the reverse transcription products) as the template, SYBR Premix Ex TaqII (Tli RNaseH Plus) as described above, and the primers (specific primers for the transduced genes or the internal standard genes respectively, for example, the primers shown as SEQ ID NO. 7 to 12).

Ct value, which is calculated from the secondary differentiation curve of the real-time PCR, is plotted on the X-axis, and the relative total RNA value is plotted on the Y-axis to prepare the standard curve to determine the amounts of each gene expression. The relative expression values of the introduced genes expressed in the swine dental pulp stem cell or those derived from human dental pulp stem cells are obtained, when the expression amounts thereof on pool cells are set as 1. By this, it is confirmed that whether entire introduced genes are expressed or not, and their expression amounts, which are used for the selection of objective clones.

As described above, both the immortalized stem cell population and the clones therefrom, which are cloned from the immortalized stem cells therefrom, are obtained.

A certain transformant is selected from the obtained transformants, for example, ampicillin is used to obtain ampicillin -resistant (herein below referred to as "Amp+") transformant. Then, the Amp+ transformant is purified, and identified by analyzing restricted enzyme sites. Next, the recombinant adenoviruses are digested with PacI to obtain their fragments, which are used to transfect to HEK293 cells for amplification thereof; and then the viruses described above is titrated. According to the conventional method, the virus is purified and transfected to the target cells, dental pulp stem cells.

The virus-transfected HEK293 cell population is stained with FITC according to the conventional method to detect STRO-1-positive cells among the population by using the flow cytometer. STRO-1 is considered as the maker of MSCs having the pluripotent capability in bone marrow, and it is an index of cell immortalization. By following the protocol as described above, the immortalized stem cells derived from dental pulp are obtained.

Next, the obtained immortalized stem cells are cultured in the basal medium described above, for example, DMEM supplemented with 10 % FBS under the conditions of 5 % $CO_2$ and 37 °C for 24 to 48 hours to obtain the conditioned medium. The conditioned medium is collected by using a pipette such as the Komagome pipette.

The immortalized stem cells secret growth factors such as Insulin-like growth factors (IGFs), Vascular Endothelial Growth Factor (VEGF), Tissue Metalloproteinase Protein (TIMP), Hepatocyte Growth Factor (HGF), and the like into the culture medium. Here, the term, "growth factor", is a generic name of polypeptides to promote cell division, to alter the cell morphology, or to enlarge the cells. The factors vary depending on the origin of the production cells. They are classified into groups such as Epidermal growth factor (EGF), fibroblast growth factor (FGF), nerve growth factor (NGF), and tumor growth factor (TGF), and the like.

Furthermore, the receptors on the cell membrane of each cell have tyrosine kinase activity. Upon binding growth factors, they phosphorylate tyrosine residues in proteins, which is a trigger of cell proliferation, differentiation, and the like. Some examples that the growth factors function as mesoderm inducers during ontogeny are known. Also, some examples that lymphokines, which regulates immune system, function as the mesoderm inducers during ontogeny are known. These growth factors may be determined by using ELISA or microarray method, both of which are publicly known.

IGF is a polypeptide of which sequence is highly similar to that of insulin, and cause reactions such as mitosis induction similar to those of insulin in cell culture. They are also known to affect neuronal growth. VEGF is a member protein of glycoprotein group relating to vasculogenesis for forming new vascular in the area without blood vessels; and angiogenesis for forming blood vessels by branching and elongating from already existing blood vessels in the embryonic stage. HGF has a variety of bioactivities against hepatocytes and other cells such as promoting cytotropism, anti-apoptosis (cell death), induction of morphogenesis, angiogenesis, protection and regeneration of other tissues and organs.

The conditioned medium containing the growth factor is obtained by culturing each stem cell described above, for example, in DMEM supplemented with 15 % FCS at 37 °C for the desirable time period. Note that the conditioned medium of the stem cells contains not only IGF, VEGF, and HGF, but also other many proteins.

15 mL portion of the obtained conditioned medium are placed into an Amicon Ultra Centrifugal Filter Units-10K (Millipore) and is concentrated to 200 μL by centrifugation at 4,000 x g for about 60 minutes. Next, equal volume of the sterilized PBS to that of the conditioned medium is added into the tube, and then it is centrifuged at 4,000 x g for about 60 minutes again to replace the solvent to PBS. 200 μL portion of the obtained solution is collected into a micro test tube as the concentrated stem cell conditioned medium.

Instead of the method using the Amicon, ethanol precipitation may be used for concentration of the medium. For example, 45 mL of 100 % ethanol is added to 5 mL of the conditioned medium and mixed together, and then stood at -20 °C for 60 minutes. After that, the mixture is centrifuged at 15,000 x g at 4 °C for 15 minutes, and then the supernatant is discarded.

Next, for example, add 10 mL of 90 % ethanol to the residue in the tube, and mixed together well, and it is centrifuged again at 15,000 x g for 5 minutes at 4 °C. The supernatant is removed, and the resulting pellet may be dissolved in, for example, 500 μL of sterilized water. After dissolution, the entire volume thereof is collected into the micro-test tube as the concentrated stem cell conditioned medium. By lyophilizing the culture supernatant obtained as described above according to the conventional method, a composition for the treatment of demyelinating diseases and peripheral neuropathy, prepared for use, may be obtained in a particle powder form.

The composition is preferable to regenerate myelin sheath or to induce the proliferation of Schwann cells, because the composition cures or alleviates demyelination diseases and peripheral nerve disorders. Here, "Schwann cells" are cells that exist around peripheral nerve cell axons and cover both of the axons and axon terminals. "Myelin (myelin)" is a structure formed by Schwann cells wrapping around the nerve cell axon and assists the nerve cells' electrical signal transmission through an insulating effect. In addition to the insulating effect, it is also known that cytokines secreted by Schwann cells are involved in neuronal regeneration. Schwann cells may be obtained by primary culture from various experimental animals or by purchasing commercially available cell lines such as IMS32, IFRS, or the like.

As the immortalized mesenchymal stem cells, there are mentioned such as dental pulp stem cells, bone marrow mesenchymal stem cells, adipose mesenchymal stem cells, umbilical cord and cord blood mesenchymal stem cells, and the like. However, dental pulp stem cells are preferably used, because they have high capability to differentiate into nerves. In particular, deciduous teeth are preferably used, because the large number of undifferentiated cells are obtained from them and have high efficacy of regeneration of the pulp sheath or Schwann cells mentioned above.

The composition induces regeneration of myelin sheath or proliferation of Schwann cells obtained as described above (hereinafter sometimes simply referred to as "composition for regeneration"). Among them, it comprising at least an ingredient acting on the receptor c-MET of hepatocyte growth factor (HGF) is preferably used, because HGF and the like activate Schwann cells to promote regeneration of peripheral nerves.

It is preferable that the pharmaceutical preparation for the treatment of demyelinating diseases and peripheral neuropathy of the present invention comprises the above-mentioned composition for regeneration as an active ingredient because of its high therapeutic effect. It is also preferable that the content of the composition for regeneration is from 10 to 90% by weight, when the total weight of the preparation is 100%. It is further preferable that the content thereof is from 25 to 75% by weight, when the total weight of the formulation is 100%.

The pharmaceutical preparations is preferably used for demyelinating diseases or peripheral neuropathy; demyelinating diseases is caused by autoimmune reactions, wherein the immune cells such as the antibodies against pathogens or T cells induced from the infection react to both of own myelin sheaths and axons, or peripheral neuropathy as a side effect caused by the treatment for other diseases. As examples of the demyelinating diseases, there are mentioned such as Guillain-Barré syndrome, Fisher syndrome, chronic inflammatory demyelinating polyradiculoneuropathy, MAG antibody-positive neuropathy, multifocal motor neuropathy, diabetic neuropathy, postherpetic neuralgia, and the like. The pharmaceutical preparations are also preferably used for peripheral neuropathy as a side effect of the treatment of other diseases such as those induced by chemotherapy for cancer.

The dosage formulation of the pharmaceutical preparation is not limited. Depending on the dosage form, the active ingredient are mixed with appropriate pharmacologically acceptable excipients, fillers, lubricants, pH adjusters and the like, or absorption enhancers, base materials to formulate a powder, a tablet, a capsule, a liquid, an injection, and plaster form. To prepare such formulations, the regenerative compositions may be prepared as a lyophilized powder. Depending on the necessity, also it may be made as a sustained-release formulation. As describe above, the pharmaceutical formulations of the invention may be manufactured.

## Examples

The present invention is specifically described by using Examples in below. However, the scope of the invention is not limited to the following Examples.

(Example 1) Preparation of virus vectors for producing immortalized SHED

1. Preparation of vectors for introducing adenovirus

(1) Reagents

(1-1) Reagents for plasmid extraction

Kanamycin (Kan), ampicillin (Amp), LB liquid medium and LB agar medium, glycogen, agarose, sterile water, ammonium acetate, sodium acetate, sodium dodecyl sulfate, and RNase A were used. 50 mg/mL of kanamycin (Kan) solution and ampicillin (Amp) solution were prepared and stored at -20 °C as stock solutions. Glycogen was prepared at 20 mg/mL of solution; 10 mg/mL RNase A solution was prepared and stored at -20 °C. 10 M (saturated) ammonium acetate ($NH_4OAc$) solution and 3 M sodium acetate (NaOAc; pH 5.2) solution were prepared.

(1-2) Restriction enzymes and the like

All of E. coli Competent Cells (Supercharge EZ10 Electrocompetent Cells, product code 636756), Swa I (product code 1111A, Smi I is the equivalent), Xho I (product code 1094A), T4 DNA Ligase (product code 2011A) NucleoBond Xtra Midi (product code 740410.10/.50/.100), NucleoSpin Plasmid (product code 740588 10/50/250) were purchased from Takara Bio Inc. Pac I was purchased from New England Biolabs.

(1-3) Buffers

1 x TE Buffer (10 mM Tris-HCl [pH 8.0] containing 1 mM EDTA), phenol: chloroform: isoamyl alcohol (25:24:1), saturated with 100 mM Tris-HCl (pH 8.0, hereinafter referred to as "PCI mixture") are prepared. Ethanol is used either at 100% or 70% concentration. The following buffers 1-4 are prepared for purification of pAdeno-X plasmid DNA which is used in mini-scale recombination.

Buffer 1: 10 mM EDTA and 25 mM Tris-HCl (pH 8.0) including 50 mM glucose (stored at 4 °C after autoclave)
Buffer 2: 0.2 M NaOH including 1 % SDS (prepared immediately before use, sealed and stored at room temperature)
Buffer 3: 5 M KOAc (stored at 4 °C after autoclave)
Buffer 4: 10 mM Tris-HCl including 1 mM EDTA and 20 μg/mL RNase (pH 8.0) (add RNase immediately before use. Store at -20 °C)

(2) Purification of adenovirus and reagents for β-gal assay

Human HEK293 cells (ATCC #CRL1573) transformed with human type 5 adenovirus are used. HEK293 cells are cultured in a complete medium. The composition of the complete medium is Dulbecco's Modified Eagle's Medium (DMEM, basic medium) supplemented with 100 units/mL sodium penicillin G, 100 μg/mL streptomycin, 4 mM glutamine and 10% FBS. Sodium Penicillin G solution is prepared at 10,000 units/mL, and streptomycin sulfate solution at is prepared at 10,000 μg/mL, and then both of them are stored as stock solutions. For culture, 60 mm diameter plates, 100 mm diameter plates, 6-well plates, T75 and T175 flasks are used.

Trypsin-EDTA (product code CC-5012) is purchased from Takara Bio Inc. Phosphate buffered saline (PBS, $Ca^{2+}$ and $Mg^{2+}$ free) and Dulbecco's phosphate buffered saline (DPBS, $Ca^{2+}$ and $Mg^{2+}$ contained) are prepared. In addition, 0.33% neutral red staining solution and 0.4 % trypan blue staining solution are used. β-gal assay is performed by using Luminescent β-gal Detection Kit II (product code 631712, Takara Bio Inc.).

(3) Preliminary experiments

(3-1) Construction of a recombinant adenovirus vector containing lacZ (pAdeno-X-lacZ)

After thawing and removal of DMSO, HEK293 cells are resuspended in 10 mL of the complete medium described above, and then the entire volume thereof is transferred to a 100 mm diameter culture plate. After the HEK293 cells adhere to a bottom of the plate, the culture medium is removed, and then the cells are washed once with sterile PBS. After that, the cells are treated with 1 mL of trypsin-EDTA solution for approximately 2 minutes. Next, 10 mL of the complete medium is added to stop the trypsin reaction, and the cells are gently suspended. Viable counts of the solution are performed, and $10^5$ cells are transferred to a 100 mm diameter plate containing 10 mL of culture medium and spread evenly.

Using pShuttle2-lacZ (a positive control vector included in Adeno-X Expression System 1) and the Adeno-X Viral DNA (PI-Sce I and I-Ceu I digested) included in the kit, recombinant adenovirus containing lacZ is constructed according to the protocol included in the kit. The target cells, SHED, are infected and assayed for β-galactosidase expression to confirm that the vector is constructed.

(3-2) Construction of recombinant pShuttle 2 plasmid

Before construction of the recombinant pShuttle 2 Vector (herein below referred to as "rpShuttle 2 Vector"), E. coli DH5α are transformed both with the pShuttle 2 Vector and pShuttle 2-lacZ Vector included in the kit. Obtained transformants are selected on LB agar plates containing 50 μg/mL kanamycin (herein below referred to as "LB/Kan"). Bacteria cells picked up from a single colony are fractionated onto new LB/Kan, and then the plates are incubated overnight at 37°C.

Next, hTERT, bmi-1, HPV-E6, and HPV-E7 are cloned into pShuttle 2 by using the following procedure. The pShuttle 2 Vector is digested with the appropriate restriction enzyme for these genes. Referring The pShuttle 2 Vector Information Packet (PT3416-5) provided with the kit, the multicloning sites matching the DNA to be inserted are determined. The restriction enzyme-treated plasmid is treated by alkaline phosphatase to be purified.

According to the conventional method, target DNA fragments are prepared and purified. The restriction enzyme-digested vector is ligated with the gene fragments, and DH5α cells (competent cells) are transformed with the ligation product. A portion of the competent cells is taken out and transformed with the control vector pShuttle2-lacZ Vector included in the kit to use as the positive control.

The mixture containing transformed E. coli is inoculated onto LB/Kan agar plates to select transformants (colonies) resistant to kanamycin (Kanr). From 5 to 10 of Kan-resistant clones are selected; and then inoculated onto a small amount of liquid medium for amplification thereof. After confirming that these clones harbored the rpShuttle 2 Vector, they are incubated overnight. The constructed plasmid DNA is then purified according to the conventional method using a commercially available silica adsorption column.

The plasmid DNA is digested with restriction enzymes and subjected to 1% agarose gel electrophoresis to identify the recombinant plasmid of interest. The direction and insertion site of the inserted fragment are confirmed by sequencing, and the positive clone is identified. Recombinant pShuttle 2 plasmid DNA (hereinbelow, referred to as "rpShuttle 2 plasmid DNA") is directly transfected into target cells, and subjected to preliminarily check of the expression of the target protein by using Western blotting.

(3-3) Double digestion of rpShuttle 2 plasmid DNA with PI-Sce I/I-Ceu I

The expression cassette of the transgene is excised from the rpShuttle2 plasmid DNA prepared as described above by using both PI-Sce I and I-Ceu I. The excised expression cassette is incorporated into Adeno-X Viral DNA according to the in vitro ligation method described in the protocol provided with the kit. 30 μl of PI-Sce I/I-Ceu I-double digestion solution of rpShuttle 2 plasmid DNA is prepared and mixed with the reagents listed in Table 1 in a 1.5 mL sterile microcentrifuge tube.

[Table 1]

| Reagents etc., | Amount (μL) | |
|---|---|---|
| | Tube 1 | Tube 2 (lacZ control) |
| Sterilized water | 19.5 | 19.5 |
| 10 x double digestion solution | 3.0 | 3.0 |
| rpShuttle 2 plasmid DNA (500 ng/μL) | 2.0 | - |
| pShuttle 2-lac Z plasmid (500 ng/μL) | - | 2.0 |
| PI-Sce I (1 unit/μL) | 2.0 | 2.0 |
| I-Ceu I (5 unit/μL) | 0.5 | 0.5 |
| 10 x BSA | 3.0 | 3.0 |

Next, after thorough mixing of the solution, it is poured into microcentrifuge tubes. They are lightly centrifuged, and then incubated at 37 °C for 3 hours. After the double digestion, the reaction solution (5 μL) is run along with 1 kb ladder (DNA size marker) on a 1% agarose/EtBr gel.

(3-4) Phenol : Chloroform : Isoamyl alcohol extraction

70 µL of 1 x TE Buffer (pH 8.0) and 100 µL of PCI mixture are added to the remainder of the double digestion solution described above (25 µL) in the centrifuge tube, and thoroughly stirred by vortex. Then, the samples are centrifuged at 14,000 rpm for 5 minutes at 4 °C using a microcentrifuge. The aqueous layer is transferred to a clean 1.5-mL microcentrifuge tube. To this, 400 µL of 95% ethanol, 25 µL of 10 M ammonium acetate, and 1 µL of glycogen (20 mg/mL) are added and thoroughly stirred by vortex.
Next, the mixed solution is centrifuged at 14,000 rpm for 5 minutes at 4 °C, the supernatant is removed by aspiration, and a pellet is obtained. To this pellet, 300 µL of 70% ethanol is added and centrifuged at 14,000 rpm for 2 minutes at room temperature. The supernatant is carefully removed by aspiration, and the pellet is air-dried at room temperature for approximately 15 minutes.
After the pellet is dried, it is dissolved in 10 µL of sterile 1 x TE Buffer (pH 8.0) and stored at -20 °C until use.

(4) Construction of recombinant Adeno-X plasmid DNA

(4-1) Subcloning of expression cassettes to Adeno-X virus genome

The reagents listed in Table 2 below are placed in 1.5-mL sterile microcentrifuge tubes sequentially as listed, and then mixed gently, centrifuged gently, and incubated at 16 °C overnight.

[Table 2]

| Reagents etc., | Amount (µL) |
|---|---|
| pShuttle 2 plasmid DNA digested by PI-Sce I/I-Ceu | 2.0 |
| pShuttle 2-lac Z plasmid DNA digested by PI-Sce I/I-Ceu I | - |
| Sterilized water | 3.0 |
| 10 x DNA Ligation Buffer | 1.0 |
| Adeno-X Viral DNA (250ng/µL) | 3.0 |
| DNA Ligase (1 unit/µL) | 1.0 |
| Total Amount | 10.0 |

To each sample, 90 µL of 1 x TE Buffer (pH 8.0) and 100 µL of PCI mixture are added and gently stirred by vortex. The mixed solutions are centrifuged at 14,000 rpm for 5 minutes at 4 °C, and the aqueous layers thereof are transferred to clean 1.5-mL microcentrifuge tubes. 400 µL of 95% ethanol, 25 µL of 10 M ammonium acetate, and 1 µL of glycogen (20 mg/mL) are added to the tubes and gently stirred by vortex.
The tubes are centrifuged at 14,000 rpm for 5 min at 4 °C, the supernatants are removed by aspiration to obtain the pellet. The following ethanol precipitation procedure is performed in the same manner as described above (3-4). After the pellet dried, they are dissolved in 15 µL of sterile deionized water.

(4-2) Digestion of recombinant Adeno-X plasmid DNA by Swa I

The digestion solution shown in Table 3 below is prepared and added to each sample in the centrifuge tubes, and they are incubated at 25 °C for 2 hours.

[Table 3]

| Reagents etc., | Amount (µL) |
|---|---|
| Ligation product | 15.0 |
| 10 x Swa I Digestion Buffer | 2.0 |
| 10x BSA | 2.0 |
| Swa I (10 units/µL) | 1.0 |
| Total Amount | 20.0 |

To each sample, both of 80 μL of 1 x TE Buffer (pH 8.0) and 100 μL of PCI mixture are added and gently stirred by vortex. The microcentrifuge tubes are centrifuged at 14,000 rpm for 5 minutes at 4 °C. The following ethanol precipitation procedure is performed as in (3-4) above, and the pellet lysate is stored at -20°C until use.

(4-3) Verification of E. coli transformation by using the recombinant Adeno-X plasmid DNAs

Competent cells for electroporation (E. coli) are transformed with the Swa I digestion product obtained in (4-2) described above using Supercharge EZ10Electrocompetent Cell (product code 636756). Obtained transformation mixtures are inoculated on the agar plates with LB medium plus ampicillin (final concentration 100 μg/mL) (herein below referred to as "LB/Amp agar plates") and incubated at 37 °C overnight. Approximately $10^6$ colonies as ampicillin-resistant (Ampr) transformants, were obtained. The obtained colonies are checked with the Adeno-X System PCR Screening Primer Set provided with the product.

Bacteria cells from the single colony are inoculated into 5 mL of fresh LB/Amp liquid medium and incubated overnight. The next day, Adeno-X plasmid DNA is purified according to the mini-scale method described below.

(5) Mini-scale preparation of recombinant Adeno-X plasmid DNA

5 mL of the culture medium in logarithmic growth phase in a tube is centrifuged at 14,000 rpm for 30 seconds and the supernatant is removed. The pellet is centrifuged again at 10,000 rpm for 1 minute and the supernatant is removed using a micropipette. Here, 150 μL of the buffer 1 is added and gently pipetted to resuspend the cells. Then, 150 μL of Buffer 2 is added into the tube, which is mixed by gentle inversion, and left on ice for 5 minutes. To the cooled cell suspension, 150 μL of Buffer 3 is added and mixed again by inversion, and then left on ice for 5 minutes.

The cell suspension is centrifuged at 14,000 rpm for 5 minutes at 4 °C, and the clear supernatant is transferred to the clean 1.5-mL centrifuge tube. To the supernatant in the tube, 450 μL of PCI mixture is added, and the mixture is inverted and then stirred. After that, the mixture is centrifuged at 14,000 rpm for 5 minutes at 4 °C, and the aqueous layer is transferred to the clean 1.5-mL microcentrifuge tube.

The following ethanol precipitation procedure is performed as in (4-1) above, and the pellet lysate is stored at -20 °C until use. The target rDNA is identified by restriction enzyme analysis and PCR as described below.

(6) Restriction enzyme site analysis for the obtained rAdeno-X plasmid DNA

Both of PI-Sce I and I-Ceu I are used for the analysis. Restriction enzyme treatment is performed by adding the reagents listed in Table 4 below and 30 μL of PI-Sce I/I-Ceu I double digestion reaction solution to the 1.5-mL sterile microcentrifuge tube, stirring thoroughly, and then to collect the content by light rotation. After that, the tube is incubated at 37 °C for 3 hours for restriction enzyme treatment. After this treatment, the reaction solution is run on a 1% agarose/ethidium bromide (EtBr) gel.

[Table 4]

| Reagents etc., | Amount (μL) |
|---|---|
| Sterilized water | 19.5 |
| 10 x double digestion solution | 3.0 |
| rpAdeno-X DNA (500 ng/μL) (500 ng/μL) | 2.0 |
| pShuttle 2-lac Z plasmid (500 ng/μL) | - |
| PI-Sce I (1 unit/μL) | 2.0 |
| I-Ceu I (5 unit/μL) | 0.5 |
| 10 x BSA | 3.0 |
| Total Amount | 30.0 |

(7) Production of recombinant adenoviruses

(7-1) Preparation of rAdeno-X plasmid DNA for HEK293 cell transfection

The reagents listed in Table 5 below are mixed in the 1.5-mL sterile centrifuge tube, and then it is lightly centrifuged with the microcentrifuge. The tube is then incubated at 37 °C for 2 hours for Pac I restriction enzyme treatment of rAdeno-X plasmid DNA.

[Table 5]

| Reagents etc., | Amount (μL) |
|---|---|
| Sterilized water | 20 |
| pAdeno-X plasmid DNA (500 ng/μL) | 10 |
| 10 x Pac I Digestion Buffer | 4 |
| 10 x BSA | 4 |
| Pac I (10 units/μL) | 2 |
| Total Amount | 40 |

60 μL of 1 x TE Buffer (pH 8.0) and 100 μL of PCI mixture are added to the tube, and it is gently stirred by vortex, and then centrifuged at 14,000 rpm for 5 minutes at 4 °C with the micro centrifuge. The aqueous layer is carefully transferred to the clean 1.5-mL sterile centrifuge tube.

The following ethanol precipitation procedure is performed as in (3-4) above, and the pellet solution is stored at -20 °C until use.

(7-2) Transfection of Adeno-X plasmid DNA digested by Pac I to HEK293 cells

24 hours prior to transfection of the plasmid DNA, HEK293 cells are inoculated so as to become a cell number about 1 to 2 $\times$ $10^6$ (approximately 100 cells/$mm^2$) per 60-mm diameter culture plate, and then the plates are incubated at 37°C in the presence of 5% $CO_2$ to reach.

HEK293 cells in each culture plate are transfected with 10 μL of Adeno-X plasmid DNA digested by Pac I according to the conventional transfection method (CalPhos Mammalian Transfection Kit product code 631312, Takara Bio Inc.). Cytopathic effect (CPE) appearance in the plate is checked from the day after transfection. After 1 week, cells adhering to the bottom or side wall inside the culture plate are released therefrom by gentle agitation. The resulting cell suspension is transferred to a 15-mL sterile conical centrifuge tube and centrifuged at 1,500 x g for 5 minutes at room temperature.

The obtained precipitate is suspended in 500 μL of sterilized PBS in the tube. The freeze-thaw procedure is repeated three times by freezing the suspended cells in dry ice/ethanol and thawing them in a 37 °C thermostatic chamber to obtain a cell lysate. The lysate is then lightly centrifuged to remove floating materials, and the supernatant is transferred to another sterilized tube for immediate use. The portion not used immediately is stored at -20 °C. 250 μL portion of the lysate is added to the cultured cells in a 60-mm plate and the culture was continued. Adenovirus titer is determined using the anti-Hexon antibody contained in the Adeno-X Rapid Titer Kit (product code 631028, Takara Bio Inc.) according to the instruction manual for the kit (PT3651-1).

(7-3) Amplification of viruses for preparing high titer viruses

Approximately 24 hours before starting this titer assay, HEK293 cells are inoculated into a T75 flask and cultured overnight at 37 °C in the presence of 5% $CO_2$ to 50 to 70% confluency.

Next day, the cells are infected with MOI = 10 in a flask, by replacing the medium with a fresh medium containing the virus. After 90 minutes of incubation at 37 °C in the presence of 5% $CO_2$, and then, 10 mL of medium is added to the flask.

The cells are incubated at 37 °C in the presence of 5% $CO_2$ for 3-4 days to confirm CPE. When 50% of the cells are detached, the cell suspension is made as described above and transferred to the 15-mL sterilized conical centrifuge tube. The cells are lysed by the same freeze-thaw procedure as above. By using the Adeno-X Rapid Titer Kit (product code 631028), the determined titer was $10^7$ PFU/mL. Western blotting is performed to verify that the packaged adenovirus genome has copies of the transcription unit specific for the target gene in a functional form.

(8) Adenovirus infection to target cells

(8-1) Infection to target cells

24 hours before infection, SHEDs are inoculated on a 6-well plate at 1 $\times$ $10^6$ cells per well. The day after inoculation, the medium in the well are removed and 1.0 mL of the medium containing the virus is put on the center of each well. The medium solution is spread evenly throughout the monolayer formed by SHEDs.

SHEDs are infected with the viruses by 4 hour incubation at 37 °C in the presence of 5% $CO_2$. Fresh medium is then added to each well and the plate is further incubated at 37 °C in the presence of 5% $CO_2$. The expression of the transgene is time-dependently analyzed from 24 to 48 hours after infection.

(8-2) Expression analysis of β-galactosidase in the infected cells

Expression of β-galactosidase in AdenoX-lacZ-infected adherent cells is assayed using the Luminescent β-gal Detection Kit II (product code 631712, Clontech ).

2. Preparation of retrovirus vectors

(1) Reagents etc.,

G3T-hi cells (Takara Bio, product code 6163), TransIT-293 (transfection reagent, Takara Bio, product code MIR2704), Retrovirus Packaging Kit Ampho (Takara Bio, product code 6161), Retrovirus Titer Set (for Real Time PCR) (Takara Bio, product code 6166), and One Step SYBR PrimeScript RT-PCR Kit (Perfect Real Time, Takara Bio, product code RR066A) are used. In addition, a real-time PCR system Thermal Cycler Dice Real System (Takara Bio, format TP800) is also used.

(2) Preparation of plasmids

In order to prepare retroviral plasmids for retroviral vector preparation, pDON-5 Neo (Takara Bio, catalog No. 3657) is used. Kozak sequence (gccacc) is added upstream of the start codon of the immortalized genes, TERT (derived from human or swine, herein below abbreviated as "hTERT" or "pTERT", respectively), human papillomavirus E6 and E7, and hBmi-1 according to the conventional method.

Five immortalized genes described above to which Kozak sequence is added is cloned into Pmac I-Hpa I site in the pDON-5 Neo DNA. Ten mL of recombinant retroviral vectors (Amphotropic Envelope) harboring the pDON-5 Neo hTERT Vector (SYN5587-1-4), pDON-5 Neo HPV16E6 Vector (SYN5587-2 -10), pDON-5 Neo HPV16 E7 Vector (SYN5587-3-7), pDON-5 Neo pigTERT Vector (SYN5587-4-9), and pDON-5 Neo hBmi1 Vector (SYN5587-5-1) that are produced respectively.

The inserted gene sequences of the plasmid DNA obtained as described above are verified by single-strand analysis. Transformants are obtained by transforming E. coli using the plasmid DNA. After culturing the transformants, the plasmid DNA of the transformation grade (about 50 μg) is purified according to the conventional method, and the plasmid DNA is obtained as a DNA solution dissolved in sterilized water.

(3) Production of recombinant retrovirus vectors

G3T-hi cells are inoculated into 5 tissue culture dishes (100 mm diameter), at $6 \times 10^6$ cells per dish, and cultured at 37 °C for approximately 24 hours. Three plasmids for retrovirus production (any one of the vectors selected from the group consisting of pDON-5 Neo hTERT Vector, pDON-5 Neo HPV16E6 Vector, pDON-5 Neo HPV16E7 Vector, pDON-5 Neo pigTERT Vector,

and pDON-5 Neo hBmi1 Vector, pGP and pE-Ampho provided in the Retrovirus Packaging Kit Ampho) are co-transfected into the cells using transfection reagents, and the cells were further cultured at 37 °C for 48 hours.

After the culture termination, the culture supernatant containing the retroviral vector is collected, and sterilized by using a 0.45 μm filter (Millipore). Obtained filtrates are dispensed 1 mL into sterile tubes.

(4) Calculation of titer of the recombinant retrovirus vectors

Titer of the recombinant retrovirus vectors are quantified with Retrovirus Titer Set (for Real Time PCR) and One Step SYBR PrimeScript RT-PCR Kit (Perfect Real Time) according to the instruction in the kit. Tables 6 and 7 below show the reaction conditions for template processing and real time PCR, when measuring the titer of retroviral vectors.

Table 6 shows the composition of the reaction mixture employed in the template treatment (digestion of the template with Dnase) for titer measurement of the retro virus vector. Table 7 shows reaction conditions. That is, the solutions listed in Table 6 below are incubated at 37°C for 30 minutes, then the temperature is raised to 70°C and hold for 10 minutes at the temperature, and rapidly decreased to 4 °C.

[Table 6]

| Dnase I treatment | |
|---|---|
| Reagents | Used Amount/Reaction |
| Recombinant retrovirus vector solution | 12.5μL |
| 10 x DNase I buffer | 2.5μL |
| DNase 15 (5 U/μL) | 2.0μL |
| RNase Inhibitor (40 U/μL) | 0.5μL |
| RNase Free $dH_2O$ | 7.5μL |
| Total | 25.0μL |

Realtime PCR is performed according to the following reaction conditions using the solutions shown in Table 7 below: (s1) 5 minutes at 42 °C, (s2) 10 seconds at 95 °C, and then 40 cycles of (s3) 5 seconds at 95 °C and 30 seconds at 60 °C. Finally, a dissociation curve is generated.

[Table 7]

| Reagents | Used Amount/Reaction |
|---|---|
| 2 x One Step SYBR RT-PCR buffer III | 12.5μL |
| TaKaRa ExTaq HS (5 U/μL) | 0.5μL |
| PrimeScript RT Enzyme Mix II | 0.5μL |
| Forward Titer Primer FRT-1 (10 pmol/μL) | 0.5μL |
| Reverse Titer Primer FRT-1 (10 pmol/μL) | 0.5μL |
| RNase Free $dH_2O$ | 8.5μL |
| Template | 2 μL |
| Total | 25.0μL |

A calibration curve was created by plotting the copy number of the RNA Control Template supplied with the kit on the X-axis and the Ct value calculated from the second derivative curve (2nd Derivative) on the Y-axis. Table 8 below shows the Ct values of the real-time PCR calibration curves. In the table below, for example, "1.00E+08" represents "$1.00 \times 10^8$". The same applies hereinafter.

[Table 8]

| STD | Copy numbers/μL | Ct(SDM) | Ct Ave. |
|---|---|---|---|
| 1 | 1.00E+08 | 15.43 | 15.40 |
| | | 15.37 | |
| 2 | 1.00E+07 | 19.12 | 19.12 |
| | | 19.12 | |
| 3 | 1.00E+06 | 22.72 | 22.78 |
| | | 22.84 | |
| 4 | 1.00E+05 | 26.30 | 26.33 |
| | | 26.35 | |
| 5 | 1.00E+04 | 29.85 | 29.72 |
| | | 29.59 | |
| 6 | 1.00E+03 | 33.19 | 33.12 |

The Ct values were calculated by the 2nd1Derivative Maximum (SDM) method. From the calculated Ct values, a slope of -3.54, an intercept of 43.88, an amplification efficiency of 91.6 and a coefficient of determination of 0.999 were obtained. From this standard curve, the titer of the tested sample was calculated, and the results are shown in Table 9.

[Table 9]

| Sample Name* | Dilution factor | Ct (SDM) | Calculated copies (copies/μL) | Titer** (copies/mL) | Average Titer (copies/mL) |
|---|---|---|---|---|---|
| Rl60l_pDON-5 Neo hTERT | 100 | 26.85 | 6.45E+04 | 1.29E+10 | 1.46E+10 |
| | 100 | 26.89 | 6.28E+04 | 1.26E+10 | |
| | 500 | 28.96 | 1.63E+04 | 1.63E+10 | |
| | 500 | 28.94 | 1.66E+04 | 1.66E+10 | |
| R1601_pDON-5 Neo HPV16E6 | 100 | 27.16 | 5..27E+04 | 1.05E+10 | 1.15E+10 |
| | 100 | 27.15 | 5.30E+04 | 1.06E+10 | |
| | 500 | 29.37 | 1.25E+04 | 1.25E+10 | |
| | 500 | 29.39 | 1.24E+04 | 1.24E+10 | |
| R1601_pDON-5 NeoHPV16E7 | 100 | 25.06 | 2.06E+05 | 4.13E+10 | 4.05E+10 |
| | 100 | 25.14 | 1.96E+05 | 3.92E+10 | |
| | 500 | 27.49 | 4.25E+04 | 4.25E+10 | |
| | 500 | 27.62 | 3.91E+04 | 3.91E+10 | |
| R1601_pDON 5 Neo pigTERT | 100 | 26.94 | 6.08E+04 | 1.22E+10 | 1.27E+1O |
| | 100 | 26.97 | 5.96E+04 | 1.19E+10 | |
| | 500 | 29.26 | 1.35E+04 | 1.35E+10 | |
| | 500 | 29.30 | 1.31E+04 | 1.31E+10 | |
| R1601_pDON-5 Neo hBmi1 | 100 | 25.73 | 1..34E+05 | 2.67E+10 | 2.83E+10 |
| | 100 | 25.82 | 1.26E+05 | 2.52E+10 | |
| | 500 | 27.88 | 3.30E+04 | 3.30E+10 | |
| E.D. | I | 35.92 | 1.77E+02 | - | - |
| | I | 33.94 | 6.41E+02 | - | |

In the table, E.D. represents using EAST Dilution (for Real Time PCR) as a dilution buffer. The titer (copies/mL) was calculated as the calculated copy number (copies/μL) x 2 (dilution factor for Dnase I treatment) x dilution factor for real-time PCR reaction (100 or 500) × 1,000 (conversion from μL to mL).

Real-time PCT results showed that pDON-5 Neo hTERT had a titer of 1.46 × $10^{10}$ copies/mL, pDON-5 Neo HPV16E6 had a titer of 1.15 × $10^{10}$ copies/mL, pDON-5 Neo HPV16E7 had a titer of 4.05 × $10^{10}$ copies/mL, pDON-5 Neo pigTERT had a titer of 1.27×$10^{10}$ copies/mL, or pDON-5 Neo hBmi1 had a titer of 2.83 × $10^{10}$ copies/mL.

(Example 2) Preparation of immortalized SHED and its culture supernatant

A deciduous tooth obtained from a 10-year-old healthy male child was used. After disinfection of the deciduous tooth with isodine solution, the crown was cut horizontally using a dental diamond point, and the pulp tissue was collected using a dental reamer. The pulp tissue was digested in a solution of 3 mg/mL type I collagenase and 4 mg/mL dispase at 37 °C for 1 hour. The solution was then filtered through a 70 mm cell strainer (Falcon).

The filtered cells are resuspended in 4 mL of the medium and inoculated into 60 mm diameter dishes for adherent cell culture. After adding Dulbecco's Modified Eagle's Medium (DMEM) containing 10 % FCS to the dish, the cells are incubated in a 5% $CO_2$ incubator at 37 °C for about 2 weeks. Adherent cells (dental pulp stem cells) that formed colonies are treated with 0.05 % trypsin/0.2 mM EDTA for 5 minutes at 37°C, and cells detached from the dish are collected.

Next, the adherent cells selected as described above are inoculated into adherent cell culture dishes (collagen-coated dishes), and primarily cultured in an incubator adjusted to 5% $CO_2$ and 37 °C. The cells are then used as primary cultured cells. When cells reached subconfluency (approximately 70% of the surface of the culture vessel is occupied by cells) or confluency by naked eye observation, cells are detached from the culture vessel and collected by treatment with 0.05% trypsin/0.2 mM EDTA for 5 min at 37 °C. The obtained cells are again inoculated into a dish containing the medium and passage several times to grow to about $1 \times 10^7$ cells/mL. The obtained cells are stored in liquid nitrogen.

After that, primary cultured cells are passaged at a concentration of approximately $1 \times 10^4$ cells/cm$^2$ using the medium. The cells after 1 to 3 passages are used for experiments. Human stem cells from human exfoliated deciduous teeth (SHED) were obtained as described above. The obtained SHEDs are sorted using FACSTARPLUS (Becton Dickinson) with approximately $1 \times 10^6$ STRO-1 positive cells for each sample as follows.

Bromodeoxyuridine (BrdU) is taken up for 12 hours according to the manufacturer's (Invitrogen) instructions for the BrdU staining kit and the growth rate of SHED is evaluated (n=3 for each group). The experiment is repeated 5 times. After one-way analysis of variance, the Tukey-Kramer test is performed to evaluate statistically significant differences.

To detect STRO-1 by immunofluorescence, SHEDs are fixed with 3% paraformaldehyde, and then rinsed twice with PBS and treated with 100 mM glycine for 20 minutes. These cells are then permeabilized with 0.2% Triton-X (Sigma-Aldrich) for 30 minutes, followed by incubation in a mixture of 5% donkey serum and 0.5% bovine serum albumin for 20 minutes.

Next, the cells are incubated for 1 hour with the primary antibody, mouse anti-human STRO-1 antibody (1: 100, R&D), and for 30 minutes with the secondary antibody, goat anti-mouse immunoglobulin M-FITC antibody (1:500, Southern Biotech) and mounted using Vectashield DAPI (Vector Laboratories Inc.). After that, $\alpha$-MEM supplemented with 15% FBS is placed in 6-well plates and sorted cells are seeded for cloning. Approximately 300 colonies from the proliferated cells were pooled for testing.

(2) Gene transfer

(2-1) Gene transfer by adenovirus vector

As described above, four genes, bmi-1, E6, E7 and hTERT, are incorporated into adenovirus vectors to create viral vectors expressing these gene products. As a control, a control vector is created that was not incorporated with these genes.

SHEDs are inoculated at $1 \times 10^6$ cells in a 100 mm diameter collagen-coated dish and incubated with DMEM supplemented with 10% FBS until subconfluent. The medium is aspirated off and 500 $\mu$L of virus solution diluted in the medium is added (MOI=10). The cells are incubated at 37 °C for 1 hour in a 5% $CO_2$ incubator to infect with the virus vectors. After 48 hours of infection, infected cells are transferred into the medium with puromycin (1 pg/mL) and cultured for 10 days for selection, and 500 to 600 resistant clones are pooled. SHEDs are inoculated at approximately $0.5 \times 10^5$ into 100 mm diameter culture Petri dishes and passaged every 3 or 4 days. SHEDs with transgenes are termed as SHED-T, and SHEDs without transgenes are termed as SHED-C.

(2-2) Gene transfer by retrovirus vector

The retroviral vectors obtained as described above are infected with human dental pulp-derived cells (KA_01_W14_P5D3) or human pulp-derived cells (KA_02_W4-6_P4D3), and drug-resistant pooled cells (herein below simply referred to as "pooled cells") are obtained. The pooled cells obtained are subjected to real-time PCR for analysis to confirm the number of inserted genes. Primers listed in Table 10 below (SEQ ID NO. 13-16, all from Takara Bio) are used for real-time PCR.

[Table 10]

| Genes | Catalog No. | Nucleotide sequence | SEQ ID No. |
|---|---|---|---|
| Target gene | CH000987_F | GCACTGCCCTCAGACTTCAAGA | 13 |
| | CH000987_R | GCGGGACTATGGTTGCTGAC | 14 |
| Control gene (Human $\beta$-actin) | HA067803_F | TGGCACCCAGCACAATGAA | 15 |
| | HA067803_R | CTAAGTCATAGTCCGCCTAGAAGCCA | 16 |

The KA_01_W14_P5D3 is cultured at 37 °C in a 5% $CO_2$ incubator in Corning adherent cell culture dish (6 or 10 cm diameter Cell culture Dish, TC treated surface). When cryopreserved cells are thawed and inoculated into the dish, the passage number of the cell is [0] (herein below referred to as "+P0"). When 1 or 2 times passaged, referred to as "+1" and "+2", respectively.

When the cells in the dish reached about 80% confluence, the culture supernatant is removed. The cells are washed 2 to 3 times with PBS, and then treated with 0.25% trypsin-EDTA solution (1 x) (containing phenol red) (Thermo Fisher Scientific, 25200-056) as a detaching agent for 3 minutes incubation at 37 °C in a 5% $CO_2$ incubator to detach adherent cells from the bottom of the dish.

To this, an appropriate amount of medium is added and pipetted to prepare a single-cell suspension. The cell suspension is diluted to an appropriate volume and transferred to a new dish. The culture is continued in the same manner, and it is confirmed that there are no abnormalities in cell proliferation or morphology. The culture supernatant collected at the time of passaging is also checked for mycoplasma contamination using the MycoAlert Mycoplasma Detection Kit (Loza, product code LT07-318).

(3) Examination of drug-resistance concentration

In order to select transgenic cells by drug, the G418 resistance concentration of target cells were examined. 6-well plates provided by Corning (6 Well Clear TC-Treated Multiple Well Plate, product code 3516) are used to inoculate 2.0 $\times 10^4$ cells in logarithmic growth phase per well. The cells are incubated in a 5% $CO_2$ incubator at 37 °C for approximately 24 hours, after which cell adhesion to the bottom of the wells is checked. The medium in each well is replaced with medium supplemented with different concentrations of G418. The condition of the cells is photographed 4, 8, and 10 days after the start of drug selection to examine the drug resistance concentration.

4 days after the start of drug selection, cell proliferative activity was lost in wells with a drug concentration of 250 μL/mL or higher, and 11 days later, most cells in wells with a drug concentration of 500 μL/mL or higher were found to be dead. From the results above, the G418 selective drug concentration for target cells was determined to be 500 μL/mL, which shows sufficient selective effect.

The retrovirus vector solution is used to infect target cells by polybrene method, and immortalized gene stable expression lines are selected by medium B. In the 6-well plates, 1.40 $\times 10^5$ cells in logarithmic growth phase are inoculated per well and incubated at 37 °C in a 5% $CO_2$ incubator for approximately 24 hours. Then, polybrene (Hexadimethrine bromide, Sigma-Aldrich, product code H9268) is added to the recombinant retrovirus vector solution diluted 4- or 10-fold in medium A to a final concentration of 8 μg/mL, and equal amounts are added to each well to infect cells with the virus. Approximately 4 hours after viral infection, 1.0 mL of medium A is added to each well. The plate is incubated at 37°C in a 5% $CO_2$ incubator, and the medium is replaced with medium B to initiate drug selection after 24 hours from the incubation.

After the 10th day of drug selection, cells that had drug-resistance and proliferated are collected, 1 mL of CELLBANKER 1 Plus (Nippon Zenyaku Kogyo, product code CB021) is added to make a suspension containing 1 $\times 10^6$ cells/mL, which is stored at - 80 °C as frozen cell stock. At the same time, a cell pellet of 5 $\times 10^5$ cells/vial is prepared for real-time PCR analysis and stored at -80 °C.

(4) Preparation of a culture supernatant of the immortalized stem cells

2 $\times 10^5$ cells/mL of immortalized dental pulp stem cells (herein below sometimes referred to as "hSHED") prepared above are placed in a culture dish (55 $cm^2$) containing 10 mL of DMEM with 10% FBS and cultured at 37 °C in a 5% $CO_2$ incubator until 70% confluent (4 to 6 days).

The 70% confluent was confirmed under a microscope, after which the culture supernatant in the culture flask is removed using an aspirator and the cells are washed twice with serum-free DMEM. Then, to this culture flask, 10 mL of serum-free DMEM (herein below sometimes referred to as "Plain DMEM") is added. The culture supernatant collected immediately after the addition is used as a control. After the addition, the cultures are incubated at 37 °C for 72 hours in a 5% $CO_2$ incubator. The culture supernatant is collected in a 15 mL sterilized centrifuge tube, centrifuged at 440 $\times$ g for 3 minutes, and the centrifuged supernatant is passed through a 0.22 mm PVDF filter (Millipore) to obtain culture supernatant of immortalized SHED (herein below sometimes simply referred to as "SHED-CM").

(Example 3) Analysis of factors contained in the culture supernatant

(1) Preparation of a culture supernatant of immortalized stem cells

2 $\times 10^5$ cells/mL of immortalized stem cells prepared in Examples 1 and 2 above are placed in a culture dish (55 $cm^2$) containing Dulbecco's modified Eagle's medium (DMEM) supplemented with 10 mL of 10% fetal bovine serum (FBS) and cultured at 37 °C in a 5% $CO_2$ incubator until 70% confluent (4 to 6 days).

The 70% confluent is confirmed under a microscope, and the culture supernatant in the culture flask is then removed using an aspirator, and the cells are washed twice with serum-free DMDM. Then, to this culture flask, 10 mL of serum-free DMEM (Plain DMEM) is added. The culture supernatant collected immediately after the addition is used as a control. After the addition, the cultures are incubated at 37 °C for 72 hours in a 5% $CO_2$ incubator. The culture supernatant is collected in a 15 mL sterilized centrifuge tube, centrifuged at 1,500 rpm (approximately 440 × g) for 3 minutes, and the centrifuged supernatant is passed through a 0.22 mm PVDF filter (Millipore) to obtain SHED-CM.

(2) Quantitative expression analysis

To identify expressed cytokines in the SHED-CM obtained as described above, quantitative expression analysis is performed using Quantibody ™ Human Cytokine Antibody Array 1000 (RayBiotech Inc., Catalog No. QAH-CAA-1000) according to the instruction in the kit.

First, the SHED-CM and the antibody immobilized on the array are contacted for 1 to 2 hours, washed, and then a cocktail of biotinylated antibodies is added to each well and incubated for 1 to 2 hours. Labeled streptavidin is then added and incubated for 2 hours. The signal of labeling is detected and data analysis is performed using GenePix ™ 4400A (Molecular Dveices, LLC). As a result, 80 cytokines were detected, which are listed in Table 11 below.

[Table 11]

| No. | Cytokine name | No. | Cytokine name | No. | Cytokine name |
|---|---|---|---|---|---|
| 1 | BLCBCA-1/CXCL13 | 28 | M-CSF | 55 | Growth Hormone |
| 2 | Eotaxin/CCL11 | 29 | MIG/CXCL9 | 56 | HB-EGF |
| 3 | Eotaxin-2/CCL24 | 30 | MIP-1a/CCL3 | 57 | HGF |
| 4 | G-CSF | 31 | MIP-1β/CCL4 | 58 | IGFBP-1 |
| 5 | GM-CSF | 32 | MIP-1δ/CCL15 | 59 | IGFBP-2 |
| 6 | I-309/CCL1 | 33 | PDGF-BB | 60 | IGFBP-3 |
| 7 | ICAM-1 | 34 | RANTES/CCL5 | 61 | IGFBP-4 |
| 8 | IFNγ | 35 | TIMP-1 | 62 | IGFBP-6 |
| 9 | IL-1α | 36 | TIMP-2 | 63 | IGF-I |
| 10 | IL-1β | 37 | TNFα | 64 | Insulin(pro & mature) |
| 11 | IL-1ra | 38 | TNFβ | 65 | M-CSF R |
| 12 | IL-2 | 39 | sTNF RI/TNFRS1A | 66 | NGF R |
| 13 | IL-4 | 40 | sTNF RII/TNFRS1B | 67 | NT-3 |
| 14 | IL-5 | 41 | Amphiregulin(AR) | 68 | NT-4 |
| 15 | IL-6 | 42 | BDNF | 69 | Osteoprotegerin(OPG) |
| 16 | IL-6 sR | 43 | bFGF/FGF-2 | 70 | PDGF-AA |
| 17 | IL-7 | 44 | BMP-4 | 71 | PIGF |
| 18 | IL-8/CXL8 | 45 | BMP-5 | 72 | SCF |
| 19 | IL-10 | 46 | BMP-7 | 73 | SCF R |
| 20 | IL-11 | 47 | β-NGF | 74 | TGFα |
| 21 | IL-12p40 | 48 | EGF | 75 | TGFB1 |
| 22 | IL-12p70 | 49 | EGF R | 76 | TGFβ3 |
| 23 | IL-13 | 50 | EG-VEGF | 77 | VEGF |
| 24 | IL-15 | 51 | FGF-4 | 78 | VEGF R2 |
| 25 | IL-16 | 52 | FGF-7/KGF | 79 | VEGF R3 |
| 26 | IL-17 | 53 | GDF-15 | 80 | VEGF-D |
| 27 | MCP-1/CCL2 | 54 | GDNF | | |

The insulin-like growth factor binding proteins, IGFBP-4, IGFBP-6 and IGFBP-2, and the tissue inhibitors of metalloproteinases, TIMP-1 and TIMP-2, were shown to be relatively high in the content. Insulin was excluded from the result because it was not measured correctly this time.

(3) Qualitative expression analysis

To identify which cytokines are expressed in the SHED-CMs obtained as described above, qualitative expression analysis was performed using RayBio (registered trademark) Human Cytokine Antibody Array G-Series 4000 (RayBiotech Inc., catalog no. AAH-CYT-G4000-4) according to the instruction in the kit.

First, the SHED-CM or standard protein cocktail is contacted with an array of antibodies immobilized on glass slides for 1 to 2 hours, followed by incubation with a cocktail of biotinylated antibodies for 1 to 2 hours. Following incubation with Cy3-equivalent labeled streptavidin for 1 hour, scanning, data extraction and data analysis are performed using GenePix (registered trademark) 4400A (Molecular Dveices, LLC).

264 types of cytokines (shown in Tables 12 to 16 below) were identified, of which 77 types had 1.5 times higher expression intensity compared to the control.

[Table 12]

| Cytokine name | | | | | |
|---|---|---|---|---|---|
| Angiogenin | BDNF | BLC | BMP-4 | BMP-6 | CKb8-1 |
| CNTF | EGF | Eotaxin | Eotaxin-2 | Eotaxin-3 | FGF-6 |
| FGF-7 | FLT-3 Ligand | Fractalkine | GCP-2 | GDNF | GM-CSF |
| I-309 | INF-Gamma | IGFBP-1 | IGFBP-2 | IGFBP-4 | IGF-1 |
| IL-10 | IL-13 | IL-15 | IL-16 | IL-1alpha | IL-1beta |
| IL-1ra | IL-2 | IL-3 | IL-4 | IL-5 | IL-6 |
| IL-7 | Leptin | LIGHT | MCP-1 | MCP-2 | MCP-3 |
| MCP-4 | M-CSF | MDC | MIG | MIP-1 delta | MIP-3 alpha |
| NAP-2 | NT-3 | PARC | PDGF-BB | RANTES | SCF |
| SDF-1 | TARC | TGF-beta1 | TGF-beta3 | TNF-alpha | TNF-beta |

[Table 13]

| Cytokine name | | | | | |
|---|---|---|---|---|---|
| Acrp30 | AgRP | Angiopoietin-2 | Amphiregulin | Axl | bFGF |
| b-NGF | BTC | CCL-28 | CTACK | Dtk | EGF-R |
| ENA-78 | Fas/TNFRSF6 | FGF-4 | FGF-9 | GCSF | GITR-Ligand |
| GITR | GRO | GRO-alpha | HCC-4 | HGF | ICAM-1 |
| ICAM-3 | IGFBP-3 | IGFBP-6 | IGF-I SR | IL-1 R4/ST2 | IL- 1 RI |
| IL-11 | IL-12 p40 | IL-12 p70 | IL-17 | IL-2 Rapha | IL-6 R |
| IL-8 | I-TAC | Lymphotactin | MIF | MIP-1 alpha | MIP-1 beta |
| MIP-3 beta | MSP-alpha | NT-4 | Osteo -protegerin | Oncostatin M | PIGF |
| sgp130 | sTNF RII | sTNF-RI | TECK | TIMP-1 | TIMP-2 |
| Thrombo -poietin | TRAIL R3 | TRAIL R4 | uPAR | VEGF | VEGF-D |

[Table 14]

| Cytokine name | | | | | |
|---|---|---|---|---|---|
| Activin A | ALCAM | B7-1(CD80) | BMP-5 | BMP-7 | Cardiotrophin-1 |
| CD14 | CXCL-16 | DR6 (TNFRSF21) | Endoglin | ErbB3 | E-Selectin |
| FAS Ligand | ICAM-2 | IGF-II | IL-1RII | IL-10 Rbeta | IL-13 Ralpha2 |
| IL-18 BPalpha | IL-18 Rbeta | IL-2 Ralpha | IL-2 Rbeta | IL-2 Rgamma | IL-21R |
| IL-5 Ralpha | IL-9 | IP-10 | LAP | Leptin R | LIF |
| L-Selectin | M-CSF R | MMP-1 | MMP-13 | MMP-9 | MPIF-1 |
| NGF R | PDGF AA | PDGF-AB | PDGF Ralpha | PDGF Rbeta | PECAM-1 |
| Prolactin | SCF R | SDF-1beta | Siglec-5 | TGF-alpha | TGF beta2 |
| Tie-1 | Tie-2 | TIMP-4 | VE-Cadherin | VEGF R2 | VEGF R3 |

[Table 15]

| Cytokine name | | | | |
|---|---|---|---|---|
| Adipsin | BCAM | CD30 | CD40 | Fcgamma RIIB/C |
| Ferritin | FLRG/FSTL3 | Follistatin | Furin | Galectin-7/ LGALS7 |
| GDF-15/MIC-1 | Growth Hormone(GH) | IL-10 R alpha | IL-22 | IL-28A1 IFN-gamma2 |
| IL-29 | IL-31 | Insulin (pro & mature) | LH | LIMPII/SR-B2 |
| LYVE-1 | Marapsin/ Pancreasin | MICA | MICB | MMP-2 |
| MMP-7 | MMP-8 | MMP-10 | NCAM-1/CD56 | - Nidogen-1/ Entactin |
| NrCAM/Bravo | NRG1 beta 1 | Osteopontin (OPN) | PAI-I | PF4 |
| PSA(total) | RAGE/AGER | RANK/ TNFRSF11A | Resistin | Serum Amyloid A (SAA) |
| Siglec-9 | TACE/ CD156b | TIM-1/HAVCR | TRAIL R3 /TNFRSF10B | Trappin-2 |
| TREM-1 | TSH | TSLP | VCAM-1 | VEGF-C |
| XEDAR | - | - | - | - |

[Table 16]

| Cytokine name | | | | |
|---|---|---|---|---|
| 4-1BB | ACE-2 | Alpha-fetoprotein | Angiopoietin-1 | Angiostatin (ANG) |
| ANGPTL4 | Beta-2 Micro -globulin | BCMA/TNFRSF17 | beta-IG-H3/ TGFBI | CA125 |
| CA15-3 | CA19-9 | Carbonic Anhydrase IX | Cathepsin S | CCL14a/ HCC-1 |
| CCL21/ 6Ckine | CD23/Fc epsilon-RII | CD40 Ligand | CEA/CD66e | CEACAM-1 |
| Cripto-1/CR1 | CRP | DAN/NBL 1 | Decorin(DCN) | Dickkopf-1 (Dkk-1) |
| Dickkopf-3 (Dkk-3) | Dickkopf-4 (Dkk-4) | DPPIV/CD26 | E-Cadherin | EDA-A2 |
| EG-VEGF | Ep-CAM/ CD326 | ErbB2/HER2 | Erythropoeitin R (Epo R) | FSH |
| HB-EGF | hCGa (intact) | HVEMITNFRSF14 | IL-13 R alpha-1 | IL-17B |
| IL-17C | IL-17F | IL-17R | Procalcitonin (PCT) | PSA(free) |
| S100b | Shh N | Thyroglobulin(TG) | Ubiquitin+1 | - |

From the results, the relative expression intensities of NGF, II, family (IL-6, IL-8, IL6R, IL-17C, IL-17R, etc.) and others were less than 10.

(Example 4) Examination of the effect of SHED-CM on autoimmune neuritis

The therapeutic effect of SHED-CM prepared above is examined using experimental autoimmune neuritis (EAN) mice that is a model mouse of Guillain-Barre syndrome. The model mice are 6-week-old male C57BL/6 mice (purchased from Sankyo Lab Service Corporation, Inc.), which are kept at room temperature in light and dark for 12 hours, with free water and feed for 1 week of acclimation, and divided into a negative control group and a treatment group (n=5).

After acclimation through rearing, a P0 peptide solution is prepared by dissolving a portion of P0 (PO peptide, Biologica co.), one of the major constituent proteins of the myelin sheath, in PBS to a concentration of 2 mg/ml. Complete Freund's adjuvant, which is prepared by adding H37RA, a heat-killed strain of Mycobacterium tuberculosis, to incomplete Freund's adjuvant (Difco Laboratories), to a concentration of 20 mg/ml. The adjuvant was made (herein below referred to as "P0(180-199)/CFA") by mixing these solutions in equal volumes as emulsion,

The administration schedule of the immunizing solution and that of treatment are shown in Figure 3(A). In Figure 3(A), the first administration date of PO(180-199)/CFA was determined as a start of the experiment (herein below referred to as "Day 0"). Administration dates of pertussis toxin and SHED-CM obtained in Example 2 described above are indicated as Day 3, Day 8, starting from Day 0. PT represents the date of administration of pertussis toxin.

Each mouse in the negative control and treatment groups is injected subcutaneously with 50 μL of PO(180-199)/CFA dorsally between the left hindlimb and tail (day 0) and again on day 8 at the same dose on the opposite side of the first injection. Furthermore, EAN model mice are generated by intraperitoneal administration of pertussis toxin to the mice at 400 ng/mouse on Day -1 and 300 ng/mouse on Day 1 and Day 3, respectively. After immunization, the mice are scored for clinical signs every 2 to 3 days according to the following criteria.

Score 0: No clinical signal
Score 1: Decrease in activity
Score 2: Tail paralysis or mild hindlimb paralysis
Score 3: Mild motor impairment or hindlimb paralysis
Score 4: Severe motor impairment

The EAN model mice prepared above are intraperitoneally injected with 200 μL of DMEM or SHED-CM 5 times on Day 15, Day 17, Day 20, Day 22, and Day 24. Clinical sign scores are scored according to the criteria, calculated as mean ± SD, and confirmed for significant differences by Student's t-test. * indicates $p < 0.05$, ** indicates $p < 0.01$, and **** indicates $p < 0.001$. The results are shown in Figure 3(B).

In the negative control group, there was a gradual increase in clinical symptom scores even after the administration of DMEM (Day 15 and beyond). In contrast, the treatment group showed a decreasing tendency in the scores on the second day (Day 17) after the administration of SHED-CM, and a significant decrease in the scores was recorded after Day 5 (Day 20) compared to the negative control group (in Figure 3(B)). The results suggest that SHED-CM has a therapeutic effect on Guillain-Barré syndrome, an autoimmune neuritis.

(Example 5) Examination of acting mechanisms of SHED-CM on autoimmune neuritis treatment

The sciatic nerves of both groups above were taken and subjected to histopathological examination using optical and electron microscopy and Western blotting to examine the effects of SHED-CM administration in EAN mice.

(1) Histopathological examination

On Day 31, mice are euthanized and the sciatic nerve is harvested. The nerve is fixed with Karnofsky's fixative and epoxy-embedded. After sectioning into 0.5-μm-thick slices, the slices are stained with toluidine blue and observed using optical (Figures 4(A1) and 4(B 1) at × 100 magnification, Figures 4(A2) and 4(B2) at × 200 magnification, Thermo Fisher Scientific, Catalog No. EVOS Cell Imaging Systems) and electron microscopy (Figure 4 (A3) and (B3) at × 500 magnification, JEOL, Catalog No. JEM-1400 Flash). This result implies that SHED-CM acts directly or indirectly on the thickness of the myelin sheath, that is, on Schwann cells.

(2) Quantitative PCR and Western blotting

EAN model mice are prepared in the same manner as in Example 4 above and divided into the negative control group (4 mice) and the treatment group (5 mice). 200 μL of SHED-CM is administered intraperitoneally 5 times, namely on Day 13, Day 15, Day 17, Day 20, and Day 22, and clinical signs are scored every 2 to 3 days according to the criteria. On Day 23, mice were euthanized, after which the sciatic and caudal nerves were harvested and the obtained samples were each divided into two equal parts.

From one of the equally divided tissues, total RNA is extracted by using the RNeasy Mini Kit (QIAGEN, performed according to the protocol provided with the kit), and then, cDNA is prepared by using Thermo Fisher Scientific's SuperScript VI reverse transcriptase. After that, the expression analysis by using quantitative PCR, of which target is mRNA of each gene, Myelin Basic Protein (MBP), TNF-α, IFN-γ, and IL - 17A. PCR conditions are the same as described above. In the analysis, hypoxanthine guanine phospho-ribosyl-transferase (HPRT) is used as an internal standard gene, and the results are expressed as relative ratios against thereof and calculated as the mean ± SD. The results are shown in Figures 5(A) and 6. In Figure 6, the statistical results are indicated by $p<0.05$ as * and $p<0.001$ as ***. In the graph of TNF-α/HPRT and IL-17A/HPRT in Figure 6(B) (caudate nerve), "0.07" represents the p value resulting from the statistical process as described above. Primer sets used for the quantitative PCR are shown in Table 17 below.

[Table 17]

| Primer Name | Sequence | SEQ ID No. |
|---|---|---|
| Mouse MBP sense | TGATTTCGAGCGTGGCAGAG | 17 |
| Mouse MBP antisense | ACATTTGGCGGCCACACATA | 18 |
| Mouse TNF-α sense | TATGGCCCAGACCCTCACA | 19 |
| Mouse TNF-α antisense | GGAGTAGACAAGGTACAACCCATC | 20 |
| Mouse IFN-γ sense | CGGCACAGTCATTGAAAGCCTA | 21 |
| Mouse IFN-γ antisense | GTTGCTGATGGCCTGATTGTC | 22 |
| Mouse IL-17A sense | CTGATCAGGACGCGCAAAC | 23 |
| Mouse IL-17A antisense | TCGCTGCTGCCTTCACTGTA | 24 |
| Mouse HPRT sense | TTGTTGTTGGATATGCCCTTGACTA | 25 |
| Mouse HPRT antisense | AGGCAGATGGCCACAGGACTA | 26 |

From another part of the equally divided tissue, cell lysate is prepared by using a detergent 1% Nonidet p40 solution containing a protease inhibitor (Product Name: Protease Inhibitor Cocktail, Sigma-Aldrich) and a phosphatase inhibitor (Product Name: Protease/Phosphatase Inhibitor Cocktail, Cell Signaling), and the protein content thereof set certain amount by using TaKaRa BCA Protein Assay Kit (Takara Bio Inc.). After that, the cells are subjected to electrophoresis with SDS-PAGE, and Western blot analysis by using anti-MBP antibody, anti-Phospho-c-met antibody (Cell Signaling), and anti-actin antibody (Santa Cruz). Results are shown in Figure 5(B) and Figure 7.

Figure 5(A) shows that the expression level of MBP mRNA increased by SHED-CM administration. Also, Figure 5(B) shows that the protein amount of MBP also increased with SHED-CM administration. MBP is a major protein configuring myelin sheath, and the result of increment of the amount is consistent with the observation results by microscopy (Figure 4) that the myelin sheath was getting thick.

Furthermore, from the results of quantitative PCR shown in Figure 6, it was found that the expression level of various types of inflammatory cytokines decreased, and the phosphorylation level of c-Met increased from Figure 7. It is known that c-Met is a receptor of hepatocyte growth factor (HGF) and is phosphorylated upon receiving HGF. Therefore, the results above indicate that HGF contained in SHED-CM and the like have anti-inflammatory effects and relates to the improvement of autoimmune neuritis (namely, healing).

(3) Confirmation of HGF function contained in SHED-CM

In order to investigate whether HGF in SHED-CM is involved in the healing of autoimmune neuritis, as in Example 4 described above, EAN model mice are produced according to the adjuvant and antigen administration schedule shown in Figure 8(A), and divided into the negative control group, the positive control group 1, the positive control group 2, and the test group (n = 5 each). Abbreviations in Figure 8(a) are the same as those in Figure 3(A). Statistical processing was performed as in Example 4 as described above.

For treatment, DMEM is administered to the negative control group and SHED-CM is administered to the positive control group 1. The positive control group 2 is treated with SHED-CM with Isotype IgG at simultaneously, and the test group is treated with SHED-CM with anti-HGF antibody, a neutralizing antibody of HGF simultaneously. The results are shown in Figure 8. Note that the time dependent changes in the clinical signs scores of the positive control group 2 and the test group are shown.

Compared to the positive control group 2, the test group did not show the improvement in the clinical sign score by administration of SHED-CM. These results indicate that HGF contained in SHED-CM is involved in the alleviation of symptoms of autoimmune neuritis.

These results indicate that SHED-CM enhances myelin sheath injury repair and regeneration and inhibits inflammation, which contribute to recovery from EAN.

(Example 6) Effects of SHED-CM on Schwann cells

In order to examine the effect of SHED-CM on Schwann cells in detail, IMS32 cells, a mouse Schwann cell strain, are used for in vitro experiments. IMS32 is cultured in a medium for IMS32 (Cosmo Bio) in the 10 cm dish at 37 °C in a 5% $CO_2$ incubator until 80% confluent. Next, the culture medium is replaced with the serum-free DMEM supplemented with SHED-CM (1%, 3%, 10%, or 30% (v/v)) containing either anti-HGF antibody (1 μg/mL) or isotype IgG (1 μg/mL) and

incubating in a 5% $CO_2$ incubator at 37 °C for 72 hours. After that, the growth of IMS32 is observed. The results are shown in Figure 9. As shown in the optical microscopic image, IMS32 cultured in normal serum-free medium (DMEM) kept round shape and hardly proliferated (Figures 9(B 1) and 9(B2)), as the same as those before the experiment (Figure 9(A)). In contrast, after 72 hours of culture in the medium supplemented with SHED-CM, the IMS32 cells had elongated shape, indicating that they are well increased (Figure 9(C1) and (C2)).

In order to quantitatively determine the proliferation of IMS32, ATP production in the cultured IMS32 is measured by using CellTiter-Glo 2.0 Cell Viability Assay (Promega). GloMaxDiscover Microplate Rreader GM3000 (Promega) was used as a measuring instrument. The results are shown in Figure 10. In Figure 10, N/C shows the control cultured with DMEM only. Further, statistical processing was performed using one-way ANOVA and Dunnett test, and the results are shown with $p<0.05$ as *, $p<0.01$ as **, and $p<0.001$ as ****.

Figure 10(A) shows that the proliferative capability of IMS32 was significantly enhanced by culturing in SHED-CM-containing medium in a SHED-CM concentration-dependent manner. Also, the proliferative capability IMS32 significantly reduced by adding anti-HGF antibody to the medium containing SHED-CM, compared to adding the control antibody (Figure 10(B)). By this, at least a part of the proliferative enhancement effect of SHED-CM is due to the action of HGF.

In order to examine whether HGF alone can enhance the proliferation of Schwann cells, the same culture experiments as above are performed using IMS32. In this experiment, IMS32 are cultured in DMEM medium supplemented with either SHED-CM (10%, 30%, 50% (v/v)) or recombinant human (rh) HGF (0.1, 1, 10, 50 ng/mL) (R&D) for 72 hours at 37 °C in a 5% $CO_2$ incubator. Then, as described above, the amount of ATP production by IMS32 after incubation is quantitatively determined, and by using this as an indicator, the proliferative capability of IMS32 is examined. The results are shown in Figure 11(A). The results of the statistical test in Figure 11 are indicated as * for $p < 0.05$ and *** for $p < 0.001$. SHED-CM enhanced the proliferative ability of the cells, whereas the medium with only HGF did not (Figure 11(A)).

In addition, the same experiment as above is performed under different conditions in DMEM containing 1% fetal bovine serum (FBS) (Sigma-Aldrich). The results are shown in Figure 11(B). In the absence of FBS, HGF did not enhance the proliferative capability of IMS32, whereas in the presence of FBS, HGF alone enhanced the proliferative capability of IMS32.

These results suggest that in addition to HGF, unidentified molecule X in SHED-CM or serum is required to enhance the enhancement of proliferation ability of Schwann cells.

Next, in order to understand how SHED-CM and HGF promote the proliferation of Schwann cells, we examined the intracellular signaling pathway. Firstly, both of DMEM for the negative control group and SHED-CM are concentrated approximately 10-fold using an Amicon Ultra Centrifugal Filter Units-10 k (Merck Ltd.) removing substances having a molecular weight not higher than 10,000. IMS32 ($2\times10^6$ cells) are suspended in 50 $\mu$L of serum-free DMEM, and then stimulated for 5 minutes by adding 50 $\mu$L of the concentrated medium. As the positive control, 50 $\mu$L of rhHGF prepared at 50 ng/mL in DMEM is also used. After that, each of the cells stimulated as described above is lysed respectively, which are subjected to SDS-PAGE and Western blotting.

The following antibodies are used: anti-Phospho-c-met antibody, anti-c-met antibody (Sigma-Aldrich), anti-Phospho-Erk1/2 antibody (Cell Signaling), anti-Erk1/2 antibody (Cell Signaling), anti-Phospho-AKT antibody (Cell Signaling), anti-AKT antibody (Cell Signaling), anti-Erk1/2 antibody (Cell Signaling), anti-Phospho-AKT antibody (Cell Signaling), and anti-AKT antibody (Cell Signaling). The results are shown in Figure 12.

Both SHED-CM and rhHGF induced phosphorylation of ERK1/2 and AKT, which are located downstream of the HGF receptor c-MET and are important signals for induction of cell proliferation. This suggests that the induction of Schwann cell proliferation and/or regeneration by HGF in SHED-CM is important for the alleviating effect of SHED-CM on neuroinflammatory symptoms.

Next, the proliferation and regeneration of Schwann cells by SHED-CM are qualitatively evaluated. IMS32 is cultured in DMEM containing 50% SHED-CM, 50 ng/mL rhHGF, 50 ng/mL NRG1 (BioLegend), 1 mM DBcMP (Sigma-Aldrich), or 50 ng/mL NRG1 + 1 mM DBcAMP and 5 % FBS, followed by Western blotting as described above. Western blotting is performed using anti-MBP, anti-p75 (GeneTex), and anti-actin (Santa Cruz) antibodies. The results are shown in Figure 13.

The upper row of Figure 13 shows that the amount of MBP, a major component of the myelin sheath, increased in IMS32 cultured in SHED-CM, HGF, and NRG1-containing medium. On the other hand, the middle row of Figure 13 shows that the amount of p75, a receptor for neurotrophic factor, increased only in IMS32 cultured in SHED-CM-containing medium. These results suggest that SHED-CM has the capability to promote Schwann cell maturation.

Since SHED-CM promotes the proliferation of Schwann cells and the regeneration of myelin sheaths, SHED-CM is effective against demyelinating diseases in general. Therefore, the present invention is used in pharmaceutical preparations for the treatment of demyelinating diseases.

(Example 7) Examination of the Mechanism of Action of SHED-CM in the Treatment of CIPN

Finally, it was examined whether administration of SHED-CM improves symptoms in a mouse model of CIPN, which appears peripheral neuropathy artificially induced by chemotherapeutic agents.

(1) Behavioral Pain Test

A total of four intraperitoneal administrations of paclitaxel (80 μg/200 μL) on Days 0, 2, 4, and 6 cause the development of peripheral neuropathy. In addition, either of 200 μL of SHED-CM or control medium DMEM is administered intraperitoneally on Days 1, 3, 5, 7 and consecutive days thereafter for treatment of CIPN. To confirm the efficacy of the treatment, changes in escape responses to non-nociceptive stimulation by microfilaments are compared (von Frey filament test). Changes in escape responses are compared by stimulating hind legs by poking them with microfilaments of fixed stimulus intensity and recording whether or not the mouse escapes by withdrawing its legs as O or X. The average of the minimum stimulus intensity required for escape stimulation was calculated as the 50% threshold value (g) based on the following equation and compared.

50% threshold value described above is calculated by the following equation.

$$50\ \%\ \text{threshold value} = (10\ [X_f + k\delta]\ )/\ 10000$$

$X_f$ : Pressure strength of Filament (Aesthesio (registered trademark); DanMic. Global, Campbell, USA) used lastly. See Figure 15 for filament pressure strength and accompanying information (cited from the Dinamic Global, LLC commentary).

k: It shows the value determined by the O / X sequence. See Figure 16 and Figure 17 for the k value. (cited are cited from ["Quantitative assessment of tactile allodynia in the rat paw"; S.R.Chaplan et al; Journal of Neuroscience Methods; Jul;53(1):55-63.1994: doi: 10.1016/0165-0270(94)90144-9.])

δ : Filament deviation (using 0.224)

The analyzed results are shown in Figure 14. Negative control group mice that did not receive paclitaxel generally remained threshold range between 1.0 and 1.5 g, while CIPN model mice treated with paclitaxel became more sensitive to low stimuli and responded to stimuli as low as 0.5 g or less. In contrast, the SHED-CM-treated CIPN model mice, as treatment continued, required the same stimuli as those given to the negative control group mice to escape. These results indicate that administration of SHED-CM is available in alleviating or treating the neurological disorder CIPN.

## Industrial Applicability

The present invention is available in the field of pharmaceuticals, especially those related to the treatment of neuritis.

[Sequence Listing Free Text]

SEQ ID No. 1: The nucleotide sequence of the DNA fragment containing the gene to be introduced into the cell (1).
SEQ ID No. 2: The nucleotide sequence of the DNA fragment containing the gene to be introduced into the cell (2).
SEQ ID No. 3: The nucleotide sequence of the DNA fragment containing the gene to be introduced into the cell (3).
SEQ ID No. 4: The nucleotide sequence of the DNA fragment containing the gene to be introduced into the cell (4).
SEQ ID No. 5: The nucleotide sequence of the DNA fragment containing the gene to be introduced into the cell (5).
SEQ ID No. 6: The nucleotide sequence of the DNA fragment containing the gene to be introduced into the cell (6).
SEQ ID No. 7: The primer for PCR (1).
SEQ ID No.8: The primer for PCR (2).
SEQ ID No. 9: The primer for PCR (3).
SEQ ID No. 10: The primer for PCR (4).
SEQ ID No. 11: The primer for PCR (5).
SEQ ID No. 12: The primer for PCR (6).
SEQ ID No. 13: The primer for PCR (7).
SEQ ID No. 14: The primer for PCR (8).

SEQ ID No. 15: The primer for PCR (9).
SEQ ID No. 16: The primer for PCR (10).
SEQ ID No. 17: The primer for PCR (11).
SEQ ID No. 18: The primer for PCR (12).
SEQ ID No. 19: The primer for PCR (13).
SEQ ID No. 20: The primer for PCR (14).
SEQ ID No. 21: The primer for PCR (15).
SEQ ID No. 22: The primer for PCR (16).
SEQ ID No. 23: The primer for PCR (17).
SEQ ID No. 24: The primer for PCR (18).
SEQ ID No. 25: The primer for PCR (19).
SEQ ID No. 26: The primer for PCR (20).

[Sequence Listing]

C:\Users\AYAFUNE11_2\Desktop\F23CY01WO\F23CY01WO.xml

## Claims

**1.** A composition that regenerates myelin sheath or proliferates Schwann cells, wherein the composition contains a culture supernatant of immortalized mesenchymal stem cells to which a gene set of telomerase reverse transcription enzyme (TERT) and two or three genes selected from a group consisting of Bmi-1 gene, HPV16-E6 gene, and HPV16-E7 gene are introduced.

**2.** The composition according to claim 1, wherein the gene set consists of Bmi-1 gene, HPVE16-E6, HPV16-E7, and hTERT.

**3.** The composition according to claims 1 or 2, wherein the immortalized mesenchymal stem cells are prepared from dental pulp stem cells.

**4.** The composition according to claim 3, wherein the dental pulp stem cells are dental pulp stem cells derived from humans.

**5.** The composition according to claim 4, wherein the composition contains culture supernatant obtained from the immortalized mesenchymal stem cells, which are immortalized by introducing said gene set with virus vectors, and by culturing them for 60 to 84 hours.

**6.** The composition according to claim 4, wherein the composition contains at least a component that acts on c-MET, a hepatocyte growth factor (HGF) receptor.

**7.** A therapeutic pharmaceutical preparation for demyelinating diseases that comprises the composition claimed in claim 4 as an active ingredient.

**8.** The pharmaceutical preparation according to claim 7, wherein the preparation contains 10 to 90 weight % of the composition, when the total weight of the preparation is 100 %.

**9.** The pharmaceutical preparation according to claim 7, wherein the demyelinating disease is central nerve one, and anyone selected from a group consisting of multiple sclerosis, neuromyelitis optica, acute disseminated encephalomyelitis, atopic myelitis, progressive multifocal leukoencephalopathy, HTLB-1-related myelopathy, HIV-related leukoencephalopathy, subacute sclerosing panencephalitis, osmotic demyelinating syndrome and drug-induced leukoencephalopathy.

**10.** The pharmaceutical preparation according to claim 7, wherein the demyelinating disease is peripheral nerve one, and anyone selected from a group consisting of Guillain-Barré syndrome, Fisher syndrome, diabetic neuropathy, chronic inflammatory demyelinating polyradiculoneuropathy, multifocal motor neuropathy, chemotherapeutic agent-induced peripheral neuropathy, and herpes zoster.

**11.** The pharmaceutical preparation according to claim 7, wherein the preparation is a dosage form selected from a group consisting of powders, tablets, capsules, liquids, injectables, and plasters.

**12.** A pharmaceutical preparation for the treatment of chemotherapeutic agent-induced peripheral neuropathy that comprises a composition as an active ingredient, wherein the composition contains a culture supernatant of immortalized mesenchymal stem cells to which a gene set of telomerase reverse transcription enzyme (TERT) and two or three genes selected from a group consisting of Bmi-1 gene, HPV16-E6 gene, and HPV16-E7 gene are introduced.

**13.** The pharmaceutical preparation according to claim 12, wherein the chemotherapeutic agent is an anti-cancer agent.

**14.** The pharmaceutical preparation according to claim 13, wherein the anti-cancer agent is a vinca alkaloid preparation or a taxane preparation.

# Figure 1

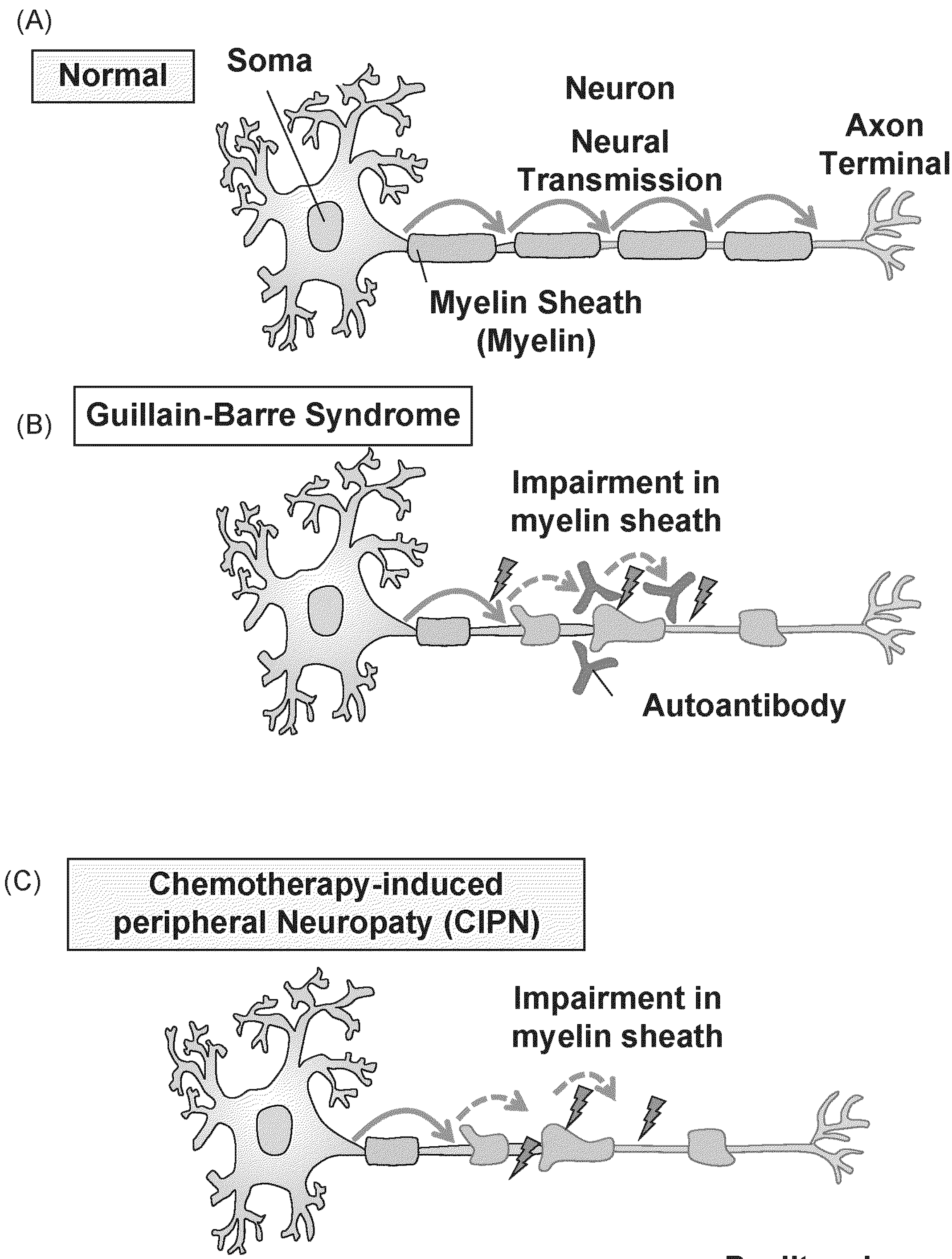


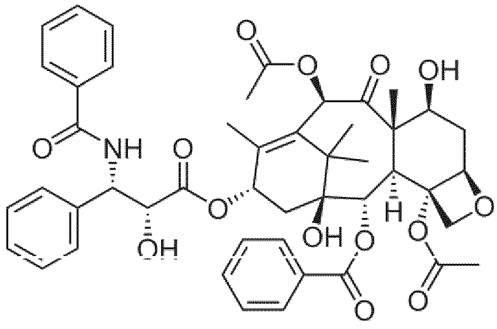

Paclitaxel

# Figure 2

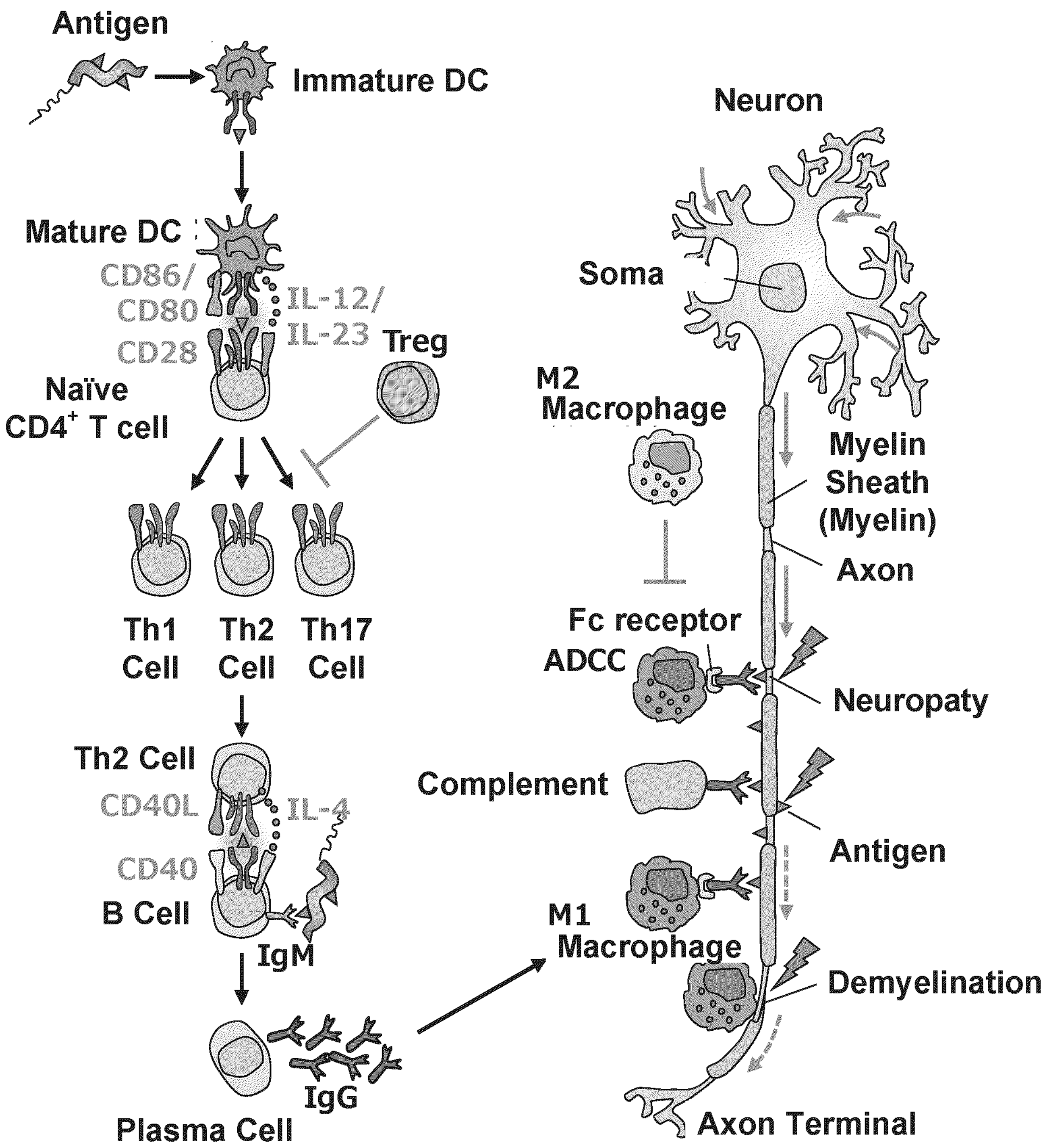
Antigen
Immature DC
Mature DC
CD86/
CD80
CD28
IL-12/
IL-23
Treg
Naïve
CD4+ T cell
Th1
Cell
Th2
Cell
Th17
Cell
Th2 Cell
CD40L
IL-4
CD40
B Cell
IgM
IgG
Plasma Cell
Neuron
Soma
M2
Macrophage
Myelin
Sheath
(Myelin)
Axon
Fc receptor
ADCC
Neuropaty
Complement
Antigen
M1
Macrophage
Demyelination
Axon Terminal

Figure 3

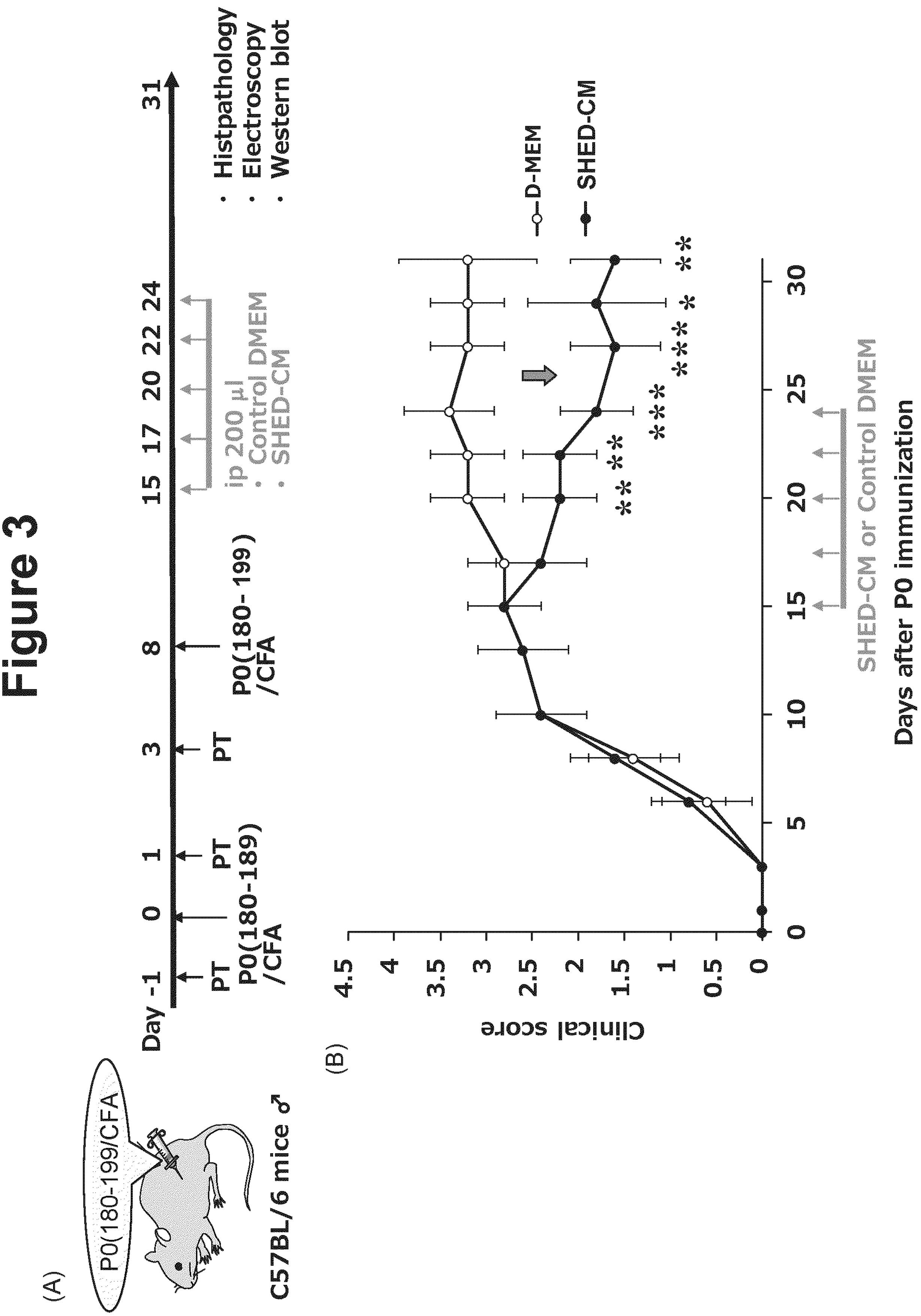
(A)
P0(180-199)/CFA
C57BL/6 mice ♂
Day -1
0
1
3
8
15
17
20
22
24
31
PT
P0(180-189)
/CFA
PT
PT
P0(180- 199)
/CFA
ip 200 µl
· Control DMEM
· SHED-CM
· Histpathology
· Electroscopy
· Western blot
(B)
Clinical score
4.5
4
3.5
3
2.5
2
1.5
1
0.5
0
0
5
10
15
20
25
30
D-MEM
SHED-CM
**
**
***
***
*
**
SHED-CM or Control DMEM
Days after P0 immunization

Figure 4

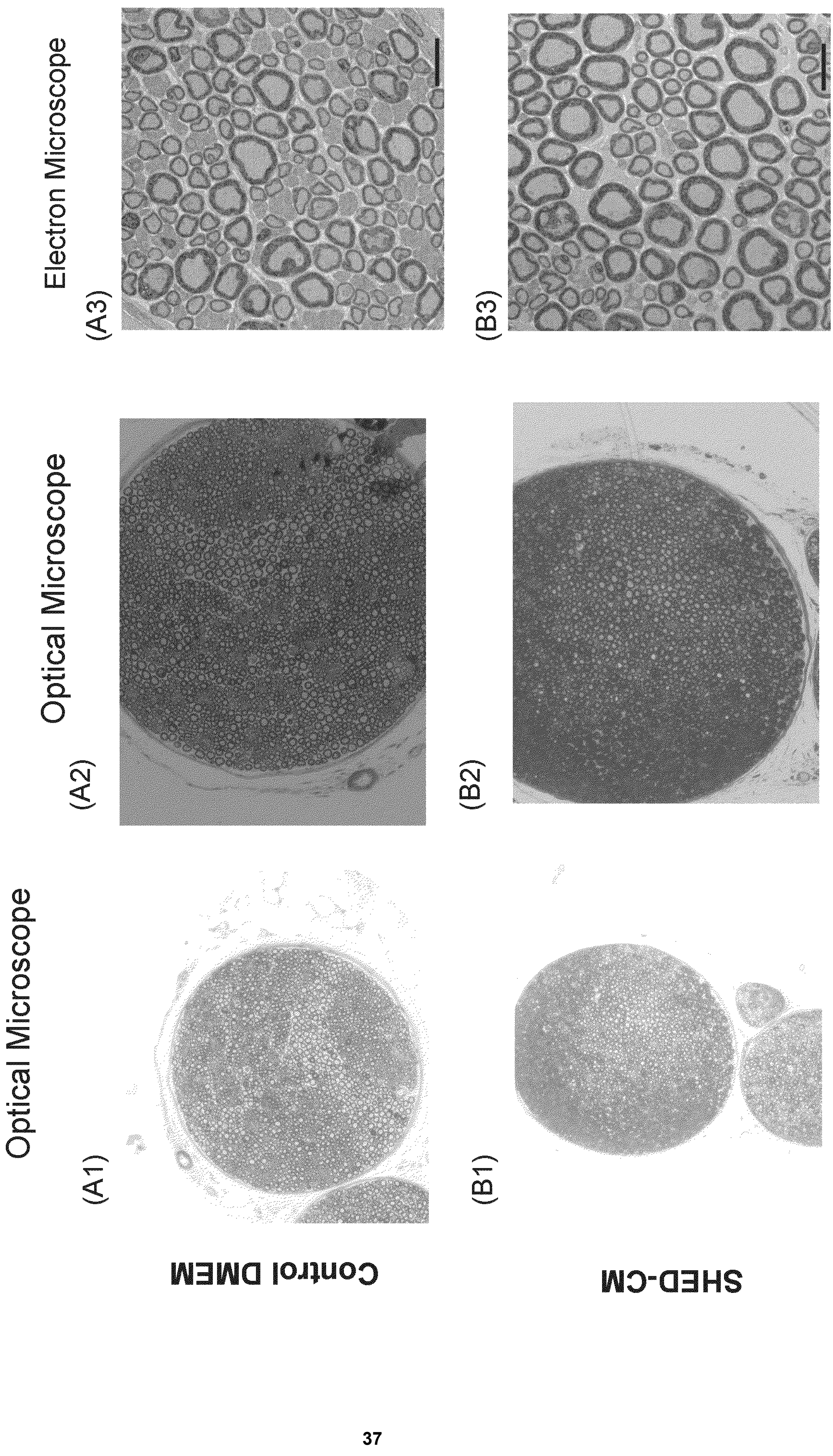
Optical Microscope
Optical Microscope
Electron Microscope
(A1)
(A2)
(A3)
(B1)
(B2)
(B3)
Control DMEM
SHED-CM

Figure 5

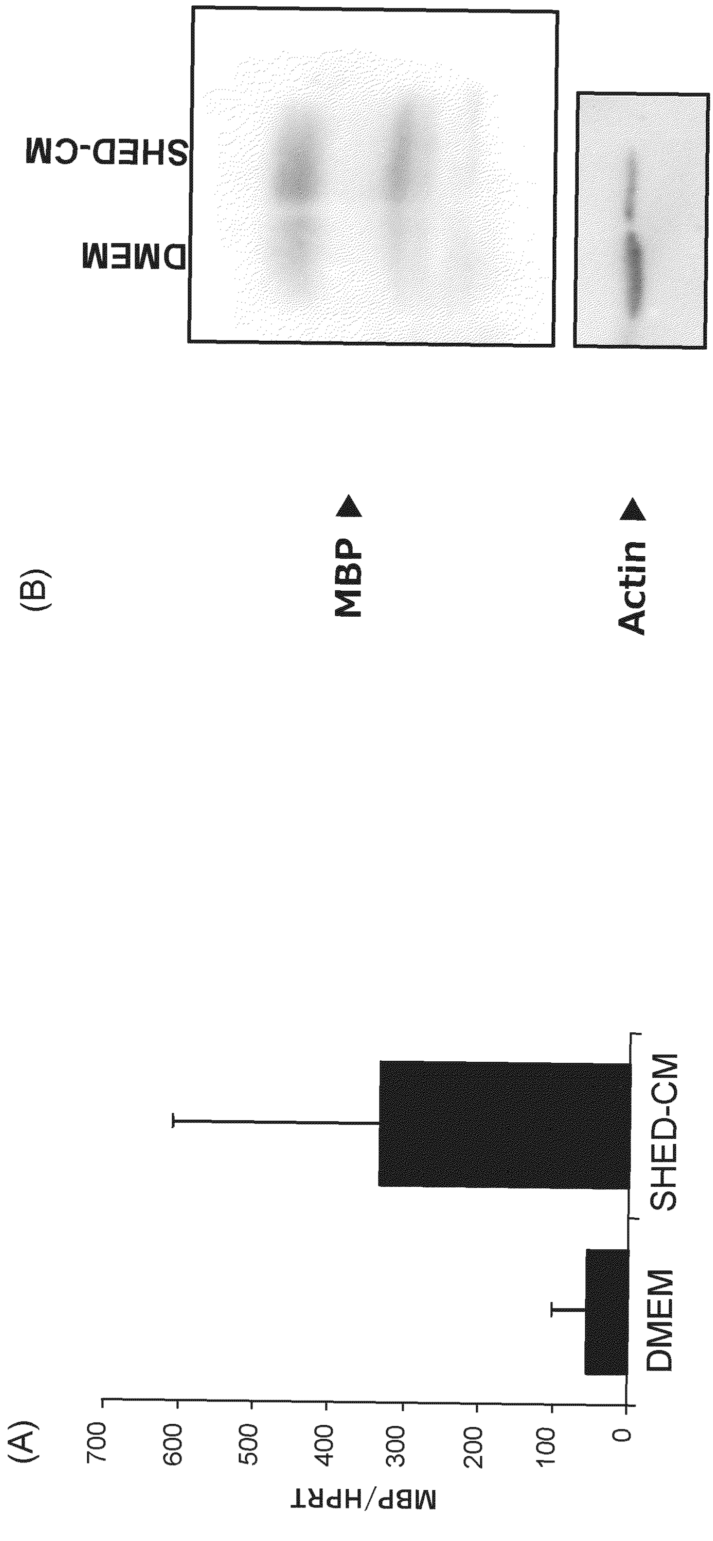
(A)
MBP/HPRT
700
600
500
400
300
200
100
0
DMEM
SHED-CM
(B)
DMEM
SHED-CM
MBP
Actin

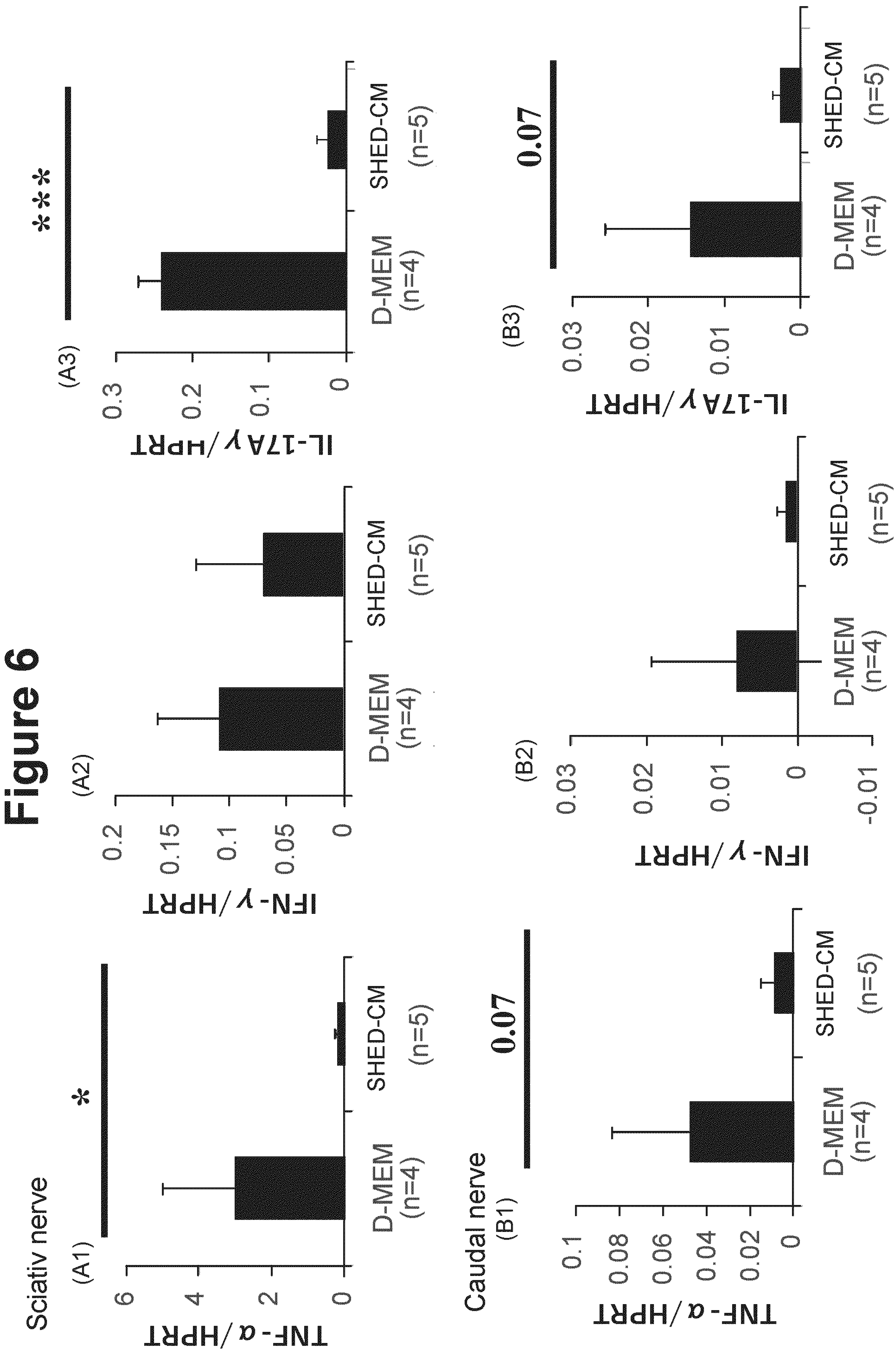
Figure 6
Sciativ nerve
(A1)
TNF-α/HPRT
6
4
2
0
*
D-MEM (n=4)
SHED-CM (n=5)
(A2)
IFN-γ/HPRT
0.2
0.15
0.1
0.05
0
D-MEM (n=4)
SHED-CM (n=5)
(A3)
IL-17Aγ/HPRT
0.3
0.2
0.1
0
***
D-MEM (n=4)
SHED-CM (n=5)
Caudal nerve
(B1)
TNF-α/HPRT
0.1
0.08
0.06
0.04
0.02
0
0.07
D-MEM (n=4)
SHED-CM (n=5)
(B2)
IFN-γ/HPRT
0.03
0.02
0.01
0
-0.01
D-MEM (n=4)
SHED-CM (n=5)
(B3)
IL-17Aγ/HPRT
0.03
0.02
0.01
0
0.07
D-MEM (n=4)
SHED-CM (n=5)

Figure 7

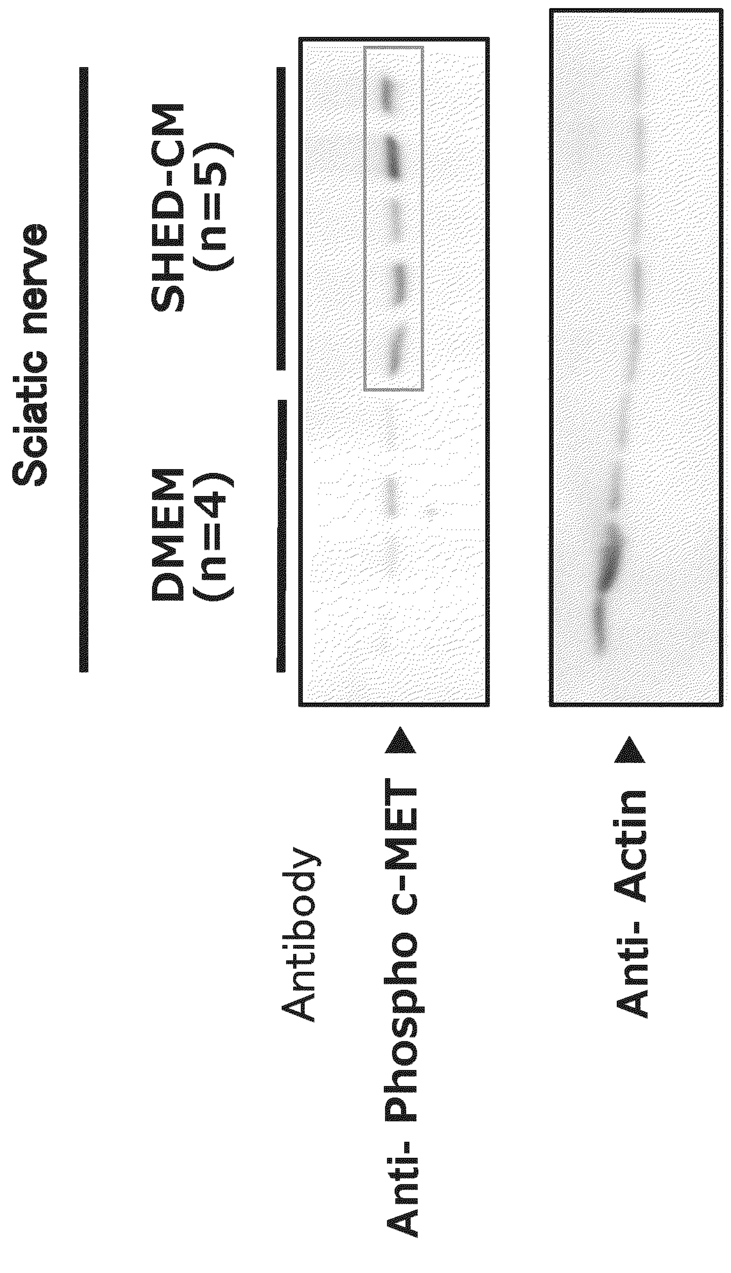
Sciatic nerve
DMEM
(n=4)
SHED-CM
(n=5)
Antibody
Anti- Phospho c-MET
Anti- Actin

Figure 8

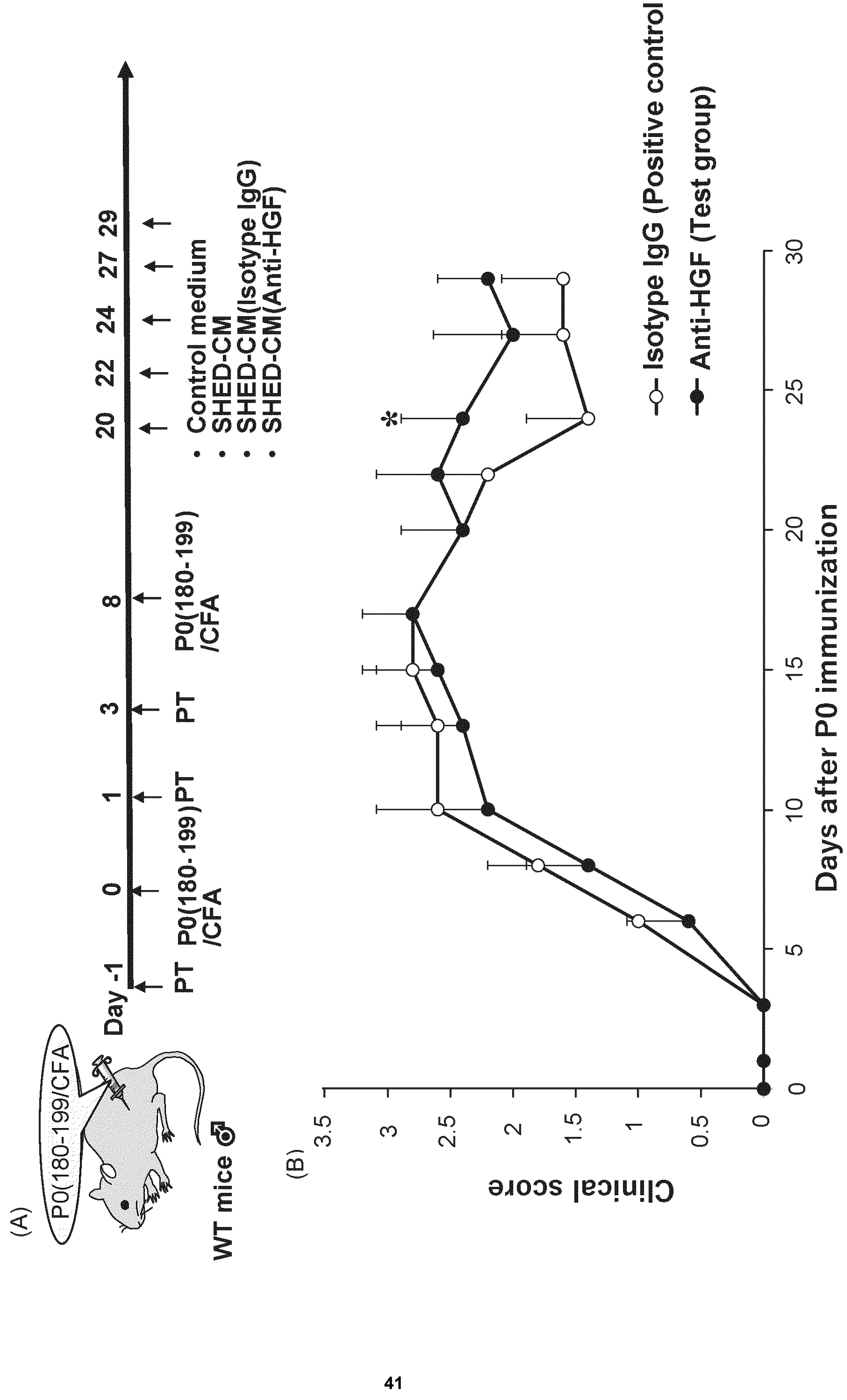
(A)
P0(180-199/CFA
WT mice ♂
Day -1
0
1
3
8
20 22 24 27 29
PT
P0(180-199)
/CFA
P0(180-199)PT
PT
P0(180-199)
/CFA
: Control medium
: SHED-CM
: SHED-CM(Isotype IgG)
: SHED-CM(Anti-HGF)
(B)
Clinical score
0
0.5
1
1.5
2
2.5
3
3.5
0
5
10
15
20
25
30
Days after P0 immunization
*
Isotype IgG (Positive control 2)
Anti-HGF (Test group)

# Figure 9

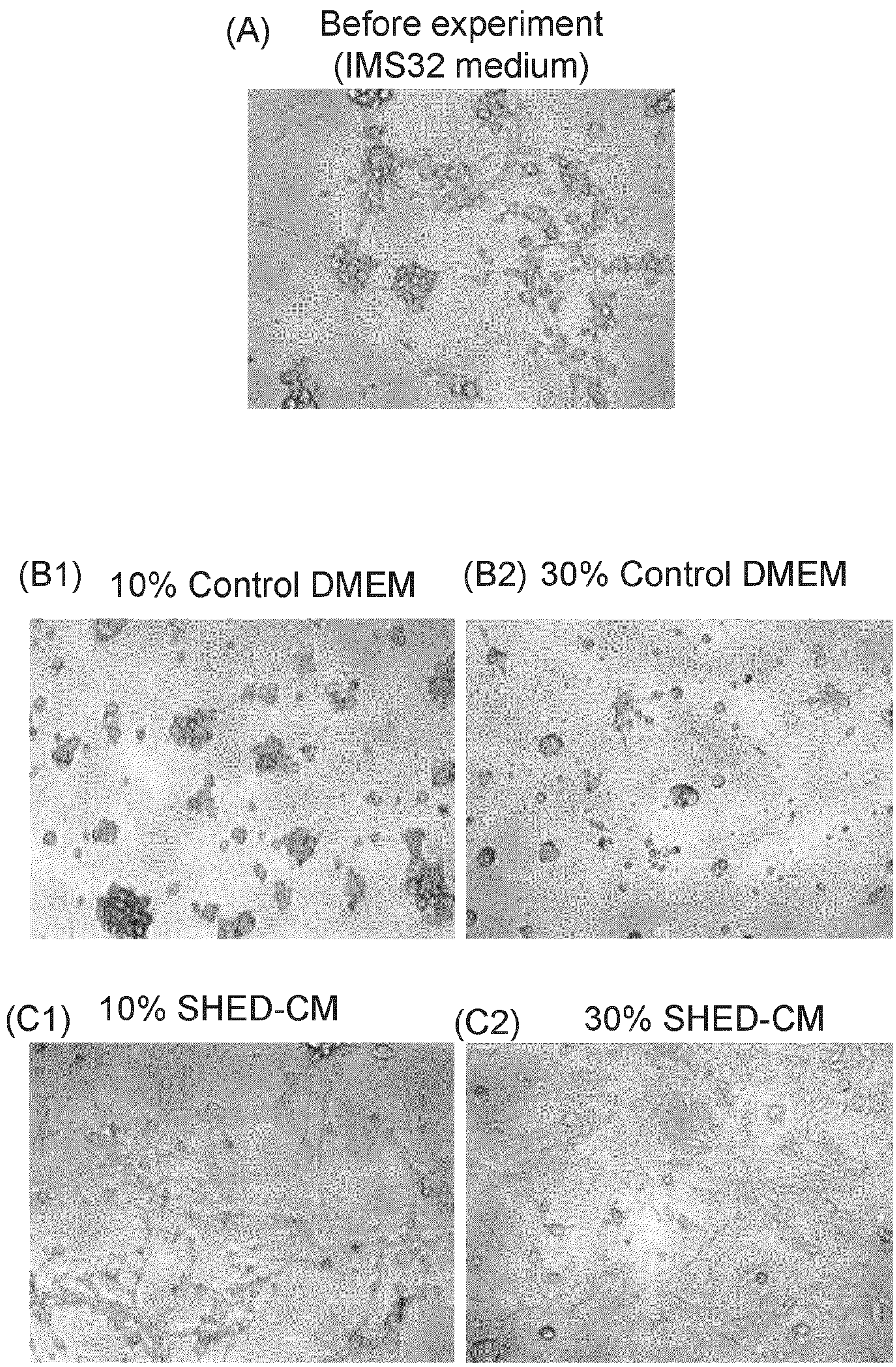
(A) Before experiment
(IMS32 medium)
(B1) 10% Control DMEM
(B2) 30% Control DMEM
(C1) 10% SHED-CM
(C2) 30% SHED-CM

# Figure 10

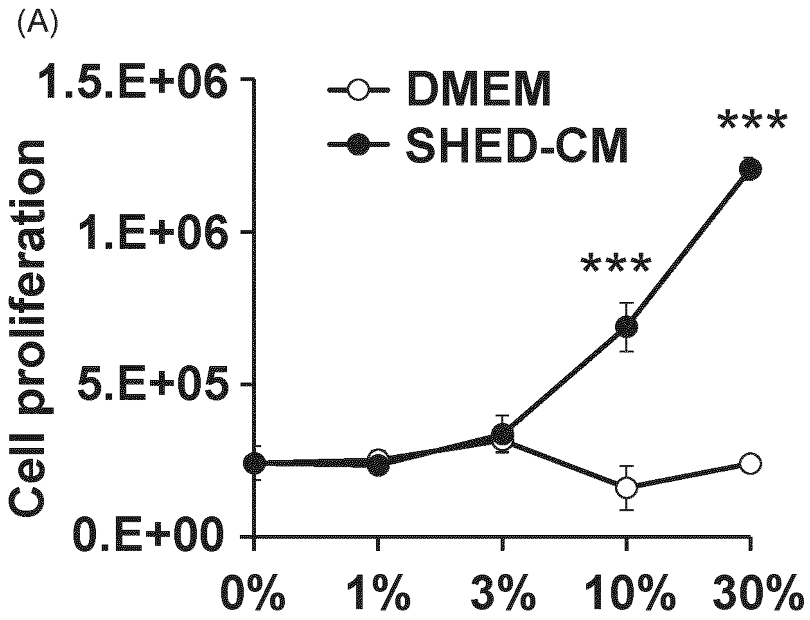
(A)
1.5.E+06
1.E+06
5.E+05
0.E+00
Cell proliferation
DMEM
SHED-CM
***
***
0%
1%
3%
10%
30%

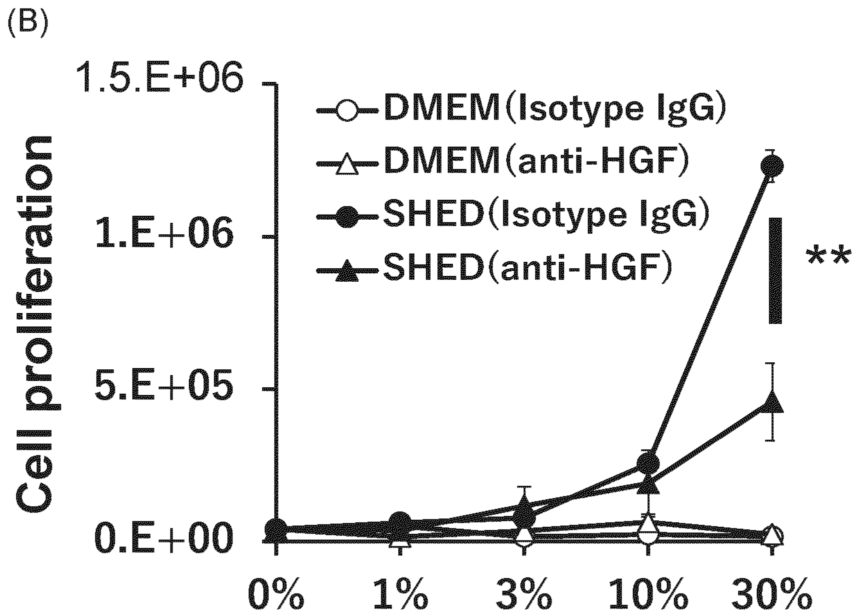
(B)
1.5.E+06
1.E+06
5.E+05
0.E+00
Cell proliferation
DMEM(Isotype IgG)
DMEM(anti-HGF)
SHED(Isotype IgG)
SHED(anti-HGF)
**
0%
1%
3%
10%
30%

# Figure 11

(A)

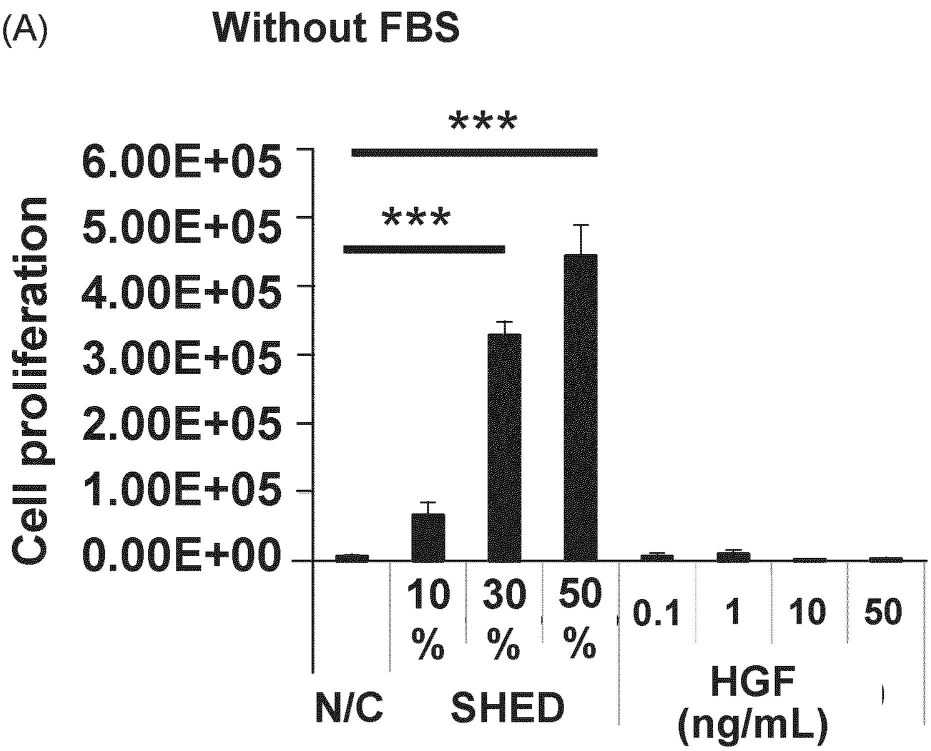
Without FBS
***
***
Cell proliferation
6.00E+05
5.00E+05
4.00E+05
3.00E+05
2.00E+05
1.00E+05
0.00E+00
10 %
30 %
50 %
0.1
1
10
50
N/C
SHED
HGF (ng/mL)

(B)

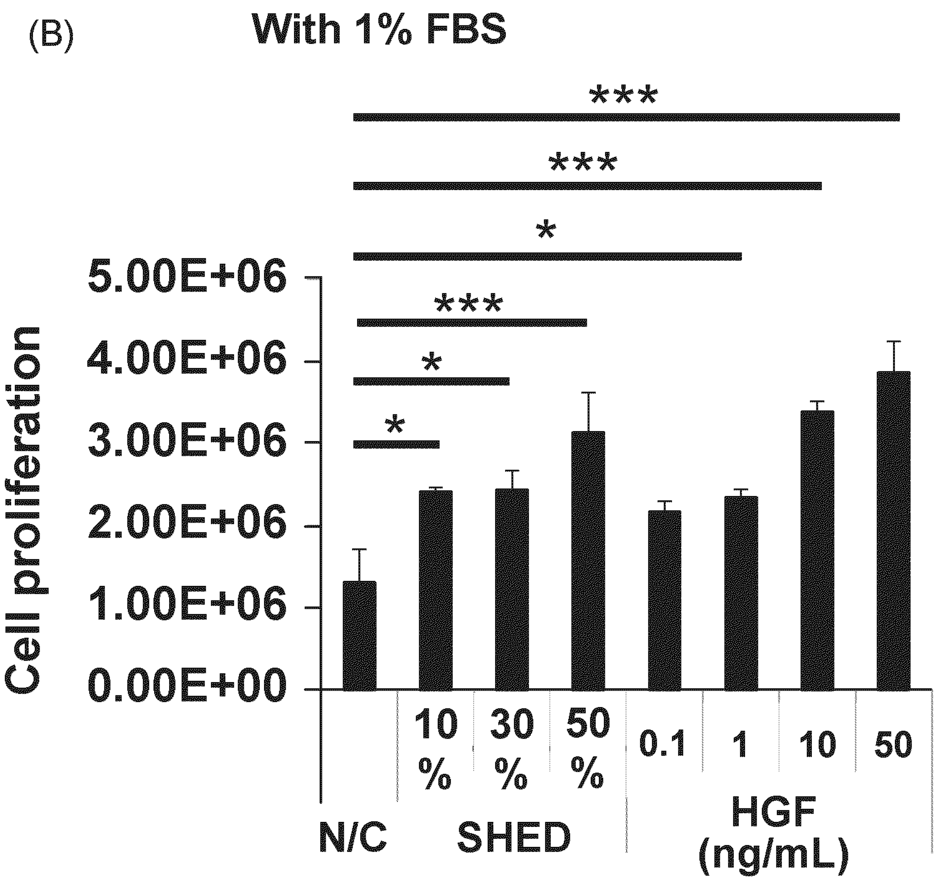
With 1% FBS
***
***
*
***
*
*
Cell proliferation
5.00E+06
4.00E+06
3.00E+06
2.00E+06
1.00E+06
0.00E+00
10 %
30 %
50 %
0.1
1
10
50
N/C
SHED
HGF (ng/mL)

# Figure 12

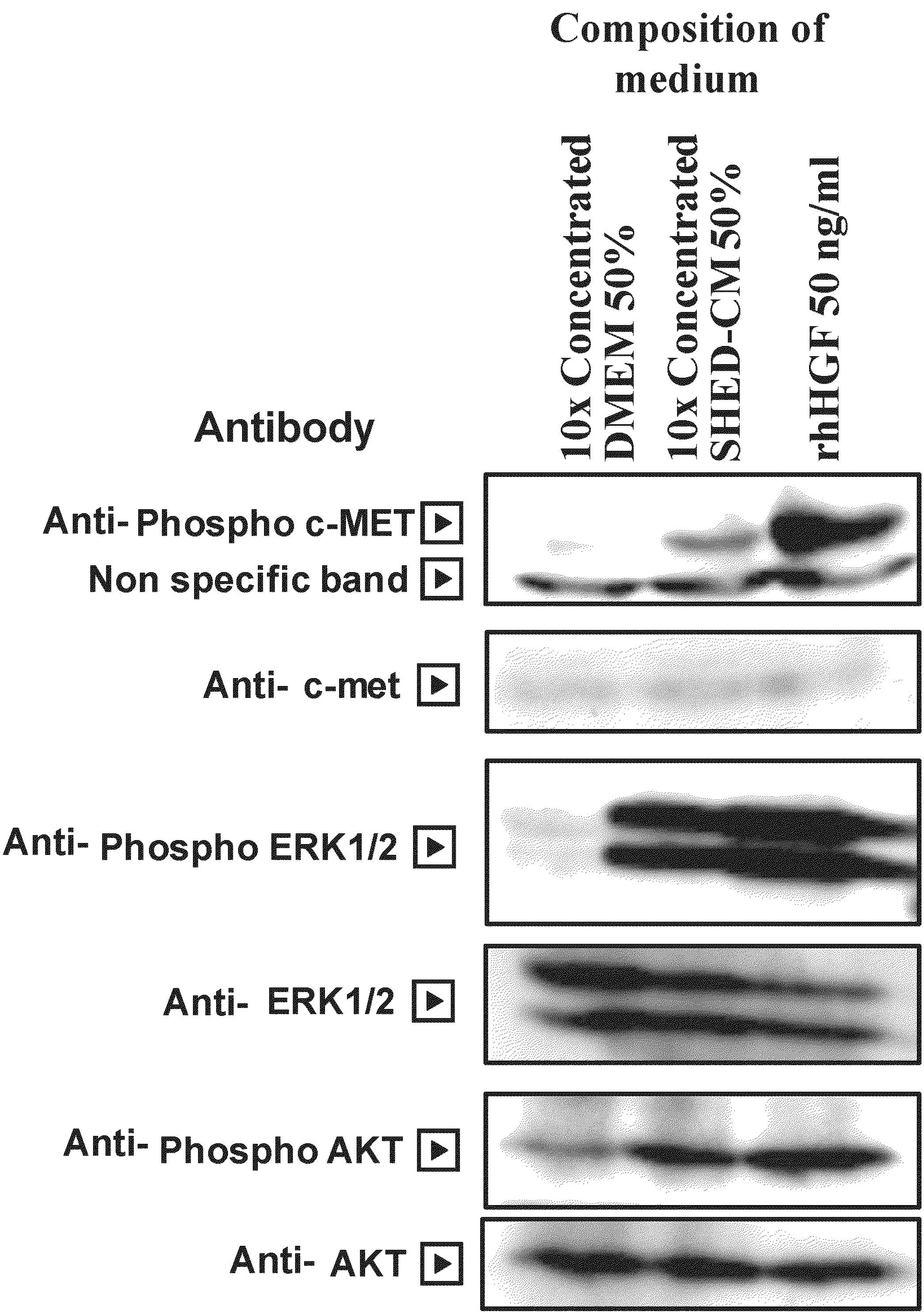
Composition of medium
10x Concentrated DMEM 50%
10x Concentrated SHED-CM 50%
rhHGF 50 ng/ml
Antibody
Anti- Phospho c-MET
Non specific band
Anti- c-met
Anti- Phospho ERK1/2
Anti- ERK1/2
Anti- Phospho AKT
Anti- AKT

# Figure 13

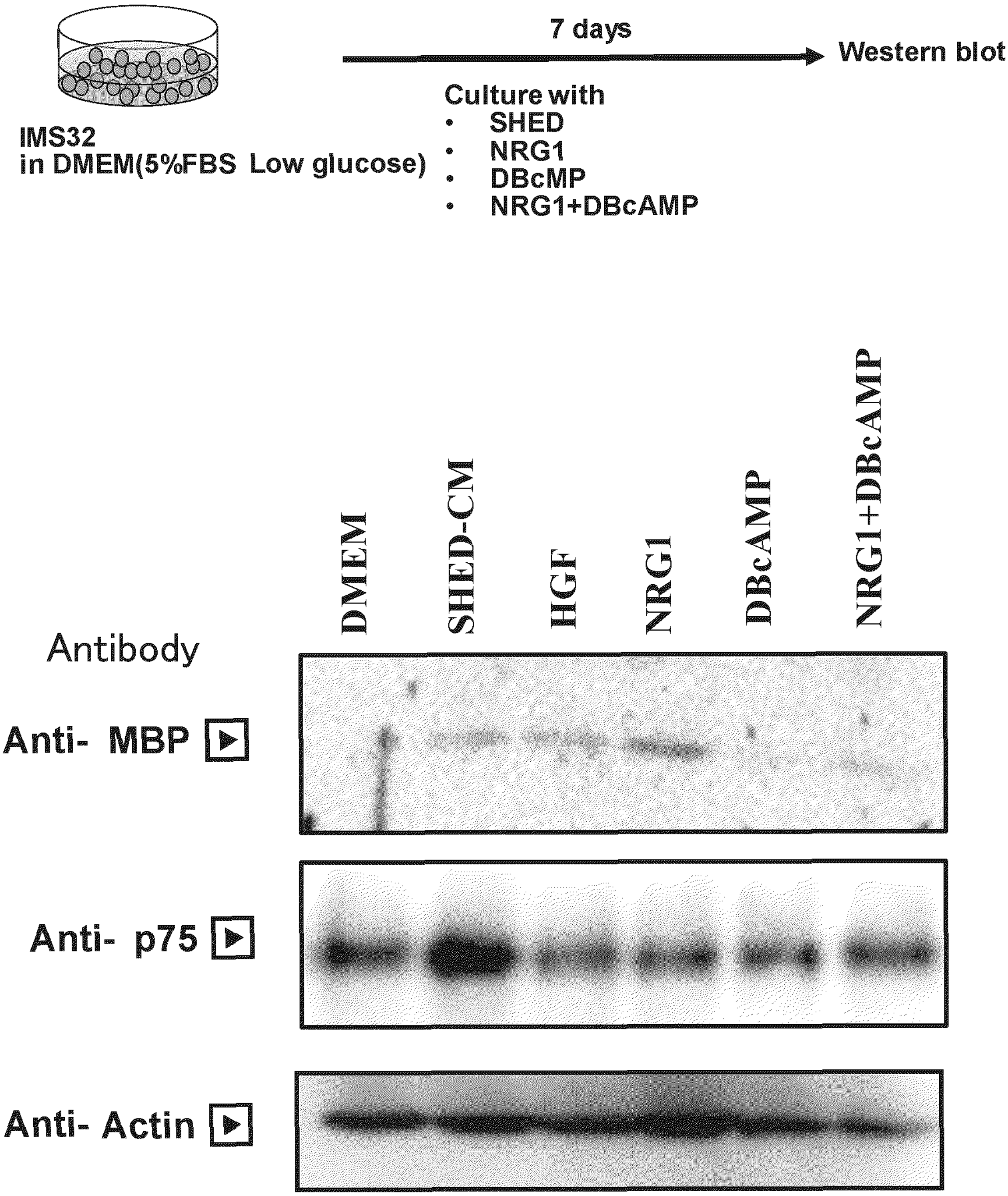
7 days
Western blot
IMS32
in DMEM(5%FBS Low glucose)
Culture with
• SHED
• NRG1
• DBcMP
• NRG1+DBcAMP
DMEM
SHED-CM
HGF
NRG1
DBcAMP
NRG1+DBcAMP
Antibody
Anti- MBP
Anti- p75
Anti- Actin

Figure 14

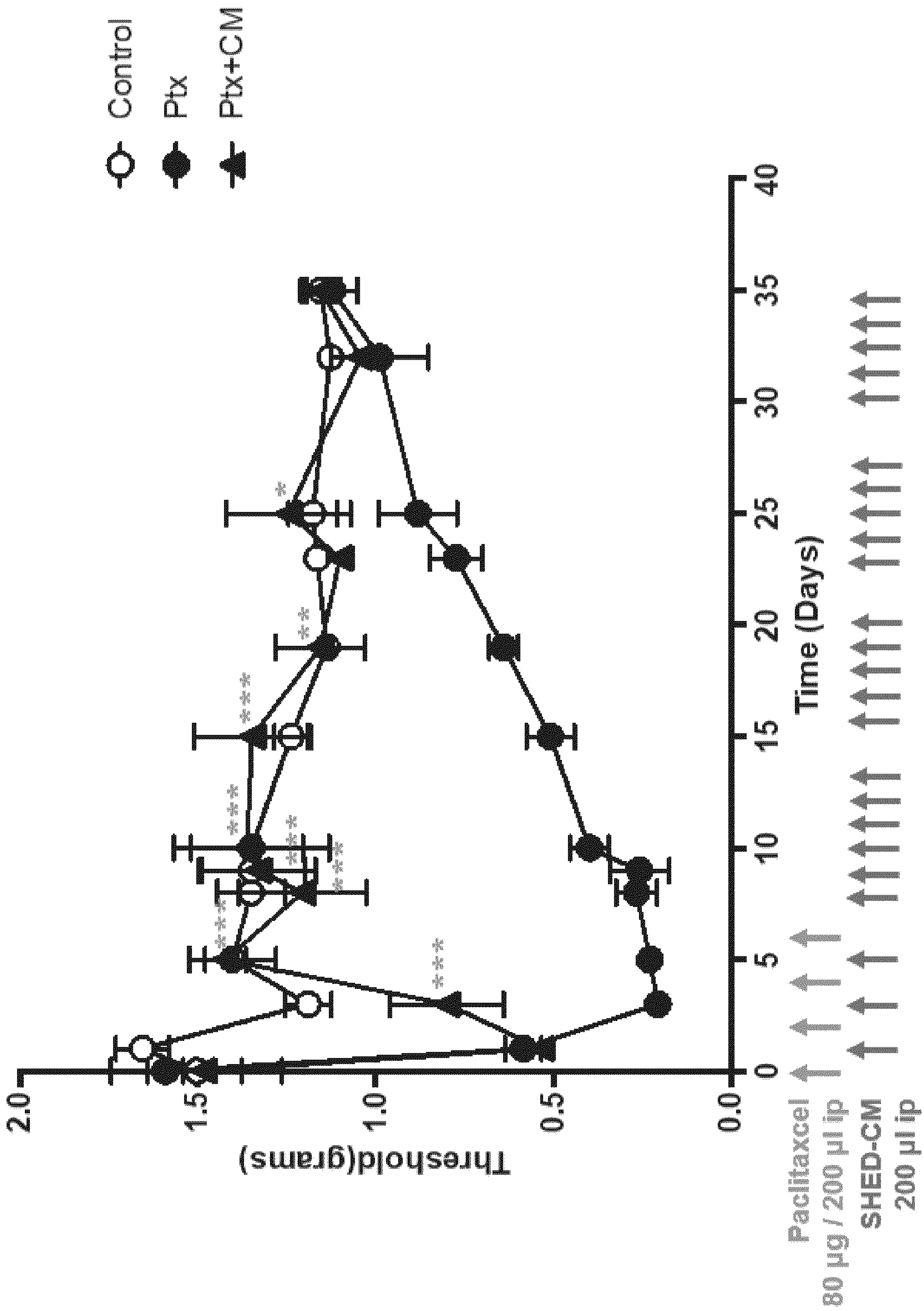
Control
Ptx
Ptx+CM
Threshold(grams)
2.0
1.5
1.0
0.5
0.0
0
5
10
15
20
25
30
35
40
Time (Days)
Paclitaxcel
80 µg / 200 µl ip
SHED-CM
200 µl ip

# Figure 15

Aesthesio Precision Tactile Sensory Evaluator Data Chart

| Color | Evaluator Size | Catalog Item No. | Target Force (grams) | Target Force (milliNewtons) | Theoretical Pressure LBS/Sq.Inch | Theoretical Pressure Grams/Sq.mm |
|---|---|---|---|---|---|---|
| Green | 1.65 | 514001 | 0.008 | 0.08 | 3.59 | 2.53 |
| | 2.36 | 514002 | 0.02 | 0.20 | 6.23 | 4.39 |
| | 2.44 | 514003 | 0.04 | 0.40 | 7.01 | 4.93 |
| | 2.83 | 514004 | 0.07 | 0.70 | 7.85 | 5.53 |
| Blue | 3.22 | 514005 | 0.16 | 1.6 | 12.5 | 8.77 |
| | 3.61 | 514006 | 0.40 | 3.9 | 22.9 | 16.1 |
| Purple | 3.84 | 514007 | 0.60 | 5.9 | 26.1 | 18.4 |
| | 4.08 | 514008 | 1.0 | 9.8 | 34.6 | 24.4 |
| | 4.17 | 514009 | 1.4 | 13.7 | 39.6 | 27.9 |
| | 4.31 | 514010 | 2.0 | 19.6 | 39.0 | 27.4 |
| Red | 4.56 | 514011 | 4.0 | 39.2 | 57.2 | 40.3 |
| | 4.74 | 514012 | 6.0 | 58.8 | 74.8 | 52.6 |
| | 4.93 | 514013 | 8.0 | 78.4 | 87.6 | 61.7 |
| | 5.07 | 514014 | 10 | 98.0 | 97.0 | 68.3 |
| | 5.18 | 514015 | 15 | 147 | 117 | 82.0 |
| | 5.46 | 514016 | 26 | 255 | 151 | 106 |
| | 5.88 | 514017 | 60 | 588 | 200 | 141 |
| | 6.10 | 514018 | 100 | 980 | 274 | 193 |
| | 6.45 | 514019 | 180 | 1760 | 316 | 222 |
| Orng | 6.65 | 514020 | 300 | 2940 | 416 | 292 |

Calculated rounded numbers.
(Conversion factor 9.80665)

# Figure 16

| Pattern | Value for k | Pattern | Value for k | Pattern | Value for k | Pattern | Value for k |
|---|---|---|---|---|---|---|---|
| OX | −0.5 | OOOXOOOO | −0.547 | XO | 0.5 | XXXOXXXX | 0.547 |
| OOX | −0.388 | OOOOXOOOO | −0.547 | XXO | 0.388 | XXXXOXXXX | 0.547 |
| OOOX | −0.378 | OXOOOX | −1.25 | XXXO | 0.378 | XOXXXO | 1.25 |
| OOOOX | −0.377 | OOXOOOX | −1.247 | XXXXO | 0.377 | XXOXXXO | 1.247 |
| OXO | 0.842 | OOOXOOOX | −1.246 | XOX | −0.842 | XXXOXXXO | 1.246 |
| OOXO | 0.89 | OOOOXOOOX | −1.246 | XXOX | −0.89 | XXXXOXXXO | 1.246 |
| OOOXO | 0.894 | OXOOXO | 0.372 | XXXOX | −0.894 | XOXXOX | −0.372 |
| OOOOXO | 0.894 | OOXOOXO | 0.38 | XXXXOX | −0.894 | XXOXXOX | −0.38 |
| OXX | −0.178 | OOOXOOXO | 0.381 | XOO | 0.178 | XXXOXXOX | −0.381 |
| OOXX | 0 | OOOOXOOXO | 0.381 | XXOO | 0 | XXXXOXXOX | −0.381 |
| OOOXX | 0.026 | OXOOXX | −0.169 | XXXOO | −0.026 | XOXXOO | 0.169 |
| OOOOXX | 0.028 | OOXOOXX | −0.144 | XXXXOO | −0.028 | XXOXXOO | 0.144 |
| OXOO | 0.299 | OOOXOOXX | −0.142 | XOXX | −0.299 | XXXOXXOO | 0.142 |
| OOXOO | 0.314 | OOOOXOOXX | −0.142 | XXOXX | −0.314 | XXXXOXXOO | 0.142 |
| OOOXOO | 0.315 | OXOXOO | 0.022 | XXXOXX | −0.315 | XOXOXX | −0.022 |
| OOOOXOO | 0.315 | OOXOXOO | 0.039 | XXXXOXX | −0.315 | XXOXOXX | −0.039 |
| OXOX | −0.5 | OOOXOXOO | 0.04 | XOXO | 0.5 | XXXOXOXX | −0.04 |
| OOXOX | −0.439 | OOOOXOXOO | 0.04 | XXOXO | 0.439 | XXXXOXOXX | −0.04 |
| OOOXOX | −0.432 | OXOXOX | −0.5 | XXXOXO | 0.432 | XOXOXO | 0.5 |
| OOOOXOX | −0.432 | OOXOXOX | −0.458 | XXXXOXO | 0.432 | XXOXOXO | 0.458 |
| OXXO | 1 | OOOXOXOX | −0.453 | XOOX | −1 | XXXOXOXO | 0.453 |
| OOXXO | 1.122 | OOOOXOXOX | −0.453 | XXOOX | −1.122 | XXXXOXOXO | 0.453 |
| OOOXXO | 1.139 | OXOXXO | 1.169 | XXXOOX | −1.139 | XOXOOX | −1.169 |
| OOOOXXO | 1.14 | OOXOXXO | 1.237 | XXXXOOX | −1.14 | XXOXOOX | −1.237 |
| OXXX | 0.194 | OOOXOXXO | 1.247 | XOOO | −0.194 | XXXOXOOX | −1.247 |
| OOXXX | 0.449 | OOOOXOXXO | 1.248 | XXOOO | −0.449 | XXXXOXOOX | −1.248 |
| OOOXXX | 0.5 | OXOXXX | 0.611 | XXXOOO | −0.5 | XOXOOO | −0.611 |
| OOOOXXX | 0.506 | OOXOXXX | 0.732 | XXXXOOO | −0.506 | XXOXOOO | −0.732 |
| OXOOO | −0.157 | OOOXOXXX | 0.756 | XOXXX | 0.157 | XXXOXOOO | −0.756 |
| OOXOOO | −0.154 | OOOOXOXXX | 0.758 | XXOXXX | 0.154 | XXXXOXOOO | −0.758 |

# Figure 17

| Pattern | Value for k | Pattern | Value for k | Pattern | Value for k | Pattern | Value for k |
|---|---|---|---|---|---|---|---|
| OOOXOOO | −0.154 | OXXOOO | −0.296 | XXXOXXX | 0.154 | XOOXXX | 0.296 |
| OOOOXOOO | −0.154 | OOXXOOO | −0.266 | XXXXOXXX | 0.154 | XXOOXXX | 0.266 |
| OXOOX | −0.878 | OOOXXOOO | −0.263 | XOXXO | 0.878 | XXXOOXXX | 0.263 |
| OOXOOX | −0.861 | OOOOXXOOO | −0.263 | XXOXXO | 0.861 | XXXXOOXXX | 0.263 |
| OOOXOOX | −0.86 | OXXOOX | −0.831 | XXXOXXO | 0.86 | XOOXXO | 0.831 |
| OOOOXOOX | −0.86 | OOXXOOX | −0.763 | XXXXOXXO | 0.86 | XXOOXXO | 0.763 |
| OXOXO | 0.701 | OOOXXOOX | −0.753 | XOXOX | −0.701 | XXXOOXXO | 0.753 |
| OOXOXO | 0.737 | OOOOXXOOX | −0.752 | XXOXOX | −0.737 | XXXXOOXXO | 0.752 |
| OOOXOXO | 0.741 | OXXOXO | 0.831 | XXXOXOX | −0.741 | XOOXOX | −0.831 |
| OOOOXOXO | 0.741 | OOXXOXO | 0.935 | XXXXOXOX | −0.741 | XXOOXOX | −0.935 |
| OXOXX | 0.084 | OOOXXOXO | 0.952 | XOXOO | −0.084 | XXXOOXOX | −0.952 |
| OOXOXX | 0.169 | OOOOXXOXO | 0.954 | XXOXOO | −0.169 | XXXXOOXOX | −0.954 |
| OOOXOXX | 0.181 | OXXOXX | 0.296 | XXXOXOO | −0.181 | XOOXOO | −0.296 |
| OOOOXOXX | 0.182 | OOXXOXX | 0.463 | XXXXOXOO | −0.182 | XXOOXOO | −0.463 |
| OXXOO | 0.305 | OOOXXOXX | 0.5 | XOOXX | −0.305 | XXXOOXOO | −0.5 |
| OOXXOO | 0.372 | OOOOXXOXX | 0.504 | XXOOXX | −0.372 | XXXXOOXOO | −0.504 |
| OOOXXOO | 0.38 | OXXXOO | 0.5 | XXXOOXX | −0.38 | XOOOXX | −0.5 |
| OOOOXXOO | 0.381 | OOXXXOO | 0.648 | XXXXOOXX | −0.381 | XXOOOXX | −0.648 |
| OXXOX | −0.305 | OOOXXXOO | 0.678 | XOOXO | 0.305 | XXXOOOXX | −0.678 |
| OOXXOX | −0.169 | OOOOXXXOO | 0.681 | XXOOXO | 0.169 | XXXXOOOXX | −0.681 |
| OOOXXOX | −0.144 | OXXXOX | −0.043 | XXXOOXO | 0.144 | XOOOXO | 0.043 |
| OOOOXXOX | −0.142 | OOXXXOX | 0.187 | XXXXOOXO | 0.142 | XXOOOXO | −0.187 |
| OXXXO | 1.288 | OOOXXXOX | 0.244 | XOOOX | −1.288 | XXXOOOXO | −0.244 |
| OOXXXO | 1.5 | OOOOXXXOX | 0.252 | XXOOOX | −1.5 | XXXXOOOXO | −0.252 |
| OOOXXXO | 1.544 | OXXXXO | 1.603 | XXXOOOX | −1.544 | XOOOOX | −1.603 |
| OOOOXXXO | 1.549 | OOXXXXO | 1.917 | XXXXOOOX | −1.549 | XXOOOOX | −1.917 |
| OXXXX | 0.555 | OOOXXXXO | 2 | XOOOO | −0.555 | XXXOOOOX | −2 |
| OOXXXX | 0.897 | OOOOXXXXO | 2.014 | XXOOOO | −0.897 | XXXXOOOOX | −2.014 |
| OOOXXXX | 0.985 | OXXXXX | 0.893 | XXXOOOO | −0.985 | XOOOOO | −0.983 |
| OOOOXXXX | 1 | OOXXXXX | 1.329 | XXXXOOOO | −1 | XXOOOOO | −1.329 |
| OXOOOO | −0.547 | OOOXXXXX | 1.465 | XOXXXX | 0.547 | XXXOOOOO | −1.465 |
| OOXOOOO | −0.547 | OOOOXXXXX | 1.496 | XXOXXXX | 0.547 | XXXXOOOOO | −1.496 |

**INTERNATIONAL SEARCH REPORT**

International application No.
**PCT/JP2023/007619**

**A. CLASSIFICATION OF SUBJECT MATTER**

***A61K 35/28***(2015.01)i; ***A61P 25/02***(2006.01)i; ***A61P 25/28***(2006.01)i; ***C12N 5/0775***(2010.01)i; ***C12N 5/10***(2006.01)i; ***C12N 15/12***(2006.01)i; ***C12N 15/37***(2006.01)i; ***C12N 15/54***(2006.01)i; ***C12N 15/86***(2006.01)i
FI: A61K35/28; A61P25/02; A61P25/28; C12N5/0775; C12N5/10; C12N15/12 ZNA; C12N15/37; C12N15/54; C12N15/86 Z

According to International Patent Classification (IPC) or to both national classification and IPC

**B. FIELDS SEARCHED**

Minimum documentation searched (classification system followed by classification symbols)

A61K35/28; A61P25/02; A61P25/28; C12N5/0775; C12N5/10; C12N15/12; C12N15/37; C12N15/54; C12N15/86

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq

**C. DOCUMENTS CONSIDERED TO BE RELEVANT**

| Category* | Citation of document, with indication, where appropriate, of the relevant passages | Relevant to claim No. |
|---|---|---|
| Y | WO 2014/126176 A1 (NATIONAL UNIVERSITY CORP. NAGOYA UNIVERSITY) 21 August 2014 (2014-08-21)<br>particularly, claims, examples | 1-11 |
| A | | 12-14 |
| Y | SHIMOJIMA, C. et al. Conditioned Medium from the Stem Cells of Human Exfoliated Deciduous Teeth Ameliorates Experimental Autoimmune Encephalomyelitis. J Immunol. 2016, 196(10), pp. 4164-71<br>abstract | 1-11 |
| A | | 12-14 |
| Y | 山本 朗仁, 他2名, 歯髄幹細胞培養上清による多発性硬化症改善効果, Bio Industry. 2017, 34(3), pp. 70-77, (YAMAMOTO, Akihito and 2 others.), non-official translation (Improvement of multiple sclerosis by dental pulp stem cell culture supernatant.)<br>p. 73, first paragraph to p. 74, fifth paragraph | 1-11 |
| A | | 12-14 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 May 2023** | **23 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/007619**

| C. DOCUMENTS CONSIDERED TO BE RELEVANT | | |
|---|---|---|
| Category* | Citation of document, with indication, where appropriate, of the relevant passages | Relevant to claim No. |
| Y<br><br>A | WO 2017/078176 A1 (QUARRYMEN&CO. INC.) 11 May 2017 (2017-05-11)<br>particularly, claims, examples, paragraphs [0010]-[0015], [0039] | 1-11<br><br>12-14 |
| Y<br><br>A | ASHIBA, K. et al. Immortalized mesenchymal stem cells producing conditioned medium in a large scale for therapeutic usage. Inflammation and Regeneration. 2015, 35(2), pp. 57-60<br>abstract, p. 58, left column, second paragraph to p. 59, right column, second paragraph | 1-11<br><br>12-14 |
| Y<br><br>A | 椙村 有紀子, 他7名, 脱落乳歯歯髄幹細胞由来液性因子を用いた末梢神経再生治療の可能性, 再生医療, 2014, 13(Suppl), p. 333, P-2-098, (SUGIMURA, Yukiko and 7 others. Regenerative Medicine.), non-official translation (Possibility of peripheral nerve regeneration treatment using humoral factors derived from deciduous tooth pulp stem cells.)<br>特に, 「材料・方法」, 「結果」, 「結論」の項, non-official translation (particularly, “Materials and Methods”, “Results”, “Conclusions”) | 1-11<br><br>12-14 |
| Y<br><br>A | WO 2015/111712 A1 (QUARRYMEN CORP.) 30 July 2015 (2015-07-30)<br>particularly, claims, examples | 1-6<br><br>7-14 |

Form PCT/ISA/210 (second sheet) (January 2015)

**INTERNATIONAL SEARCH REPORT**

International application No.
**PCT/JP2023/007619**

**Box No. I    Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "The form of Annex C/ST.25 text file" above shall read as "the form of ST.26.".

Form PCT/ISA/210 (continuation of first sheet) (January 2015)

**INTERNATIONAL SEARCH REPORT**
**Information on patent family members**

International application No.
**PCT/JP2023/007619**

| Patent document cited in search report | Publication date (day/month/year) | Patent family member(s) | Publication date (day/month/year) |
|---|---|---|---|
| WO 2014/126176 A1 | 21 August 2014 | US 2015/0366917 A1<br>particularly, claims, examples | |
| WO 2017/078176 A1 | 11 May 2017 | US 2019/0017030 A1<br>particularly, claims, examples, paragraphs [0010]-[0016], [0050]<br>EP 3372681 A1<br>CN 108368501 A | |
| WO 2015/111712 A1 | 30 July 2015 | US 2017/0007677 A1<br>particularly, claims, examples<br>EP 3093023 A1<br>CN 106102754 A<br>KR 10-2016-0120726 A<br>JP 2017-160264 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **VIZOSO FJ** ; **EIRO N** ; **CID S** ; **SCHNEIDER J** ; **PEREZ-FERNANDEZ R**. Mesenchymal Stem Cell Secretome: Toward Cell-Free Therapeutic Strategies in Regenerative Medicine. *Int J Mol Sci.*, 25 August 2017, vol. 18 (9), 1852
- **DIMACHKIE MM** ; **BAROHN RJ**. Guillain-Barre syndrome and variants. *Neurol Clin*, May 2013, vol. 31 (2), 491-510
- **TSAI CH** ; **LIN YH** ; **LI YS** ; **HO TL** ; **HOAI THUONG LH** ; **LIU YH**. Integrated Medicine for Chemotherapy-Induced Peripheral Neuropathy. *Int J Mol Sci*, 26 August 2021, vol. 22 (17), 9257
- **S.R.CHAPLAN et al.** Quantitative assessment of tactile allodynia in the rat paw. *Journal of Neuroscience Methods*, vol. 53 (1), 55-63